# EUROPEAN PATENT SPECIFICATION

(11) **EP 2 927 316 B1**
(45) Date of publication and mention of the grant of the patent: **07.11.2018**
(21) Application number: 14162869.3
(22) Date of filing: 31.03.2014
(51) Int. Cl.: C12P 19/18, C12P 19/04, C12N 9/10, C12N 15/70

(54) **Total fermentation of oligosaccharides**
Vollständige Fermentation von Oligosacchariden
Fermentation totale d'oligosaccharides

(43) Date of publication of application: 07.10.2015
(73) Proprietor: Jennewein Biotechnologie GmbH, 53619 Rheinbreitbach (DE)
(72) Inventor: Jennewein, Stefan, Dr., 53604 Bad Honnef (DE)
(74) Representative: Witte, Weller & Partner Patentanwälte mbB

(56) References cited:
- WO-A1-2010/142305
- WO-A2-2012/007481
- US-A1- 2002 042 369
- US-A1- 2009 082 307
- PRIEM BERNARD ET AL: "A new fermentation process allows large-scale production of human milk oligosaccharides by metabolically engineered bacteria", GLYCOBIOLOGY, OXFORD UNIVERSITY PRESS, US, vol. 12, no. 4, 1 April 2002 (2002-04-01), pages 235-240, XP002380632, ISSN: 0959-6658, DOI: 10.1093/GLYCOB/12.4.235
- RUFFING ANNE ET AL: "Metabolic engineering of microbes for oligosaccharide and polysaccharide synthesis", MICROBIAL CELL FACTORIES, BIOMED CENTRAL, LONDON, NL, vol. 5, no. 1, 21 July 2006 (2006-07-21), page 25, XP021017786, ISSN: 1475-2859, DOI: 10.1186/1475-2859-5-25
- COTTAZ ET AL: "Genetic engineering of Escherichia coli for the production of N<I>,N<II>-diacetylchitobiose (chitinbiose) and its utilization as a primer for the synthesis of complex carbohydrates", METABOLIC ENGINEERING, ACADEMIC PRESS, US, vol. 7, no. 4, 1 July 2005 (2005-07-01), pages 311-317, XP005052642, ISSN: 1096-7176, DOI: 10.1016/J.YMBEN.2005.05.004
- BETTLER E ET AL: "The living factory: in vivo production of N-acetyllactosamine containing carbohydrates in E. coli", GLYCOCONJUGATE JOURNAL, CHAPMAN & HALL, vol. 16, no. 3, 1 March 1999 (1999-03-01), pages 205-212, XP002134857, ISSN: 0282-0080, DOI: 10.1023/A:1007024320183
- NAM SOO HAN ET AL: "Biotechnological production of human milk oligosaccharides", BIOTECHNOLOGY ADVANCES, vol. 30, no. 6, 1 November 2012 (2012-11-01), pages 1268-1278, XP055074947, ISSN: 0734-9750, DOI: 10.1016/j.biotechadv.2011.11.003

### SEQUENCE LISTING

<110> Jennewein Biotechnologie GmbH
<120> Total fermentation of oligosaccharides
<130> 2827P109EP
<160> 34
<170> PatentIn version 3.5
<210> 1
   <211> 2851
   <212> DNA
   <213> Artificial Sequence
<220>
   <223> Ptet-lacY-FRT-aadA-FRT
<400> 1
   tggccagatg attaattcct aatttttgtt gacactctat cattgataga gttattttac      60
   cactccctat cagtgataga gaaaagtgaa atgaatagtt cgacaaaaat ctagaaataa     120
   ttttgtttaa ctttaagaag gagatataca aatgtactat ttaaaaaaca caaacttttg     180
   gatgttcggt ttattctttt tcttttactt ttttatcatg ggagcctact tcccgttttt     240
   cccgatttgg ctacatgaca tcaaccatat cagcaaaagt gatacgggta ttatttttgc     300
   cgctatttct ctgttctcgc tattattcca accgctgttt ggtctgcttt ctgacaaact     360
   cgggctgcgc aaatacctgc tgtggattat taccggcatg ttagtgatgt ttgcgccgtt     420
   ctttattttt atcttcgggc cactgttaca atacaacatt ttagtaggat cgattgttgg     480
   tggtatttat ctaggctttt gttttaacgc cggtgcgcca gcagtagagg catttattga     540
   gaaagtcagc cgtcgcagta atttcgaatt tggtcgcgcg cggatgtttg gctgtgttgg     600
   ctgggcgctg tgtgcctcga ttgtcggcat catgttcacc atcaataatc agtttgtttt     660
   ctggctgggc tctggctgtg cactcatcct cgccgtttta ctctttttcg ccaaaacgga     720
   tgcgccctct tctgccacgg ttgccaatgc ggtaggtgcc aaccattcgg catttagcct     780
   taagctggca ctggaactgt tcagacagcc aaaactgtgg tttttgtcac tgtatgttat     840
   tggcgtttcc tgcacctacg atgtttttga ccaacagttt gctaatttct ttacttcgtt     900
   ctttgctacc ggtgaacagg gtacgcgggt atttggctac gtaacgacaa tgggcgaatt     960
   acttaacgcc tcgattatgt tctttgcgcc actgatcatt aatcgcatcg gtgggaaaaa    1020
   cgccctgctg ctggctggca ctattatgtc tgtacgtatt attggctcat cgttcgccac    1080
   ctcagcgctg gaagtggtta ttctgaaaac gctgcatatg tttgaagtac cgttcctgct    1140
   ggtgggctgc tttaaatata ttaccagcca gtttgaagtg cgtttttcag cgacgattta    1200
   tctggtctgt ttctgcttct ttaagcaact ggcgatgatt tttatgtctg tactggcggg    1260
   caatatgtat gaaagcatcg gtttccaggg cgcttatctg gtgctgggtc tggtggcgct 1320
   gggcttcacc ttaatttccg tgttcacgct tagcggcccc ggcccgcttt ccctgctgcg 1380
   tcgtcaggtg aatgaagtcg ctgggagcta agcggccgcg tcgacacgca aaaaggccat 1440
   ccgtcaggat ggccttctgc ttaatttgat gcctggcagt ttatggcggg cgtcctgccc 1500
   gccaccctcc gggccgttgc ttcgcaacgt tcaaatccgc tcccggcgga tttgtcctac 1560
   tcaggagagc gttcaccgac aaacaacaga taaaacgaaa ggcccagtct ttcgactgag 1620
   cctttcgttt tatttgatgc ctggcagttc cctactctcg catggggaga ccccacacta 1680
   ccatcatgta tgaatatcct ccttagttcc tattccgaag ttcctattct ctagaaagta 1740
   taggaacttc ggcgcgtcct acctgtgaca cgcgtgccgc agtctcacgc ccggagcgta 1800
   gcgaccgagt gagctagcta tttgtttatt tttctaaata cattcaaata tgtatccgct 1860
   catgagacaa taaccctgat aaatgcttca ataatattga aaaaggaaga gtatgaggga 1920
   agcggtgatc gccgaagtat cgactcaact atcagaggta gttggcgtca tcgagcgcca 1980
   tctcgaaccg acgttgctgg ccgtacattt gtacggctcc gcagtggatg gcggcctgaa 2040
   gccacacagt gatattgatt tgctggttac ggtgaccgta aggcttgatg aaacaacgcg 2100
   gcgagctttg atcaacgacc ttttggaaac ttcggcttcc cctggagaga gcgagattct 2160
   ccgcgctgta gaagtcacca ttgttgtgca cgacgacatc attccgtggc gttatccagc 2220
   taagcgcgaa ctgcaatttg gagaatggca gcgcaatgac attcttgcag gtatcttcga 2280
   gccagccacg atcgacattg atctggctat cttgctgaca aaagcaagag aacatagcgt 2340
   tgccttggta ggtccagcgg cggaggaact ctttgatccg gttcctgaac aggatctatt 2400
   tgaggcgcta aatgaaacct taacgctatg gaactcgccg cccgactggg ctggcgatga 2460
   gcgaaatgta gtgcttacgt tgtcccgcat ttggtacagc gcagtaaccg gcaaaatcgc 2520
   gccgaaggat gtcgctgccg actgggcaat ggagcgcctg ccggcccagt atcagcccgt 2580
   catacttgaa gctagacagg cttatcttgg acaagaagaa gatcgcttgg cctcgcgcgc 2640
   agatcagttg gaagaatttg tccactacgt gaaaggcgag atcaccaagg tagtcggcaa 2700
   ataatgtcta acaattcgtt caagccgagg ggccgcaaga tccggccacg atgacccggt 2760
   cgtcgggtac cggcagggcg gggcgtaagg cgcgccattt aaatgaagtt cctattccga 2820
   agttcctatt ctctagaaag tataggaact t 2851
<210> 2
   <211> 2858
   <212> DNA
   <213> Artificial Sequence
<220>
   <223> Ptet-wbgL-FRT-neo-FRT
<400> 2
   ggccagatga ttaattccta atttttgttg acactctatc attgatagag ttattttacc        60
   actccctatc agtgatagag aaaagtgaaa tgaatagttc gacaaaaatc tagaaataat       120
   tttgtttaac tttaagaagg agatatacaa atgggcagca ttattcgtct gcagggtggt       180
   ctgggtaatc agctgtttca gtttagcttt ggttatgccc tgagcaaaat taatggtaca       240
   ccgctgtatt tcgacattag ccattatgcc gaaaacgatg atcatggtgg ttatcgtctg       300
   aataatctgc agattccgga agaatatctg cagtattata ccccgaaaat taataatatt       360
   tataaactgc tggtgcgtgg cagccgtctg tatccggata tttttctgtt tctgggcttt       420
   tgcaacgaat ttcatgccta tggctacgat tttgaatata ttgcccagaa atggaaaagc       480
   aaaaaataca ttggctactg gcagagcgaa cacttttttc ataaacatat tctggacctg       540
   aaagaatttt ttattccgaa aaatgtgagc gaacaggcaa atctgctggc agcaaaaatt       600
   ctggaaagcc agagcagcct gagcattcat attcgtcgtg gcgattatat taaaaacaaa       660
   accgcaaccc tgacacatgg tgtttgtagc ctggaatatt ataaaaaagc cctgaacaaa       720
   atccgcgatc tggcaatgat tcgtgatgtg tttatcttta gcgacgatat cttctggtgc       780
   aaagaaaata ttgaaaccct gctgagcaaa aaatataata tttattatag cgaagatctg       840
   agccaagaag aggatctgtg gctgatgagc ctggcaaatc atcatattat tgccaatagc       900
   agctttagtt ggtggggtgc atatctgggt agcagcgcaa gccagattgt tatttatccg       960
   accccgtggt atgatattac cccgaaaaac acctatatcc cgattgtgaa ccattggatc      1020
   aacgttgata aacatagcag ctgctaagcg gccgcgtcga cacgcaaaaa ggccatccgt      1080
   caggatggcc ttctgcttaa tttgatgcct ggcagtttat ggcgggcgtc ctgcccgcca      1140
   ccctccgggc cgttgcttcg caacgttcaa atccgctccc ggcggatttg tcctactcag      1200
   gagagcgttc accgacaaac aacagataaa acgaaaggcc cagtctttcg actgagcctt      1260
   tcgttttatt tgatgcctgg cagttcccta ctctcgcatg gggagacccc acactaccat      1320
   catgtatgaa tatcctcctt agttcctatt ccgaagttcc tattctctag aaagtatagg      1380
   aacttcggcg cgtcctacct gtgacacgcg tcaagatccc ctcacgctgc cgcaagcact      1440
   cagggcgcaa gggctgctaa aggaagcgga acacgtagaa agccagtccg cagaaacggt      1500
   gctgaccccg gatgaatgtc agctactggg ctatctggac aagggaaaac gcaagcgcaa      1560
   agagaaagca ggtagcttgc agtgggctta catggcgata gctagactgg gcggttttat      1620
   ggacagcaag cgaaccggaa ttgccagctg gggcgccctc tggtaaggtt gggaagccct      1680
   gcaaagtaaa ctggatggct ttcttgccgc caaggatctg atggcgcagg ggatcaagat      1740
   ctgatcaaga gacaggatga ggatcgtttc gcatgattga acaagatgga ttgcacgcag      1800
   gttctccggc cgcttgggtg gagaggctat tcggctatga ctgggcacaa cagacaatcg      1860
   gctgctctga tgccgccgtg ttccggctgt cagcgcaggg gcgcccggtt ctttttgtca 1920
   agaccgacct gtccggtgcc ctgaatgaac tgcaggacga ggcagcgcgg ctatcgtggc 1980
   tggccacgac gggcgttcct tgcgcagctg tgctcgacgt tgtcactgaa gcgggaaggg 2040
   actggctgct attgggcgaa gtgccggggc aggatctcct gtcatctcac cttgctcctg 2100
   ccgagaaagt atccatcatg gctgatgcaa tgcggcggct gcatacgctt gatccggcta 2160
   cctgcccatt cgaccaccaa gcgaaacatc gcatcgagcg agcacgtact cggatggaag 2220
   ccggtcttgt cgatcaggat gatctggacg aagagcatca ggggctcgcg ccagccgaac 2280
   tgttcgccag gctcaaggcg cgcatgcccg acggcgagga tctcgtcgtg acccatggcg 2340
   atgcctgctt gccgaatatc atggtggaaa atggccgctt ttctggattc atcgactgtg 2400
   gccggctggg tgtggcggac cgctatcagg acatagcgtt ggctacccgt gatattgctg 2460
   aagagcttgg cggcgaatgg gctgaccgct tcctcgtgct ttacggtatc gccgctcccg 2520
   attcgcagcg catcgccttc tatcgccttc ttgacgagtt cttctgagcg ggactctggg 2580
   gttcgaaatg accgaccaag cgacgcccaa cctgccatca cgagatttcg attccaccgc 2640
   cgccttctat gaaaggttgg gcttcggaat cgttttccgg gacgccggct ggatgatcct 2700
   ccagcgcggg gatctcatgc tggagttctt cgcccacccc agcttcaaaa gcgctctcgg 2760
   taccggcagg gcggggcgta aggcgcgcca tttaaatgaa gttcctattc cgaagttcct 2820
   attctctaga aagtatagga acttcgaagc agctccag 2858
<210> 3
   <211> 2631
   <212> DNA
   <213> Artificial Sequence
<220>
   <223> Ptet-yberc0001_9420-FRT-cat-FRT
<400> 3
   ggccagatga ttaattccta atttttgttg acactctatc attgatagag ttattttacc      60
   actccctatc agtgatagag aaaagtgaaa tgaatagttc gacaaaaatc tagaaataat     120
   tttgtttaac tttaagaagg agatatacaa atgaagtcgg cactgacctt ttcccgtcgc     180
   atcaatccgg tgtttctggc gttctttgtc gttgcttttc tgagcggtat cgcaggcgca     240
   ctgcaggctc cgaccctgag tctgtttctg tccacggaag tgaaagttcg tccgctgtgg     300
   gttggtctgt tctataccgt caacgcaatc gctggcatta cggttagctt tatcctggcg     360
   aaacgttcag attcgcgcgg tgaccgtcgc aagctgatta tggtgtgcta tctgatggcg     420
   gttggcaact gtctgctgtt tgccttcaat cgtgattacc tgaccctgat cacggcaggt     480
   gtgctgctgg cgagcgttgc caacaccgca atgccgcaga ttttcgcgct ggcccgtgaa     540
   tatgccgaca gctctgcacg cgaagtggtt atgtttagtt ccatcatgcg cgctcaactg     600
   agtctggcat gggtgattgg tccgccgctg tcctttatgc tggcgctgaa ttacggtttt 660
   accctgatgt tctcaatcgc ggccggcatt ttcgttctgt cggccctggt cgtgtggttt 720
   atcctgccga gtgtcccgcg tgcagaaccg gttgtcgatg caccggtggt tgtccagggt 780
   tcactgttcg cagacaaaaa cgttctgctg ctgtttatcg cgtcgatgct gatgtggacc 840
   tgcaatacga tgtatattat cgatatgccg ctgtacatta ccgcaagcct gggtctgccg 900
   gaacgtctgg ctggtctgct gatgggtacc gcagctggcc tggaaattcc gatcatgctg 960
   ctggcgggtt attctgtgcg ttactttggc aaacgcaaga ttatgctgtt cgctgttctg 1020
   gcgggtgtcc tgttttatac cggcctggtt ctgtttaaat tcaagacggc cctgatgctg 1080
   ctgcagatct ttaacgcaat tttcatcggt attgtggctg gcattggtat gctgtacttc 1140
   caagatctga tgccgggtcg tgcaggtgca gcaaccacgc tgtttaccaa tagcatctct 1200
   acgggtgtca ttctggcagg cgtgctgcaa ggcggtctga ccgaaacgtg gggccatgac 1260
   agcgtctatg tgatggcgat ggtcctgtct attctggccc tgattatctg tgcacgtgtg 1320
   cgcgaagctt aaatcgatac tagcataacc ccttggggcc tctaaacgcg tcgacacgca 1380
   aaaaggccat ccgtcaggat ggccttctgc ttaatttgat gcctggcagt ttatggcggg 1440
   cgtcctgccc gccaccctcc gggccgttgc ttcgcaacgt tcaaatccgc tcccggcgga 1500
   tttgtcctac tcaggagagc gttcaccgac aaacaacaga taaaacgaaa ggcccagtct 1560
   ttcgactgag cctttcgttt tatttgatgc ctggcagttc cctactctcg catggggaga 1620
   ccccacacta ccatcatgta tgaatatcct ccttagttcc tattccgaag ttcctattct 1680
   ctagaaagta taggaacttc ggcgcgtcct acctgtgacg gaagatcact tcgcagaata 1740
   aataaatcct ggtgtccctg ttgataccgg gaagccctgg gccaactttt ggcgaaaatg 1800
   agacgttgat cggcacgtaa gaggttccaa ctttcaccat aatgaaataa gatcactacc 1860
   gggcgtattt tttgagttgt cgagattttc aggagctaag gaagctaaaa tggagaaaaa 1920
   aatcactgga tataccaccg ttgatatatc ccaatggcat cgtaaagaac attttgaggc 1980
   atttcagtca gttgctcaat gtacctataa ccagaccgtt cagctggata ttacggcctt 2040
   tttaaagacc gtaaagaaaa ataagcacaa gttttatccg gcctttattc acattcttgc 2100
   ccgcctgatg aatgctcatc cggaattacg tatggcaatg aaagacggtg agctggtgat 2160
   atgggatagt gttcaccctt gttacaccgt tttccatgag caaactgaaa cgttttcatc 2220
   gctctggagt gaataccacg acgatttccg gcagtttcta cacatatatt cgcaagatgt 2280
   ggcgtgttac ggtgaaaacc tggcctattt ccctaaaggg tttattgaga atatgttttt 2340
   cgtctcagcc aatccctggg tgagtttcac cagttttgat ttaaacgtgg ccaatatgga 2400
   caacttcttc gcccccgttt tcaccatggg caaatattat acgcaaggcg acaaggtgct 2460
   gatgccgctg gcgattcagg ttcatcatgc cgtttgtgat ggcttccatg tcggcagatg     2520
   cttaatgaat acaacagtac tgcgatgagt ggcagggcgg ggcgtaaggc gcgccattta     2580
   aatgaagttc ctattccgaa gttcctattc tctagaaagt ataggaactt c              2631
<210> 4
   <211> 6783
   <212> DNA
   <213> Artificial Sequence
<220>
   <223> Ptet-manCB,PT5-gmd-wcaG-FRT-dhfr-FRT
<400> 4
   gccagatgat taattcctaa tttttgttga cactctatca ttgatagagt tattttacca      60
   ctccctatca gtgatagaga aaagtgaaat gaatagttcg acaaaaatct agaaataatt     120
   ttgtttaact ttaagaagga gatatacaat ttcgtcgaca cacaggaaac atattaaaaa     180
   ttaaaacctg caggagtttg aaggagatag aaccatggcg cagtcgaaac tctatccagt     240
   tgtgatggca ggtggctccg gtagccgctt atggccgctt tcccgcgtac tttatcccaa     300
   gcagttttta tgcctgaaag gcgatctcac catgctgcaa accaccatct gccgcctgaa     360
   cggcgtggag tgcgaaagcc cggtggtgat ttgcaatgag cagcaccgct ttattgtcgc     420
   ggaacagctg cgtcaactga acaaacttac cgagaacatt attctcgaac cggcagggcg     480
   aaacacggca cctgccattg cgctggcggc gctggcggca aaacgtcata gcccggagag     540
   cgacccgtta atgctggtat tggcggcgga tcatgtgatt gccgatgaag acgcgttccg     600
   tgccgccgtg cgtaatgcca tgccatatgc cgaagcgggc aagctggtga ccttcggcat     660
   tgtgccggat ctaccagaaa ccggttatgg ctatattcgt cgcggtgaag tgtctgcggg     720
   tgagcaggat atggtggcct ttgaagtggc gcagtttgtc gaaaaaccga atctggaaac     780
   cgctcaggcc tatgtggcaa gcggcgaata ttactggaac agcggtatgt tcctgttccg     840
   cgccggacgc tatctcgaag aactgaaaaa atatcgcccg gatatcctcg atgcctgtga     900
   aaaagcgatg agcgccgtcg atccggatct caattttatt cgcgtggatg aagaagcgtt     960
   tctcgcctgc ccggaagagt cggtggatta cgcggtcatg gaacgtacgg cagatgctgt    1020
   tgtggtgccg atggatgcgg gctggagcga tgttggctcc tggtcttcat tatgggagat    1080
   cagcgcccac accgccgagg gcaacgtttg ccacggcgat gtgattaatc acaaaactga    1140
   aaacagctat gtgtatgctg aatctggcct ggtcaccacc gtcggggtga aagatctggt    1200
   agtggtgcag accaaagatg cggtgctgat tgccgaccgt aacgcggtac aggatgtgaa    1260
   aaaagtggtc gagcagatca aagccgatgg tcgccatgag catcgggtgc atcgcgaagt    1320
   gtatcgtccg tggggcaaat atgactctat cgacgcgggc gaccgctacc aggtgaaacg    1380
   catcaccgtg aaaccgggcg agggcttgtc ggtacagatg caccatcacc gcgcggaaca    1440
   ctgggtggtt gtcgcgggaa cggcaaaagt caccattgat ggtgatatca aactgcttgg 1500
   tgaaaacgag tccatttata ttccgctggg ggcgacgcat tgcctggaaa acccggggaa 1560
   aattccgctc gatttaattg aagtgcgctc cggctcttat ctcgaagagg atgatgtggt 1620
   gcgtttcgcg gatcgctacg gacgggtgta aacgtcgcat caggcaatga atgcgaaacc 1680
   gcggtgtaaa taacgacaaa aataaaattg gccgcttcgg tcagggccaa ctattgcctg 1740
   aaaaagggta acgatatgaa aaaattaacc tgctttaaag cctatgatat tcgcgggaaa 1800
   ttaggcgaag aactgaatga agatatcgcc tggcgcattg gtcgcgccta tggcgaattt 1860
   ctcaaaccga aaaccattgt gttaggcggt gatgtccgcc tcaccagcga aaccttaaaa 1920
   ctggcgctgg cgaaaggttt acaggatgcg ggcgttgacg tgctggatat tggtatgtcc 1980
   ggcaccgaag agatctattt cgccacgttc catctcggcg tggatggcgg cattgaagtt 2040
   accgccagcc ataatccgat ggattataac ggcatgaagc tggttcgcga gggggctcgc 2100
   ccgatcagcg gagataccgg actgcgcgac gtccagcgtc tggctgaagc caacgacttt 2160
   cctcccgtcg atgaaaccaa acgcggtcgc tatcagcaaa tcaacctgcg tgacgcttac 2220
   gttgatcacc tgttcggtta tatcaatgtc aaaaacctca cgccgctcaa gctggtgatc 2280
   aactccggga acggcgcagc gggtccggtg gtggacgcca ttgaagcccg ctttaaagcc 2340
   ctcggcgcgc ccgtggaatt aatcaaagtg cacaacacgc cggacggcaa tttccccaac 2400
   ggtattccta acccactact gccggaatgc cgcgacgaca cccgcaatgc ggtcatcaaa 2460
   cacggcgcgg atatgggcat tgcttttgat ggcgattttg accgctgttt cctgtttgac 2520
   gaaaaagggc agtttattga gggctactac attgtcggcc tgttggcaga agcattcctc 2580
   gaaaaaaatc ccggcgcgaa gatcatccac gatccacgtc tctcctggaa caccgttgat 2640
   gtggtgactg ccgcaggtgg cacgccggta atgtcgaaaa ccggacacgc ctttattaaa 2700
   gaacgtatgc gcaaggaaga cgccatctat ggtggcgaaa tgagcgccca ccattacttc 2760
   cgtgatttcg cttactgcga cagcggcatg atcccgtggc tgctggtcgc cgaactggtg 2820
   tgcctgaaag ataaaacgct gggcgaactg gtacgcgacc ggatggcggc gtttccggca 2880
   agcggtgaga tcaacagcaa actggcgcaa cccgttgagg cgattaaccg cgtggaacag 2940
   cattttagcc gtgaggcgct ggcggtggat cgcaccgatg gcatcagcat gacctttgcc 3000
   gactggcgct ttaacctgcg cacctccaat accgaaccgg tggtgcgcct gaatgtggaa 3060
   tcgcgcggtg atgtgccgct gatggaagcg cgaacgcgaa ctctgctgac gttgctgaac 3120
   gagtaaaaac gcggccgcga tatcgttgta aaacgacggc cagtgcaaga atcataaaaa 3180
   atttatttgc tttcaggaaa atttttctgt ataatagatt cataaatttg agagaggagt 3240
   ttttgtgagc ggataacaat tccccatctt agtatattag ttaagtataa atacaccgcg 3300
   gaggacgaag gagatagaac catgtcaaaa gtcgctctca tcaccggtgt aaccggacaa 3360
   gacggttctt acctggcaga gtttctgctg gaaaaaggtt acgaggtgca tggtattaag 3420
   cgtcgcgcat cgtcattcaa caccgagcgc gtggatcaca tttatcagga tccgcacacc 3480
   tgcaacccga aattccatct gcattatggc gacctgagtg atacctctaa cctgacgcgc 3540
   attttgcgtg aagtacagcc ggatgaagtg tacaacctgg gcgcaatgag ccacgttgcg 3600
   gtctcttttg agtcaccaga atataccgct gacgtcgacg cgatgggtac gctgcgcctg 3660
   ctggaggcga tccgcttcct cggtctggaa aagaaaactc gtttctatca ggcttccacc 3720
   tctgaactgt atggtctggt gcaggaaatt ccgcagaaag agaccacgcc gttctacccg 3780
   cgatctccgt atgcggtcgc caaactgtac gcctactgga tcaccgttaa ctaccgtgaa 3840
   tcctacggca tgtacgcctg taacggaatt ctcttcaacc atgaatcccc gcgccgcggc 3900
   gaaaccttcg ttacccgcaa aatcacccgc gcaatcgcca acatcgccca ggggctggag 3960
   tcgtgcctgt acctcggcaa tatggattcc ctgcgtgact ggggccacgc caaagactac 4020
   gtaaaaatgc agtggatgat gctgcagcag gaacagccgg aagatttcgt tatcgcgacc 4080
   ggcgttcagt actccgtgcg tcagttcgtg gaaatggcgg cagcacagct gggcatcaaa 4140
   ctgcgctttg aaggcacggg cgttgaagag aagggcattg tggtttccgt caccgggcat 4200
   gacgcgccgg gcgttaaacc gggtgatgtg attatcgctg ttgacccgcg ttacttccgt 4260
   ccggctgaag ttgaaacgct gctcggcgac ccgaccaaag cgcacgaaaa actgggctgg 4320
   aaaccggaaa tcaccctcag agagatggtg tctgaaatgg tggctaatga cctcgaagcg 4380
   gcgaaaaaac actctctgct gaaatctcac ggctacgacg tggcgatcgc gctggagtca 4440
   taagcatgag taaacaacga gtttttattg ctggtcatcg cgggatggtc ggttccgcca 4500
   tcaggcggca gctcgaacag cgcggtgatg tggaactggt attacgcacc cgcgacgagc 4560
   tgaacctgct ggacagccgc gccgtgcatg atttctttgc cagcgaacgt attgaccagg 4620
   tctatctggc ggcggcgaaa gtgggcggca ttgttgccaa caacacctat ccggcggatt 4680
   tcatctacca gaacatgatg attgagagca acatcattca cgccgcgcat cagaacgacg 4740
   tgaacaaact gctgtttctc ggatcgtcct gcatctaccc gaaactggca aaacagccga 4800
   tggcagaaag cgagttgttg cagggcacgc tggagccgac taacgagcct tatgctattg 4860
   ccaaaatcgc cgggatcaaa ctgtgcgaat catacaaccg ccagtacgga cgcgattacc 4920
   gctcagtcat gccgaccaac ctgtacgggc cacacgacaa cttccacccg agtaattcgc 4980
   atgtgatccc agcattgctg cgtcgcttcc acgaggcgac ggcacagaat gcgccggacg 5040
   tggtggtatg gggcagcggt acaccgatgc gcgaatttct gcacgtcgat gatatggcgg 5100
   cggcgagcat tcatgtcatg gagctggcgc atgaagtctg gctggagaac acccagccga 5160
   tgttgtcgca cattaacgtc ggcacgggcg ttgactgcac tatccgcgag ctggcgcaaa 5220
   ccatcgccaa agtggtgggt tacaaaggcc gggtggtttt tgatgccagc aaaccggatg 5280
   gcacgccgcg caaactgctg gatgtgacgc gcctgcatca gcttggctgg tatcacgaaa 5340
   tctcactgga agcggggctt gccagcactt accagtggtt ccttgagaat caagaccgct 5400
   ttcggggggg gagctaacgc gccatttaaa tcaacctcag cggtcatagc tgtttcctgt 5460
   gactgagcaa taactagcat aaccccttgg ggcctctaaa cgggtcttga ggggtttttt 5520
   gctgaaacca atttgcctgg cggcagtagc gcggtggtcc cacctgaccc catgccgaac 5580
   tcagaagtga aacgccgtag cgccgatggt agtgtggggt ctccccatgc gagagtaggg 5640
   aactgccagg catcaaataa aacgaaaggc tcagtcgaaa gactgggcct ttcgggatcc 5700
   aggccggcct gttaacgaat taatcttccg cggcggtatc gataagcttg atatcgaatt 5760
   ccgaagttcc tattctctag aaagtatagg aacttcaggt ctgaagagga gtttacgtcc 5820
   agccaagcta gcttggctgc aggtcgtcga aattctaccg ggtaggggag gcgcttttcc 5880
   caaggcagtc tggagcatgc gctttagcag ccccgctggg cacttggcgc tacacaagtg 5940
   gcctctggcc tcgcacacat tccacatcca ccggtaggcg ccaaccggct ccgttctttg 6000
   gtggcccctt cgcgccacct tctactcctc ccctagtcag gaagttcccc cccgccccgc 6060
   agctcgcgtc gtgcaggacg tgacaaatgg aagtagcacg tctcactagt ctcgtgcaga 6120
   tggacagcac cgctgagcaa tggaagcggg taggcctttg gggcagcggc caatagcagc 6180
   tttgctcctt cgctttctgg gctcagaggc tgggaagggg tgggtccggg ggcgggctca 6240
   ggggcgggct caggggcggg gcgggcgccc gaaggtcctc cggaggcccg gcattctgca 6300
   cgcttcaaaa gcgcacgtct gccgcgctgt tctcctcttc ctcatctccg ggcctttcga 6360
   cctgcagcct gttgacaatt aatcatcggc atagtatatc ggcatagtat aatacgacaa 6420
   ggtgaggaac taaaccatgg gtcaaagtag cgatgaagcc aacgctcccg ttgcagggca 6480
   gtttgcgctt cccctgagtg ccacctttgg cttaggggat cgcgtacgca agaaatctgg 6540
   tgccgcttgg cagggtcaag tcgtcggttg gtattgcaca aaactcactc ctgaaggcta 6600
   tgcggtcgag tccgaatccc acccaggctc agtgcaaatt tatcctgtgg ctgcacttga 6660
   acgtgtggcc taatgagggg atcaattctc tagagctcgc tgatcagaag ttcctattct 6720
   ctagaaagta taggaacttc gatggcgcct catccctgaa gccaataggg ataacagggt 6780
   aat 6783
<210> 5
   <211> 4844
   <212> DNA
   <213> Artificial Sequence
<220>
   <223> cscBKAR
<400> 5
   ctatattgct gaaggtacag gcgtttccat aactatttgc tcgcgttttt tactcaagaa        60
   gaaaatgcca aatagcaaca tcaggcagac aatacccgaa attgcgaaga aaactgtctg       120
   gtagcctgcg tggtcaaaga gtatcccagt cggcgttgaa agcagcacaa tcccaagcga       180
   actggcaatt tgaaaaccaa tcagaaagat cgtcgacgac aggcgcttat caaagtttgc       240
   cacgctgtat ttgaagacgg atatgacaca aagtggaacc tcaatggcat gtaacaactt       300
   cactaatgaa ataatccagg ggttaacgaa cagcgcgcag gaaaggatac gcaacgccat       360
   aatcacaact ccgataagta atgcattttt tggccctacc cgattcacaa agaaaggaat       420
   aatcgccatg cacagcgctt cgagtaccac ctggaatgag ttgagataac catacaggcg       480
   cgttcctaca tcgtgtgatt cgaataaacc tgaataaaag acaggaaaaa gttgttgatc       540
   aaaaatgtta tagaaagacc acgtccccac aataaatatg acgaaaaccc agaagtttcg       600
   atccttgaaa actgcgataa aatcctcttt ttttacccct cccgcatctg ccgctacgca       660
   ctggtgatcc ttatctttaa aacgcatgtt gatcatcata aatacagcgc caaatagcga       720
   gaccaaccag aagttgatat ggggactgat actaaaaaat atgccggcaa agaacgcgcc       780
   aatagcatag ccaaaagatc cccaggcgcg cgctgttcca tattcgaaat gaaaatttcg       840
   cgccattttt tcggtgaagc tatcaagcaa accgcatccc gccagatacc ccaagccaaa       900
   aaatagcgcc cccagaatta gacctacaga aaaattgctt tgcagtaacg gttcataaac       960
   gtaaatcata aacggtccgg tcaagaccag gatgaaactc atacaccaga tgagcggttt      1020
   cttcagaccg agtttatcct gaacgatgcc gtagaacatc ataaatagaa tgctggtaaa      1080
   ctggttgacc gaataaagtg tacctaattc cgtccctgtc aaccctagat gtcctttcag      1140
   ccaaatagcg tataacgacc accacagcga ccaggaaata aaaaagagaa atgagtaact      1200
   ggatgcaaaa cgatagtacg catttctgaa tggaatattc agtgccataa ttacctgcct      1260
   gtcgttaaaa aattcacgtc ctatttagag ataagagcga cttcgccgtt tacttctcac      1320
   tattccagtt cttgtcgaca tggcagcgct gtcattgccc ctttcgccgt tactgcaagc      1380
   gctccgcaac gttgagcgag atcgataatt cgtcgcattt ctctctcatc tgtagataat      1440
   cccgtagagg acagacctgt gagtaacccg gcaacgaacg catctcccgc ccccgtgcta      1500
   tcgacacaat tcacagacat tccagcaaaa tggtgaactt gtcctcgata acagaccacc      1560
   accccttctg cacctttagt caccaacagc atggcgatct catactcttt tgccagggcg      1620
   catatatcct gatcgttctg tgtttttcca ctgataagtc gccattcttc ttccgagagc      1680
   ttgacgacat ccgccagttg tagcgcctgc cgcaaacaca agcggagcaa atgctcgtct      1740
   tgccatagat cttcacgaat attaggatcg aagctgacaa aacctccggc atgccggatc      1800
   gccgtcatcg cagtaaatgc gctggtacgc gaaggctcgg cagacaacgc aattgaacag      1860
   agatgtaacc attcgccatg tcgccagcag ggcaagtctg tcgtctctaa aaaaagatcg 1920
   gcactggggc ggaccataaa cgtaaatgaa cgttcccctt gatcgttcag atcgacaagc 1980
   accgtggatg tccggtgcca ttcatcttgc ttcagatacg tgatatcgac tccctcagtt 2040
   agcagcgttc tttgcattaa cgcaccaaaa ggatcatccc ccacccgacc tataaaccca 2100
   cttgttccgc ctaatctggc gattcccacc gcaacgttag ctggcgcgcc gccaggacaa 2160
   ggcagtaggc gcccgtctga ttctggcaag agatctacga ccgcatcccc taaaacccat 2220
   actttggctg acattttttt cccttaaatt catctgagtt acgcatagtg ataaacctct 2280
   ttttcgcaaa atcgtcatgg atttactaaa acatgcatat tcgatcacaa aacgtcatag 2340
   ttaacgttaa catttgtgat attcatcgca tttatgaaag taagggactt tatttttata 2400
   aaagttaacg ttaacaattc accaaatttg cttaaccagg atgattaaaa tgacgcaatc 2460
   tcgattgcat gcggcgcaaa acgccctagc aaaacttcat gagcaccggg gtaacacttt 2520
   ctatccccat tttcacctcg cgcctcctgc cgggtggatg aacgatccaa acggcctgat 2580
   ctggtttaac gatcgttatc acgcgtttta tcaacatcat ccgatgagcg aacactgggg 2640
   gccaatgcac tggggacatg ccaccagcga cgatatgatc cactggcagc atgagcctat 2700
   tgcgctagcg ccaggagacg ataatgacaa agacgggtgt ttttcaggta gtgctgtcga 2760
   tgacaatggt gtcctctcac ttatctacac cggacacgtc tggctcgatg gtgcaggtaa 2820
   tgacgatgca attcgcgaag tacaatgtct ggctaccagt cgggatggta ttcatttcga 2880
   gaaacagggt gtgatcctca ctccaccaga aggaatcatg cacttccgcg atcctaaagt 2940
   gtggcgtgaa gccgacacat ggtggatggt agtcggggcg aaagatccag gcaacacggg 3000
   gcagatcctg ctttatcgcg gcagttcgtt gcgtgaatgg accttcgatc gcgtactggc 3060
   ccacgctgat gcgggtgaaa gctatatgtg ggaatgtccg gactttttca gccttggcga 3120
   tcagcattat ctgatgtttt ccccgcaggg aatgaatgcc gagggataca gttaccgaaa 3180
   tcgctttcaa agtggcgtaa tacccggaat gtggtcgcca ggacgacttt ttgcacaatc 3240
   cgggcatttt actgaacttg ataacgggca tgacttttat gcaccacaaa gctttttagc 3300
   gaaggatggt cggcgtattg ttatcggctg gatggatatg tgggaatcgc caatgccctc 3360
   aaaacgtgaa ggatgggcag gctgcatgac gctggcgcgc gagctatcag agagcaatgg 3420
   caaacttcta caacgcccgg tacacgaagc tgagtcgtta cgccagcagc atcaatctgt 3480
   ctctccccgc acaatcagca ataaatatgt tttgcaggaa aacgcgcaag cagttgagat 3540
   tcagttgcag tgggcgctga agaacagtga tgccgaacat tacggattac agctcggcac 3600
   tggaatgcgg ctgtatattg ataaccaatc tgagcgactt gttttgtggc ggtattaccc 3660
   acacgagaat ttagacggct accgtagtat tcccctcccg cagcgtgaca cgctcgccct 3720
   aaggatattt atcgatacat catccgtgga agtatttatt aacgacgggg aagcggtgat 3780
   gagtagtcga atctatccgc agccagaaga acgggaactg tcgctttatg cctcccacgg 3840
   agtggctgtg ctgcaacatg gagcactctg gctactgggt taacataata tcaggtggaa 3900
   caacggatca acagcgggca agggatccgc gtcactcttc ccccttcacg accttcaata 3960
   atatgcaatg cagcttcccg cccgataatg tcatgtggaa gctgaattgt ggtcagcggc 4020
   ggtaaaaaca gatgcccgac gccaaccaga ttatcaaagc ccattacggc gacatcctgc 4080
   gggattcgta cccccttcgc cagaagaacc tgataagcca caaaggctgc gcgatcgtta 4140
   ccacatatca gaacatcaaa atctggtttg cccggtttga agtgggcatt gagtaaactt 4200
   gcgagatcgg tgtagtgatc atcacctgtt gccatgtgaa attgtttcac ctcagccaga 4260
   tctcgtccag catcacgcca ggcctgctca aatccctgcc gacgataccc tgttgccaac 4320
   gcactttccg gtagccagaa gcataacggt tgacgatagc ccgccgcgag caaatgctgt 4380
   gttgattcat attgtgcagt gtaatcatca gggatataac tgggtaacgc tgggtcatcc 4440
   gccacacagt tcgccaatac aatattttca ccatacagag actcaggcag cgtgatatgt 4500
   cgcagcccca ttgtagtata gataatgcca tccggacggt gggcaagcag ctgacgtgcc 4560
   gcgcgggcag cgtcatcttc agaaaaaata ttgattaaaa aactattcca gccgaactcg 4620
   ctggcggttt gctcaatggc aagcagaata tcaacagaga aaggagtggt agccgtgtcc 4680
   tgcgccagca cggcgagagt cgacggctta cgtccttgag cgcgcatctt acgggcggaa 4740
   agatcaggaa cataattcag ggtctggatt gcctgcaata cgcggtcacg cgttgcagga 4800
   cgcacagatt ctgcattatg catcacccgg gagactgtca tcat 4844
<210> 6
   <211> 4259
   <212> DNA
   <213> Artificial Sequence
<220>
   <223> galMKTE
<400> 6
   ttactcagca ataaactgat attccgtcag gctggaatac tcttcgccag gacgcaggaa 60
   gcagtccggt tgcggccatt cagggtggtt cgggctgtcc ggtagaaact cgctttccag 120
   agccagccct tgccagtcgg cgtaaggttc ggttccccgc gacggtgtgc cgccgaggaa 180
   gttgccggag tagaattgca gagccggagc ggtggtgtag accttcagct gcaatttttc 240
   atctgctgac cagacatgcg ccgccacttt cttgccatcg cctttggcct gtaacaagaa 300
   tgcgtgatcg taacctttca ctttgcgctg atcgtcgtcg gcaagaaact cactggcgat 360
   gattttggcg ctgcggaaat caaaagacgt tccggcgaca gatttcaggc cgtcgtgcgg 420
   aatgccgcct tcatcaaccg gcagatattc gtccgccaga atctgcaact tgtgattgcg 480
   cacgtcagac tgctcgccgt caagattgaa atagacgtga ttagtcatat tcaccgggca 540
   aggtttatca actgtggcgc gataagtaat ggagatacgg ttatcgtcgg tcagacgata 600
   ttgcaccgtc gcgccgagat tacccgggaa gccctgatca ccatcatctg aactcagggc 660
   aaacagcacc tgacgatcgt tctggttcac aatctgccag cgacgtttgt cgaacccttc 720
   cggcccgccg tgcagctggt taacgccctg acttggcgaa agcgtcacgg tttcaccgtc 780
   aaaggtataa cggctattgg cgatacggtt ggcataacga ccaatagagg cccccagaaa 840
   cgcggcctga tcctgatagc attccgggct ggcacagccg agcagcgcct cgcggacgct 900
   gccatcggaa agcggaatac gggcggaaag taaagtcgca ccccagtcca tcagcgtgac 960
   taccatccct gcgttgttac gcaaagttaa cagtcggtac ggctgaccat cgggtgccag 1020
   tgcgggagtt tcgttcagca ctgtcctgct ccttgtgatg gtttacaaac gtaaaaagtc 1080
   tctttaatac ctgtttttgc ttcatattgt tcagcgacag cttgctgtac ggcaggcacc 1140
   agctcttccg ggatcagcgc gacgatacag ccgccaaatc cgccgccggt catgcgtacg 1200
   ccacctttgt cgccaatcac agctttgacg atttctacca gagtgtcaat ttgcggcacg 1260
   gtgatttcga aatcatcgcg catagaggca tgagactccg ccatcaactc gcccatacgt 1320
   ttcaggtcgc cttgctccag cgcgctggca gcttcaacgg tgcgggcgtt ttcagtcagt 1380
   atatgacgca cgcgttttgc cacgatcggg tccagttcat gcgcaacagc gttgaactct 1440
   tcaatggtga catcacgcag ggctggctgc tggaagaaac gcgcaccggt ttcgcactgt 1500
   tcacgacggg tgttgtattc gctgccaacc agggtacgtt tgaagttact gttgatgatg 1560
   acgacagcca cacctttggg catggaaact gctttggtcc ccagtgagcg gcaatcgatc 1620
   agcaaggcat gatctttctt gccgagcgcg gaaattagct gatccatgat cccgcagtta 1680
   cagcctacaa actggttttc tgcttcctga ccgttaagcg cgatttgtgc gccgtccagc 1740
   ggcagatgat aaagctgctg caatacggtt ccgaccgcga cttccagtga agcggaagaa 1800
   cttaacccgg caccctgcgg cacattgccg ctgatcacca tgtccacgcc gccgaagctg 1860
   ttgttacgca gttgcagatg tttcaccacg ccacgaacgt agttagccca ttgatagttt 1920
   tcatgtgcga caatgggcgc atcgagggaa aactcgtcga gctgattttc ataatcggct 1980
   gccatcacgc gaactttacg gtcatcgcgt ggtgcacaac tgatcacggt ttgataatca 2040
   atcgcgcagg gcagaacgaa accgtcgttg tagtcggtgt gttcaccaat caaattcacg 2100
   cggccaggcg cctgaatggt gtgagtggca gggtagccaa atgcgttggc aaacagagat 2160
   tgtgtttttt ctttcagact catttcttac actccggatt cgcgaaaatg gatatcgctg 2220
   actgcgcgca aacgctctgc tgcctgttct gcggtcaggt ctcgctgggt ctctgccagc 2280
   atttcataac caaccataaa tttacgtacg gtggcggagc gcagcagagg cggataaaag 2340
   tgcgcgtgca gctgccagtg ttgattctct tcgccattaa atggcgcgcc gtgccagccc 2400
   atagagtagg ggaaggagca ctggaagagg ttgtcataac gactggtcag ctttttcaac 2460
   gccagcgcca gatcgctgcg ctgggcgtcg gtcaaatcgg tgatccgtaa aacgtgggct 2520
   ttgggcagca gtagcgtttc gaacggccag gcagcccagt aaggcacgac ggctaaccag 2580
   tgttcggttt cgacaacggt acggctaccg tctgccagct cgcgctgaac ataatccacc 2640
   agcattggtg atttctgttc ggcaaaatat tctttttgca ggcggtcttc gcgctcagct 2700
   tcgttaggca ggaagctatt tgcccaaatc tgaccgtgcg gatgcgggtt agagcagccc 2760
   atcgccgcgc ctttgttttc aaaaacctgc acccatgggt acgttttccc cagttctgcg 2820
   gtttgctcct gccaggtttt gacgatttcc gtcaatgctg caacgctgag ctctggcagc 2880
   gttttactgt gatccggtga aaagcagatc acccggctgg tgccgcgcgc gctctggcaa 2940
   cgcatcagcg gatcgtgact ttctggcgca tctggcgtgt cagacatcaa agccgcaaag 3000
   tcattagtga aaacgtaagt cccggtgtaa tcggggtttt tatcgcctgt cacccgcaca 3060
   ttacctgcgc agaggaagca atctggatcg tgcgcaggta acacctgttt ggctggcgtt 3120
   tcctgcgccc cctgccaggg gcgcttagcg cggtgcggtg aaaccagaat ccattgcccg 3180
   gtgagcgggt tgtagcggcg atgtggatga tcaacgggat taaattgcgt catggtcgtt 3240
   ccttaatcgg gatatccctg tggatggcgt gactgccagt gccaggtgtc ctgcgccatt 3300
   tcatcgagtg tgcgcgttac gcgccagttc agttcacggt cggctttgct ggcgtccgcc 3360
   cagtaggccg gaaggtcgcc ctcgcgacgc ggtgcaaaat gataattaac cggtttgccg 3420
   caggctttgc tgaaggcatt aaccacgtcc agcacgctgt tgcctacgcc agcgccgagg 3480
   ttgtagatgt gtacgcctgg cttgttcgcc agtttttcca tcgccacgac gtgaccgtcc 3540
   gccagatcca ttacgtggat gtaatcgcgt acgccagtac catcttcggt cggataatcg 3600
   ttaccaaaaa tcgccagcga gtcgcgacgg cctacagcaa cctgggcgat gtatggcatc 3660
   aggttattcg gaatgccttg cggatcttcg cccatatcgc ccgacggatg cgcgccaacc 3720
   gggttgaagt agcgcagcag ggcaatgctc cagtccggct gggctttttg cagatcggtg 3780
   aggatctgtt ccaccatcag cttgcttttg ccgtaagggc tttgcggtgt gccggtcggg 3840
   aagctttcaa cgtatggaat tttgggctga tcgccataaa cggtggcgga ggagctaaaa 3900
   ataaagtttt tgacgttagc ggcgcgcatg gcgctaatca ggcgcagagt gccgttgaca 3960
   ttgttgtcgt aatattccag cggtttttgt accgattcgc ccacggcttt cagcccggcg 4020
   aagtggatca cggtgtcgat agcgtgatcg tgcaggatct cggtcatcaa cgcttcgtta 4080
   cgaatatcgc cttcaacaaa cgttggatgt ttgccgccta aacgctcgat aacaggcagt 4140
   acgctgcgct tactgttaca gaggttatca agaatgatga catcatgacc gttttgcagt 4200
   aattgcacac aggtatgact tccaatgtaa ccgctaccac cggtaaccag aactctcat 4259
<210> 7
   <211> 4692
   <212> DNA
   <213> Artificial Sequence
<220>
   <223> pCDF-galE
<400> 7
   ggggaattgt gagcggataa caattcccct gtagaaataa ttttgtttaa ctttaataag      60
   gagatatacc aagcttgcgg ccgcataatg cttaagtcga acagaaagta atcgtattgt     120
   acacggccgc ataatcgaaa ttaatacgac tcactatagg ggaattgtga gcggataaca     180
   attccccatc ttagtatatt agttaagtat aagaaggaga tatacagatc acatatgaga      240
   gttctggtta ccggtggtag cggttacatt ggaagtcata cctgtgtgca attactgcaa     300
   aacggtcatg atgtcatcat tcttgataac ctctgtaaca gtaagcgcag cgtactgcct     360
   gttatcgagc gtttaggcgg caaacatcca acgtttgttg aaggcgatat tcgtaacgaa     420
   gcgttgatga ccgagatcct gcacgatcac gctatcgaca ccgtgatcca cttcgccggg     480
   ctgaaagccg tgggcgaatc ggtacaaaaa ccgctggaat attacgacaa caatgtcaac     540
   ggcactctgc gcctgattag cgccatgcgc gccgctaacg tcaaaaactt tatttttagc     600
   tcctccgcca ccgtttatgg cgatcagccc aaaattccat acgttgaaag cttcccgacc     660
   ggcacaccgc aaagccctta cggcaaaagc aagctgatgg tggaacagat cctcaccgat     720
   ctgcaaaaag cccagccgga ctggagcatt gccctgctgc gctacttcaa cccggttggc     780
   gcgcatccgt cgggcgatat gggcgaagat ccgcaaggca ttccgaataa cctgatgcca     840
   tacatcgccc aggttgctgt aggccgtcgc gactcgctgg cgatttttgg taacgattat     900
   ccgaccgaag atggtactgg cgtacgcgat tacatccacg taatggatct ggcggacggt     960
   cacgtcgtgg cgatggaaaa actggcgaac aagccaggcg tacacatcta caacctcggc    1020
   gctggcgtag gcaacagcgt gctggacgtg gttaatgcct tcagcaaagc ctgcggcaaa    1080
   ccggttaatt atcattttgc accgcgtcgc gagggcgacc ttccggccta ctgggcggac    1140
   gccagcaaag ccgaccgtga actgaactgg cgcgtaacgc gcacactcga tgaaatggcg    1200
   caggacacct ggcactggca gtcacgccat ccacagggat atcccgatta atgactcgag    1260
   tgatctcgag tctggtaaag aaaccgctgc tgcgaaattt gaacgccagc acatggactc    1320
   gtctactagc gcagcttaat taacctaggc tgctgccacc gctgagcaat aactagcata    1380
   accccttggg gcctctaaac gggtcttgag gggttttttg ctgaaacctc aggcatttga    1440
   gaagcacacg gtcacactgc ttccggtagt caataaaccg gtaaaccagc aatagacata    1500
   agcggctatt taacgaccct gccctgaacc gacgaccggg tcatcgtggc cggatcttgc    1560
   ggcccctcgg cttgaacgaa ttgttagaca ttatttgccg actaccttgg tgatctcgcc 1620
   tttcacgtag tggacaaatt cttccaactg atctgcgcgc gaggccaagc gatcttcttc 1680
   ttgtccaaga taagcctgtc tagcttcaag tatgacgggc tgatactggg ccggcaggcg 1740
   ctccattgcc cagtcggcag cgacatcctt cggcgcgatt ttgccggtta ctgcgctgta 1800
   ccaaatgcgg gacaacgtaa gcactacatt tcgctcatcg ccagcccagt cgggcggcga 1860
   gttccatagc gttaaggttt catttagcgc ctcaaataga tcctgttcag gaaccggatc 1920
   aaagagttcc tccgccgctg gacctaccaa ggcaacgcta tgttctcttg cttttgtcag 1980
   caagatagcc agatcaatgt cgatcgtggc tggctcgaag atacctgcaa gaatgtcatt 2040
   gcgctgccat tctccaaatt gcagttcgcg cttagctgga taacgccacg gaatgatgtc 2100
   gtcgtgcaca acaatggtga cttctacagc gcggagaatc tcgctctctc caggggaagc 2160
   cgaagtttcc aaaaggtcgt tgatcaaagc tcgccgcgtt gtttcatcaa gccttacggt 2220
   caccgtaacc agcaaatcaa tatcactgtg tggcttcagg ccgccatcca ctgcggagcc 2280
   gtacaaatgt acggccagca acgtcggttc gagatggcgc tcgatgacgc caactacctc 2340
   tgatagttga gtcgatactt cggcgatcac cgcttccctc atactcttcc tttttcaata 2400
   ttattgaagc atttatcagg gttattgtct catgagcgga tacatatttg aatgtattta 2460
   gaaaaataaa caaatagcta gctcactcgg tcgctacgct ccgggcgtga gactgcggcg 2520
   ggcgctgcgg acacatacaa agttacccac agattccgtg gataagcagg ggactaacat 2580
   gtgaggcaaa acagcagggc cgcgccggtg gcgtttttcc ataggctccg ccctcctgcc 2640
   agagttcaca taaacagacg cttttccggt gcatctgtgg gagccgtgag gctcaaccat 2700
   gaatctgaca gtacgggcga aacccgacag gacttaaaga tccccaccgt ttccggcggg 2760
   tcgctccctc ttgcgctctc ctgttccgac cctgccgttt accggatacc tgttccgcct 2820
   ttctccctta cgggaagtgt ggcgctttct catagctcac acactggtat ctcggctcgg 2880
   tgtaggtcgt tcgctccaag ctgggctgta agcaagaact ccccgttcag cccgactgct 2940
   gcgccttatc cggtaactgt tcacttgagt ccaacccgga aaagcacggt aaaacgccac 3000
   tggcagcagc cattggtaac tgggagttcg cagaggattt gtttagctaa acacgcggtt 3060
   gctcttgaag tgtgcgccaa agtccggcta cactggaagg acagatttgg ttgctgtgct 3120
   ctgcgaaagc cagttaccac ggttaagcag ttccccaact gacttaacct tcgatcaaac 3180
   cacctcccca ggtggttttt tcgtttacag ggcaaaagat tacgcgcaga aaaaaaggat 3240
   ctcaagaaga tcctttgatc ttttctactg aaccgctcta gatttcagtg caatttatct 3300
   cttcaaatgt agcacctgaa gtcagcccca tacgatataa gttgtaattc tcatgttagt 3360
   catgccccgc gcccaccgga aggagctgac tgggttgaag gctctcaagg gcatcggtcg 3420
   agatcccggt gcctaatgag tgagctaact tacattaatt gcgttgcgct cactgcccgc 3480
   tttccagtcg ggaaacctgt cgtgccagct gcattaatga atcggccaac gcgcggggag      3540
   aggcggtttg cgtattgggc gccagggtgg tttttctttt caccagtgag acgggcaaca      3600
   gctgattgcc cttcaccgcc tggccctgag agagttgcag caagcggtcc acgctggttt      3660
   gccccagcag gcgaaaatcc tgtttgatgg tggttaacgg cgggatataa catgagctgt      3720
   cttcggtatc gtcgtatccc actaccgaga tgtccgcacc aacgcgcagc ccggactcgg      3780
   taatggcgcg cattgcgccc agcgccatct gatcgttggc aaccagcatc gcagtgggaa      3840
   cgatgccctc attcagcatt tgcatggttt gttgaaaacc ggacatggca ctccagtcgc      3900
   cttcccgttc cgctatcggc tgaatttgat tgcgagtgag atatttatgc cagccagcca      3960
   gacgcagacg cgccgagaca gaacttaatg ggcccgctaa cagcgcgatt tgctggtgac      4020
   ccaatgcgac cagatgctcc acgcccagtc gcgtaccgtc ttcatgggag aaaataatac      4080
   tgttgatggg tgtctggtca gagacatcaa gaaataacgc cggaacatta gtgcaggcag      4140
   cttccacagc aatggcatcc tggtcatcca gcggatagtt aatgatcagc ccactgacgc      4200
   gttgcgcgag aagattgtgc accgccgctt tacaggcttc gacgccgctt cgttctacca      4260
   tcgacaccac cacgctggca cccagttgat cggcgcgaga tttaatcgcc gcgacaattt      4320
   gcgacggcgc gtgcagggcc agactggagg tggcaacgcc aatcagcaac gactgtttgc      4380
   ccgccagttg ttgtgccacg cggttgggaa tgtaattcag ctccgccatc gccgcttcca      4440
   ctttttcccg cgttttcgca gaaacgtggc tggcctggtt caccacgcgg gaaacggtct      4500
   gataagagac accggcatac tctgcgacat cgtataacgt tactggtttc acattcacca      4560
   ccctgaattg actctcttcc gggcgctatc atgccatacc gcgaaaggtt ttgcgccatt      4620
   cgatggtgtc cgggatctcg acgctctccc ttatgcgact cctgcattag gaaattaata      4680
   cgactcacta ta                                                          4692
<210> 8
   <211> 5493
   <212> DNA
   <213> Artificial Sequence
<220>
   <223> pDEST-IgtB
<400> 8
   ttgtacaaag tggtgatgat ccggctgcta acaaagcccg aaaggaagct gagttggctg      60
   ctgccaccgc tgagcaataa ctagcataac cccttggggc ctctaaacgg gtcttgaggg     120
   gttttttgct gaaaggagga actatatccg gatatccaca ggacgggtgt ggtcgccatg    180
   atcgcgtagt cgatagtggc tccaagtagc gaagcgagca ggactgggcg gcggccaaag     240
   cggtcggaca gtgctccgag aacgggtgcg catagaaatt gcatcaacgc atatagcgct     300
   agcagcacgc catagtgact ggcgatgctg tcggaatgga cgatatcccg caagaggccc     360
   ggcagtaccg gcataaccaa gcctatgcct acagcatcca gggtgacggt gccgaggatg     420
   acgatgagcg cattgttaga tttcatacac ggtgcctgac tgcgttagca atttaactgt     480
   gataaactac cgcattaaag cttatcgatg ataagctgtc aaacatgaga attcttgaag     540
   acgaaagggc ctcgtgatac gcctattttt ataggttaat gtcatgataa taatggtttc     600
   ttagacgtca ggtggcactt ttcggggaaa tgtgcgcgga acccctattt gtttattttt     660
   ctaaatacat tcaaatatgt atccgctcat gagacaataa ccctgataaa tgcttcaata     720
   atattgaaaa aggaagagta tgagtattca acatttccgt gtcgccctta ttcccttttt     780
   tgcggcattt tgccttcctg tttttgctca cccagaaacg ctggtgaaag taaaagatgc     840
   tgaagatcag ttgggtgcac gagtgggtta catcgaactg gatctcaaca gcggtaagat     900
   ccttgagagt tttcgccccg aagaacgttt tccaatgatg agcactttta aagttctgct     960
   atgtggcgcg gtattatccc gtgttgacgc cgggcaagag caactcggtc gccgcataca    1020
   ctattctcag aatgacttgg ttgagtactc accagtcaca gaaaagcatc ttacggatgg    1080
   catgacagta agagaattat gcagtgctgc cataaccatg agtgataaca ctgcggccaa    1140
   cttacttctg acaacgatcg gaggaccgaa ggagctaacc gcttttttgc acaacatggg    1200
   ggatcatgta actcgccttg atcgttggga accggagctg aatgaagcca taccaaacga    1260
   cgagcgtgac accacgatgc ctgcagcaat ggcaacaacg ttgcgcaaac tattaactgg    1320
   cgaactactt actctagctt cccggcaaca attaatagac tggatggagg cggataaagt    1380
   tgcaggacca cttctgcgct cggcccttcc ggctggctgg tttattgctg ataaatctgg    1440
   agccggtgag cgtgggtctc gcggtatcat tgcagcactg gggccagatg gtaagccctc    1500
   ccgtatcgta gttatctaca cgacggggag tcaggcaact atggatgaac gaaatagaca    1560
   gatcgctgag ataggtgcct cactgattaa gcattggtaa ctgtcagacc aagtttactc    1620
   atatatactt tagattgatt taaaacttca tttttaattt aaaaggatct aggtgaagat    1680
   cctttttgat aatctcatga ccaaaatccc ttaacgtgag ttttcgttcc actgagcgtc    1740
   agaccccgta gaaaagatca aaggatcttc ttgagatcct ttttttctgc gcgtaatctg    1800
   ctgcttgcaa acaaaaaaac caccgctacc agcggtggtt tgtttgccgg atcaagagct    1860
   accaactctt tttccgaagg taactggctt cagcagagcg cagataccaa atactgtcct    1920
   tctagtgtag ccgtagttag gccaccactt caagaactct gtagcaccgc ctacatacct    1980
   cgctctgcta atcctgttac cagtggctgc tgccagtggc gataagtcgt gtcttaccgg    2040
   gttggactca agacgatagt taccggataa ggcgcagcgg tcgggctgaa cggggggttc    2100
   gtgcacacag cccagcttgg agcgaacgac ctacaccgaa ctgagatacc tacagcgtga    2160
   gctatgagaa agcgccacgc ttcccgaagg gagaaaggcg gacaggtatc cggtaagcgg    2220
   cagggtcgga acaggagagc gcacgaggga gcttccaggg ggaaacgcct ggtatcttta 2280
   tagtcctgtc gggtttcgcc acctctgact tgagcgtcga tttttgtgat gctcgtcagg 2340
   ggggcggagc ctatggaaaa acgccagcaa cgcggccttt ttacggttcc tggccttttg 2400
   ctggcctttt gctcacatgt tctttcctgc gttatcccct gattctgtgg ataaccgtat 2460
   taccgccttt gagtgagctg ataccgctcg ccgcagccga acgaccgagc gcagcgagtc 2520
   agtgagcgag gaagcggaag agcgcctgat gcggtatttt ctccttacgc atctgtgcgg 2580
   tatttcacac cgcatatatg gtgcactctc agtacaatct gctctgatgc cgcatagtta 2640
   agccagtata cactccgcta tcgctacgtg actgggtcat ggctgcgccc cgacacccgc 2700
   caacacccgc tgacgcgccc tgacgggctt gtctgctccc ggcatccgct tacagacaag 2760
   ctgtgaccgt ctccgggagc tgcatgtgtc agaggttttc accgtcatca ccgaaacgcg 2820
   cgaggcagct gcggtaaagc tcatcagcgt ggtcgtgaag cgattcacag atgtctgcct 2880
   gttcatccgc gtccagctcg ttgagtttct ccagaagcgt taatgtctgg cttctgataa 2940
   agcgggccat gttaagggcg gttttttcct gtttggtcac tgatgcctcc gtgtaagggg 3000
   gatttctgtt catgggggta atgataccga tgaaacgaga gaggatgctc acgatacggg 3060
   ttactgatga tgaacatgcc cggttactgg aacgttgtga gggtaaacaa ctggcggtat 3120
   ggatgcggcg ggaccagaga aaaatcactc agggtcaatg ccagcgcttc gttaatacag 3180
   atgtaggtgt tccacagggt agccagcagc atcctgcgat gcagatccgg aacataatgg 3240
   tgcagggcgc tgacttccgc gtttccagac tttacgaaac acggaaaccg aagaccattc 3300
   atgttgttgc tcaggtcgca gacgttttgc agcagcagtc gcttcacgtt cgctcgcgta 3360
   tcggtgattc attctgctaa ccagtaaggc aaccccgcca gcctagccgg gtcctcaacg 3420
   acaggagcac gatcatgcgc acccgtggcc aggacccaac gctgcccgag atgcgccgcg 3480
   tgcggctgct ggagatggcg gacgcgatgg atatgttctg ccaagggttg gtttgcgcat 3540
   tcacagttct ccgcaagaat tgattggctc caattcttgg agtggtgaat ccgttagcga 3600
   ggtgccgccg gcttccattc aggtcgaggt ggcccggctc catgcaccgc gacgcaacgc 3660
   ggggaggcag acaaggtata gggcggcgcc tacaatccat gccaacccgt tccatgtgct 3720
   cgccgaggcg gcataaatcg ccgtgacgat cagcggtcca gtgatcgaag ttaggctggt 3780
   aagagccgcg agcgatcctt gaagctgtcc ctgatggtcg tcatctacct gcctggacag 3840
   catggcctgc aacgcgggca tcccgatgcc gccggaagcg agaagaatca taatggggaa 3900
   ggccatccag cctcgcgtcg cgaacgccag caagacgtag cccagcgcgt cggccgccat 3960
   gccggcgata atggcctgct tctcgccgaa acgtttggtg gcgggaccag tgacgaaggc 4020
   ttgagcgagg gcgtgcaaga ttccgaatac cgcaagcgac aggccgatca tcgtcgcgct 4080
   ccagcgaaag cggtcctcgc cgaaaatgac ccagagcgct gccggcacct gtcctacgag 4140
   ttgcatgata aagaagacag tcataagtgc ggcgacgata gtcatgcccc gcgcccaccg 4200
   gaaggagctg actgggttga aggctctcaa gggcatcggt cgatcgacgc tctcccttat 4260
   gcgactcctg cattaggaag cagcccagta gtaggttgag gccgttgagc accgccgccg 4320
   caaggaatgg tgcatgcaag gagatggcgc ccaacagtcc cccggccacg gggcctgcca 4380
   ccatacccac gccgaaacaa gcgctcatga gcccgaagtg gcgagcccga tcttccccat 4440
   cggtgatgtc ggcgatatag gcgccagcaa ccgcacctgt ggcgccggtg atgccggcca 4500
   cgatgcgtcc ggcgtagagg atcgagatct cgatcccgcg aaattaatac gactcactat 4560
   agggagacca caacggtttc cctctagatc acaagtttgt acaaaaaagc aggcttcgaa 4620
   ggagatagaa ccatggccca ccatcaccat caccatcaaa accacgttat cagcttagct 4680
   tccgccgcag aacgcagggc gcacattgcc gataccttcg gcaggcacgg catcccgttt 4740
   cagtttttcg acgcactgat gccgtctgaa aggctggaac aggcaatggc ggaactcgtc 4800
   cccggcttgt cggcgcaccc ctatttgagc ggagtggaaa aagcctgctt tatgagccac 4860
   gccgtattgt ggaagcaggc attggacgaa ggtctgccgt atatcaccgt atttgaggac 4920
   gacgttttac tcggcgaagg tgcggaaaaa ttccttgccg aagacgcttg gctgcaagaa 4980
   cgctttgacc cggataccgc ctttatcgtc cgcttggaaa cgatgtttat gcacgtcctg 5040
   acctcgccct ccggcgtggc ggattactgc gggcgcgcct ttccgctgtt ggaaagcgaa 5100
   cactggggga cggcgggcta tatcatttcc cgaaaagcga tgcggttttt cctggacagg 5160
   tttgccgccc tgccgcccga agggctgcac cccgtcgatc tgatgatgtt cagcgatttt 5220
   ttcgacaggg aaggaatgcc ggtttgccag ctcaatcccg ccttgtgcgc ccaagagctg 5280
   cattatgcca agtttcacga ccaaaacagc gcattgggca gcctgatcga acacgaccgc 5340
   ctcctgaacc gcaaacagca aaggcgcgat tcccccgcca acacattcaa acaccgcctg 5400
   atccgcgcct tgaccgatat cagcagggaa agggaaaaac gccggcaaag gcgcgaacag 5460
   ttcattgtgc ctttccaata agacccagct ttc 5493
<210> 9
   <211> 4568
   <212> DNA
   <213> Artificial Sequence
<220>
   <223> Ptet-IgtA-PT5-galT-FRT-kanR-FRT
<400> 9
   acaggttggc tgataagtcc ccggtctagc ttgcatgcag attgcagcat tacacgtctt       60
   gagcgattgt gtaggctgga gctgcttcga agttcctata ctttctagag aataggaact      120
   tcggaatagg aacttcattt aaatggcgcg ccttacgccc cgccctgccg gtaccgagag      180
   cgcttttgaa gctggggtgg gcgaagaact ccagcatgag atccccgcgc tggaggatca 240
   tccagccggc gtcccggaaa acgattccga agcccaacct ttcatagaag gcggcggtgg 300
   aatcgaaatc tcgtgatggc aggttgggcg tcgcttggtc ggtcatttcg aaccccagag 360
   tcccgctcag aagaactcgt caagaaggcg atagaaggcg atgcgctgcg aatcgggagc 420
   ggcgataccg taaagcacga ggaagcggtc agcccattcg ccgccaagct cttcagcaat 480
   atcacgggta gccaacgcta tgtcctgata gcggtccgcc acacccagcc ggccacagtc 540
   gatgaatcca gaaaagcggc cattttccac catgatattc ggcaagcagg catcgccatg 600
   ggtcacgacg agatcctcgc cgtcgggcat gcgcgccttg agcctggcga acagttcggc 660
   tggcgcgagc ccctgatgct cttcgtccag atcatcctga tcgacaagac cggcttccat 720
   ccgagtacgt gctcgctcga tgcgatgttt cgcttggtgg tcgaatgggc aggtagccgg 780
   atcaagcgta tgcagccgcc gcattgcatc agccatgatg gatactttct cggcaggagc 840
   aaggtgagat gacaggagat cctgccccgg cacttcgccc aatagcagcc agtcccttcc 900
   cgcttcagtg acaacgtcga gcacagctgc gcaaggaacg cccgtcgtgg ccagccacga 960
   tagccgcgct gcctcgtcct gcagttcatt cagggcaccg gacaggtcgg tcttgacaaa 1020
   aagaaccggg cgcccctgcg ctgacagccg gaacacggcg gcatcagagc agccgattgt 1080
   ctgttgtgcc cagtcatagc cgaatagcct ctccacccaa gcggccggag aacctgcgtg 1140
   caatccatct tgttcaatca tgcgaaacga tcctcatcct gtctcttgat cagatcttga 1200
   tcccctgcgc catcagatcc ttggcggcaa gaaagccatc cagtttactt tgcagggctt 1260
   cccaacctta ccagagggcg ccccagctgg caattccggt tcgcttgctg tccataaaac 1320
   cgcccagtct agctatcgcc atgtaagccc actgcaagct acctgctttc tctttgcgct 1380
   tgcgttttcc cttgtccaga tagcccagta gctgacattc atccggggtc agcaccgttt 1440
   ctgcggactg gctttctacg tgttccgctt cctttagcag cccttgcgcc ctgagtgctt 1500
   gcggcagcgt gaggggatct tgacgcgtgt cacaggtagg acgcgccgaa gttcctatac 1560
   tttctagaga ataggaactt cggaatagga actaaggagg atattcatac atgatggtag 1620
   tgttcgaaat taatacgact cactataggg gaattgattc tggtaccaaa tgagtcgacc 1680
   ggccagatga ttaattccta atttttgttg acactctatc attgatagag ttattttacc 1740
   actccctatc agtgatagag aaaagtgaaa tgaatagttc gacaaaaatc tagaaataat 1800
   tttgtttaac tttaagaagg agatatacaa atgccgtccg aagcattccg tcgtcaccgt 1860
   gcttatcgcg aaaacaaact gcagccactg gtctctgtcc tgatctgcgc atacaacgtt 1920
   gagaaatact tcgcacagtc tctggcagct gtagttaacc agacctggcg taacctggat 1980
   atcctgatcg tagatgacgg ctctacggat ggtacgctgg cgatcgcaca gcgtttccag 2040
   gaacaggacg gtcgtatccg cattctcgct cagccgcgta actctggtct gatcccgtct 2100
   ctgaacatcg gtctggacga actggccaaa tctggtggtg gtggcgaata catcgcccgt 2160
   actgacgccg acgacattgc ggccccggat tggatcgaaa aaatcgtagg tgaaatggag 2220
   aaagaccgct ctatcatcgc gatgggtgct tggctggaag ttctgtccga agagaaagac 2280
   ggtaaccgtc tggcccgtca ccatgaacac ggcaaaatct ggaaaaaacc gacccgtcac 2340
   gaagatatcg cggacttctt cccgttcggt aacccgatcc ataacaacac catgatcatg 2400
   cgtcgtagcg taatcgacgg tggtctgcgt tacaacaccg aacgtgattg ggcagaagac 2460
   taccagtttt ggtatgacgt gtctaaactg ggtcgtctgg cttactaccc agaagcgctg 2520
   gttaaatacc gtctgcacgc caaccaggtt agctccaaat actccatccg tcagcacgaa 2580
   atcgcacagg gtatccagaa aacggctcgt aacgacttcc tgcagtccat gggtttcaaa 2640
   acccgtttcg actctctgga gtaccgtcag atcaaagcgg ttgcgtatga gctgctggag 2700
   aaacacctgc cggaagagga ctttgaacgt gcgcgtcgtt tcctgtacca gtgcttcaaa 2760
   cgtaccgaca ctctgccggc gggtgcatgg ctcgactttg cagcggatgg tcgtatgcgt 2820
   cgtctgttta ccctgcgtca gtacttcggt atcctgcatc gtctcctgaa aaaccgctaa 2880
   tgatttcgtc gacacacagg aaacatatta aaaattaaaa cctgcaggag tttaaacgcg 2940
   gccgcgatat cgttgtaaaa cgacggccag tgcaagaatc ataaaaaatt tatttgcttt 3000
   caggaaaatt tttctgtata atagattcat aaatttgaga gaggagtttt tgtgagcgga 3060
   taacaattcc ccatcttagt atattagtta agtataaata cacaaggaga tataccatga 3120
   cgcaatttaa tcccgttgat catccacatc gccgctacaa cccgctcacc gggcaatgga 3180
   ttctggtttc accgcaccgc gctaagcgcc cctggcaggg ggcgcaggaa acgccagcca 3240
   aacaggtgtt acctgcgcac gatccagatt gcttcctctg cgcaggtaat gtgcgggtga 3300
   caggcgataa aaaccccgat tacaccggga cttacgtttt cactaatgac tttgcggctt 3360
   tgatgtctga cacgccagat gcgccagaaa gtcacgatcc gctgatgcgt tgccagagcg 3420
   cgcgcggcac cagccgggtg atctgctttt caccggatca cagtaaaacg ctgccagagc 3480
   tcagcgttgc agcattgacg gaaatcgtca aaacctggca ggagcaaacc gcagaactgg 3540
   ggaaaacgta cccatgggtg caggtttttg aaaacaaagg cgcggcgatg ggctgctcta 3600
   acccgcatcc gcacggtcag atttgggcaa atagcttcct gcctaacgaa gctgagcgcg 3660
   aagaccgcct gcaaaaagaa tattttgccg aacagaaatc accaatgctg gtggattatg 3720
   ttcagcgcga gctggcagac ggtagccgta ccgttgtcga aaccgaacac tggttagccg 3780
   tcgtgcctta ctgggctgcc tggccgttcg aaacgctact gctgcccaaa gcccacgttt 3840
   tacggatcac cgatttgacc gacgcccagc gcagcgatct ggcgctggcg ttgaaaaagc 3900
   tgaccagtcg ttatgacaac ctcttccagt gctccttccc ctactctatg ggctggcacg 3960
   gcgcgccatt taatggcgaa gagaatcaac actggcagct gcacgcgcac ttttatccgc 4020
   ctctgctgcg ctccgccacc gtacgtaaat ttatggttgg ttatgaaatg ctggcagaga 4080
   cccagcgaga cctgaccgca gaacaggcag cagagcgttt gcgcgcagtc agcgatatcc 4140
   attttcgcga atccggagtg taacgcggag gcgcgccatt taaatcaacc tcagcggtca 4200
   tagctgtttc ctgtgactga gcaataacta gcataacccc ttggggcctc taaacgggtc 4260
   ttgaggggtt ttttgctgaa accaatttgc ctggcggcag tagcgcggtg gtcccacctg 4320
   accccatgcc gaactcagaa gtgaaacgcc gtagcgccga tggtagtgtg gggtctcccc 4380
   atgcgagagt agggaactgc caggcatcaa ataaaacgaa aggctcagtc gaaagactgg 4440
   gcctttcggg atccaggccg gcctgttaac gaattaatct tccgcggcaa caaaaattag 4500
   gaattaatca tctggccaat ttcaggtggc acttttcggg cagaccgggg acttatcagc 4560
   caacctgt 4568
<210> 10
   <211> 6521
   <212> DNA
   <213> Artificial Sequence
<220>
   <223> Ptet-glmUM-PT5-glmS-FRT-dhfr-FRT
<400> 10
   acaggttggc tgataagtcc ccggtctagc ttgcatgcag attgcagcat tacacgtctt      60
   gagcgattgt gtaggctgga gctgcttcga aattaatacg actcactata ggggaattga     120
   ttctggtacc aaatgagtcg accggccaga tgattaattc ctaatttttg ttgacactct     180
   atcattgata gagttatttt accactccct atcagtgata gagaaaagtg aaatgaatag     240
   ttcgacaaaa atctagaaat aattttgttt aactttaaga aggagatata caaatgctga     300
   acaacgcgat gtctgttgtt atcctggcgg cgggtaaagg tacccgtatg tactctgacc     360
   tgccgaaagt tctgcacacc ctggcgggta aagcgatggt tcagcacgtt atcgacgcgg     420
   cgaacgaact gggtgcggcg cacgttcacc tggtttacgg tcacggtggt gacctgctga     480
   aacaggcgct gaaagacgac aacctgaact gggttctgca ggcggaacag ctgggtaccg     540
   gtcacgcgat gcagcaggcg gcgccgttct tcgcggacga cgaagacatc ctgatgctgt     600
   acggtgacgt tccgctgatc tctgttgaaa ccctgcagcg tctgcgtgac gcgaaaccgc     660
   agggtggtat cggtctgctg accgttaaac tggacgaccc gaccggttac ggtcgtatca     720
   cccgtgaaaa cggtaaagta accggtatcg ttgaacacaa agacgcgacc gacgaacagc     780
   gtcagatcca ggagatcaac accggtatcc tgatcgcgaa cggtgcagac atgaaacgtt     840
   ggctggcgaa actgaccaac aacaacgcgc agggtgaata ctacatcacc gacatcatcg     900
   cgctggcgta ccaggaaggt cgtgaaatcg ttgcggttca cccgcagcgt ctgtctgaag 960
   ttgaaggtgt taacaaccgt ctgcagctgt ctcgtctgga acgtgtttac cagtctgaac 1020
   aggcggaaaa actgctgctg gcgggtgtta tgctgcgtga cccggcgcgt ttcgacctgc 1080
   gtggtaccct gacccacggt cgtgacgttg aaatcgacac caacgttatc atcgaaggta 1140
   acgttaccct gggtcaccgt gtaaaaatcg gcaccggttg cgttatcaaa aactctgtta 1200
   tcggtgacga ctgcgaaatc tctccgtaca ccgttgttga agacgcgaac ctggcggcgg 1260
   cgtgcaccat cggtccgttc gcgcgtctgc gtccgggtgc ggaactgctg gaaggtgcgc 1320
   acgttggtaa cttcgttgaa atgaaaaaag cgcgtctggg taaaggttct aaagcgggtc 1380
   acctgaccta cctgggtgac gcggaaatcg gtgacaacgt taacatcggt gcgggtacca 1440
   tcacctgcaa ctacgacggt gcgaacaaat tcaaaaccat catcggtgac gacgttttcg 1500
   ttggttctga cacccagctg gttgcgccgg ttaccgttgg taaaggtgcg accatcgcgg 1560
   cgggtaccac cgttacccgt aacgttggtg aaaacgcgct ggcgatctct cgtgttccgc 1620
   agacccagaa agaaggttgg cgtcgtccgg ttaaaaaaaa ataacgaagg agatagaacc 1680
   atgtccaacc gtaaatactt cggtacggac ggtatccgtg gtcgtgtagg tgatgctccg 1740
   attacgccgg atttcgtcct gaaactcggt tgggcagcgg gtaaagttct cgcacgtcac 1800
   ggctctcgta aaatcatcat cggtaaagac acccgtatct ctggttacat gctcgaatct 1860
   gcactggaag cgggtctggc tgcagctggt ctgtctgcac tgttcacggg tccgatgcca 1920
   accccagctg tagcgtacct gactcgcact ttccgtgcag aagcaggtat cgtgatctct 1980
   gcctctcaca acccgttcta cgacaacggt atcaaattct tcagcatcga tggtaccaaa 2040
   ctcccagacg cggttgaaga ggctatcgaa gcggaaatgg agaaagaaat ctcttgtgta 2100
   gactctgccg aactcggtaa agcgtctcgt atcgttgatg cagcgggtcg ttacatcgag 2160
   ttctgcaaag ccacctttcc gaacgaactg agcctgtctg agctgaaaat cgtcgtagac 2220
   tgtgccaacg gtgcgactta ccacattgcc ccaaacgtac tgcgtgagct gggtgctaac 2280
   gtcatcgcga tcggttgtga accgaacggt gtcaacatca acgcggaagt aggtgcgacc 2340
   gatgttcgtg cactgcaggc tcgtgtactc gcggagaaag cggatctcgg tatcgccttt 2400
   gacggtgatg gtgaccgtgt tatcatggtt gaccacgaag gtaacaaagt ggatggtgac 2460
   cagatcatgt acatcattgc ccgtgaaggt ctgcgtcagg gtcagctgcg tggtggtgca 2520
   gtaggtaccc tcatgagcaa catgggtctg gaactggccc tgaaacagct gggtatccca 2580
   ttcgctcgtg ctaaagtagg cgaccgttac gttctggaga aaatgcagga gaaaggttgg 2640
   cgtatcggtg ccgaaaactc tggtcacgtc atcctgctgg acaaaaccac taccggtgac 2700
   ggtatcgtag caggtctgca ggtactcgcc gctatggccc gtaaccacat gtccctccat 2760
   gacctctgct ctggtatgaa aatgttcccg cagatcctgg ttaacgttcg ttacaccgca 2820
   ggttctggtg atccgctgga acacgagtct gtgaaagccg ttaccgcaga agtggaagcg 2880
   gccctgggta accgtggtcg tgtactgctg cgtaaatccg gtactgagcc actgatccgt 2940
   gttatggttg agggcgaaga tgaagcccag gtcaccgaat ttgcgcaccg tattgccgac 3000
   gcagtcaaag cggtttaatt tcgtcgacac acaggaaaca tattaaaaat taaaacctgc 3060
   aggagtttaa acgcggccgc gatatcgttg taaaacgacg gccagtgcaa gaatcataaa 3120
   aaatttattt gctttcagga aaatttttct gtataataga ttcataaatt tgagagagga 3180
   gtttttgtga gcggataaca attccccatc ttagtatatt agttaagtat aaatacacaa 3240
   ggagatatac atatgtgcgg tatcgttggt gctatcgcac agcgtgatgt agcggagatc 3300
   ctcctggaag gtctgcgtcg tctcgaatac cgtggttacg actctgccgg tctggcagta 3360
   gtggatgcag aaggtcacat gactcgtctg cgtcgtctgg gtaaagtgca gatgctcgcg 3420
   caggcggcgg aagaacaccc actccacggt ggtacgggta tcgcacacac tcgttgggca 3480
   acccacggtg aaccgtctga ggtcaacgca cacccgcatg ttagcgagca catcgtagtc 3540
   gttcacaacg gtatcatcga gaaccacgaa ccactccgtg aggaactcaa agcccgtggt 3600
   tacaccttcg taagcgaaac cgacacggaa gttatcgccc acctcgttaa ctgggaactc 3660
   aaacagggtg gtactctgcg tgaagcagtt ctgcgtgcca ttccacagct gcgtggtgca 3720
   tacggtaccg tgatcatgga ctctcgtcat ccggataccc tgctcgccgc acgttctggt 3780
   tctccactcg ttatcggtct gggtatgggt gagaacttca tcgcctctga tcagctggcc 3840
   ctgctcccag ttacccgtcg cttcatcttc ctggaagagg gtgacatcgc cgaaatcacc 3900
   cgtcgttccg ttaacatctt cgacaaaacg ggtgcggaag ttaaacgtca ggacatcgag 3960
   tctaacctgc agtatgacgc tggtgacaaa ggcatctacc gtcactacat gcagaaagag 4020
   atctacgaac agccgaacgc gatcaaaaac accctgaccg gtcgtatctc tcacggtcag 4080
   gttgacctgt ctgagctggg tccaaacgcg gacgaactcc tgtccaaagt cgagcacatc 4140
   cagatcctgg cttgtggtac ctcttacaac tccggtatgg tttctcgtta ctggttcgaa 4200
   tctctggcag gtatcccatg cgacgttgaa atcgcctccg aattccgtta tcgtaaatct 4260
   gcggtacgtc gtaactccct catgatcacc ctgtctcagt ctggtgaaac cgctgatact 4320
   ctggcaggtc tgcgtctcag caaagaactg ggttacctgg gttctctggc catctgcaac 4380
   gttccgggtt ctagcctggt tcgtgagtct gacctggctc tgatgaccaa cgcgggtacg 4440
   gagatcggtg ttgcctctac caaagcgttc actacccagc tcactgtcct gctgatgctg 4500
   gttgccaaac tgtctcgtct caaaggcctc gacgctagca tcgaacacga catcgtacac 4560
   ggtctgcagg ccctcccatc tcgtatcgag cagatgctgt ctcaggacaa acgtatcgaa 4620
   gcactggcag aagacttcag cgacaaacac cacgcgctgt ttctgggtcg tggtgaccag 4680
   tacccaattg cgctggaagg tgccctgaaa ctgaaagaga tcagctacat ccatgcagag    4740
   gcatacgcag cgggtgagct gaaacatggt ccactggccc tgatcgacgc agatatgccg    4800
   gttattgtgg ttgctccgaa caacgaactg ctggagaaac tgaaatccaa catcgaggaa    4860
   gtacgtgcgc gtggtggtca gctgtacgtg tttgctgacc aggacgcggg tttcgtttcc    4920
   agcgacaaca tgcacatcat cgaaatgccg catgttgaag aggtaatcgc gccaatcttc    4980
   tacaccgtac cgctgcagct gctggcgtac catgtagccc tgatcaaagg tacggacgtt    5040
   gaccagccgc gtaacctggc gaaatccgtg accgtggaat aacgcggagg cgcgccattt    5100
   aaatcaacct cagcggtcat agctgtttcc tgtgactgag caataactag cataacccct    5160
   tggggcctct aaacgggtct tgaggggttt tttgctgaaa ccaatttgcc tggcggcagt    5220
   agcgcggtgg tcccacctga ccccatgccg aactcagaag tgaaacgccg tagcgccgat    5280
   ggtagtgtgg ggtctcccca tgcgagagta gggaactgcc aggcatcaaa taaaacgaaa    5340
   ggctcagtcg aaagactggg cctttcggga tccaggccgg cctgttaacg aattaatctt    5400
   ccgcggcggt atcgataagc ttgatatcga attccgaagt tcctattctc tagaaagtat    5460
   aggaacttca ggtctgaaga ggagtttacg tccagccaag ctagcttggc tgcaggtcgt    5520
   cgaaattcta ccgggtaggg gaggcgcttt tcccaaggca gtctggagca tgcgctttag    5580
   cagccccgct gggcacttgg cgctacacaa gtggcctctg gcctcgcaca cattccacat    5640
   ccaccggtag gcgccaaccg gctccgttct ttggtggccc cttcgcgcca ccttctactc    5700
   ctcccctagt caggaagttc ccccccgccc cgcagctcgc gtcgtgcagg acgtgacaaa    5760
   tggaagtagc acgtctcact agtctcgtgc agatggacag caccgctgag caatggaagc    5820
   gggtaggcct ttggggcagc ggccaatagc agctttgctc cttcgctttc tgggctcaga    5880
   ggctgggaag gggtgggtcc gggggcgggc tcaggggcgg gctcaggggc ggggcgggcg    5940
   cccgaaggtc ctccggaggc ccggcattct gcacgcttca aaagcgcacg tctgccgcgc    6000
   tgttctcctc ttcctcatct ccgggccttt cgacctgcag cctgttgaca attaatcatc    6060
   ggcatagtat atcggcatag tataatacga caaggtgagg aactaaacca tgggtcaaag    6120
   tagcgatgaa gccaacgctc ccgttgcagg gcagtttgcg cttcccctga gtgccacctt    6180
   tggcttaggg gatcgcgtac gcaagaaatc tggtgccgct tggcagggtc aagtcgtcgg    6240
   ttggtattgc acaaaactca ctcctgaagg ctatgcggtc gagtccgaat cccacccagg    6300
   ctcagtgcaa atttatcctg tggctgcact tgaacgtgtg gcctaatgag gggatcaatt    6360
   ctctagagct cgctgatcag aagttcctat tctctagaaa gtataggaac ttcgatggcg    6420
   cctcatccct gaagccaata caacaaaaat taggaattaa tcatctggcc aatttcaggt    6480
   ggcacttttc gggcagaccg gggacttatc agccaacctg t                        6521
<210> 11
   <211> 4187
   <212> DNA
   <213> Artificial Sequence
<220>
   <223> PT5-lgtB-FRT-tetR-FRT
<400> 11
   cacaggttgg ctgataagtc cccggtctag cttgcatgca gattgcagca ttacacgtct      60
   tgagcgattg tgtaggctgg agctgctcaa gcccgtcagg gcgcgtcagc gggtgttggc     120
   gggtgtcggg gctggcttaa ctatgcggca tcagagcaga ttgtactgag agtgcaccat     180
   atgcggtgtg aaataccgca cagatgcgta aggagaaaat accgcatcag gcgccattcg     240
   ccattcaggc tgcgcaactg ttgggaaggg cgatcggtgc gggcctcttc gctattacgc     300
   cagctggcga aagggggatg tgctgcaagg cgattaagtt gggtaacgcc agggttttcc     360
   cagtcacgac gttgtaaaac gacggccagt gaattcgagc tcggtacctc gcgaatgcat     420
   ctagattgta aaacgacggc cagtgaattc ccttagggat aacagggtaa ttacggcccc     480
   aaggtccaaa cggtgaaggt accgggcccc ccctcgaggt cgaggtaccc aaatatgcat     540
   aatcgaaatt aatacgactc actatagggg aattgattct ggtaccaaat gagtcgaccg     600
   gccagatgat taattcctaa tttttgttga cactctatca ttgatagagt tattttacca     660
   ctccctatca gtgatagaga aaagtgaaat gaatagttcg acaaaaatct agaaataatt     720
   ttgtttaact ttaagaagga gatatacaat ttcgtcgaca cacaggaaac atattaaaaa     780
   ttaaaacctg caggagttta aacgcggccg cgatatcgtt gtaaaacgac ggccagtgca     840
   agaatcataa aaaatttatt tgctttcagg aaaatttttc tgtataatag attcataaat     900
   ttgagagagg agtttttgtg agcggataac aattccccat cttagtatat tagttaagta     960
   taaatacacc gcggaggcgt cgaaggagat acaaccatgc aaaaccacgt tatcagctta    1020
   gcttccgccg cagaacgcag ggcgcacatt gccgatacct tcggcaggca cggcatcccg    1080
   tttcagtttt tcgacgcact gatgccgtct gaaaggctgg aacaggcaat ggcggaactc    1140
   gtccccggct tgtcggcgca cccctatttg agcggagtgg aaaaagcctg ctttatgagc    1200
   cacgccgtat tgtggaagca ggcattggac gaaggtctgc cgtatatcac cgtatttgag    1260
   gacgacgttt tactcggcga aggtgcggaa aaattccttg ccgaagacgc ttggctgcaa    1320
   gaacgctttg acccggatac cgcctttatc gtccgcttgg aaacgatgtt tatgcacgtc    1380
   ctgacctcgc cctccggcgt ggcggattac tgcgggcgcg cctttccgct gttggaaagc    1440
   gaacactggg ggacggcggg ctatatcatt tcccgaaaag cgatgcggtt tttcctggac    1500
   aggtttgccg ccctgccgcc cgaagggctg caccccgtcg atctgatgat gttcagcgat    1560
   tttttcgaca gggaaggaat gccggtttgc cagctcaatc ccgccttgtg cgcccaagag    1620
   ctgcattatg ccaagtttca cgaccaaaac agcgcattgg gcagcctgat cgaacacgac 1680
   cgcctcctga accgcaaaca gcaaaggcgc gattcccccg ccaacacatt caaacaccgc 1740
   ctgatccgcg ccttgaccga tatcagcagg gaaagggaaa aacgccggca aaggcgcgaa 1800
   cagttcattg tgcctttcca ataacgccat ttaaatcaac ctcagcggtc atagctgttt 1860
   cctgtgactg agcaataact agcataaccc cttggggcct ctaaacgggt cttgaggggt 1920
   tttttgctga aaccaatttg cctggcggca gtagcgcggt ggtcccacct gaccccatgc 1980
   cgaactcaga agtgaaacgc cgtagcgccg atggtagtgt ggggtctccc catgcgagag 2040
   tagggaactg ccaggcatca aataaaacga aaggctcagt cgaaagactg ggcctttcgg 2100
   gatccaggcc ggcctgttaa cgaattaatc ttccgcggcg gtatcgataa gcttgatatc 2160
   gacactacca tcatgtatga atatcctcct tagttcctat tccgaagttc ctattctcta 2220
   gaaagtatag gaacttcggc gacgtctaag aaaccattat tatcatgaca ttaacctata 2280
   aaaataggcg tatcacgagg ccctttcgtc ttcaagaatt ctcatgtttg acagcttatc 2340
   atcgataagc tttaatgcgg tagtttatca cagttaaatt gctaacgcag tcaggcaccg 2400
   tgtatgaaat ctaacaatgc gctcatcgtc atcctcggca ccgtcaccct ggatgctgta 2460
   ggcataggct tggttatgcc ggtactgccg ggcctcttgc gggatatcgt ccattccgac 2520
   agcatcgcca gtcactatgg cgtgctgcta gcgctatatg cgttgatgca atttctatgc 2580
   gcacccgttc tcggagcact gtccgaccgc tttggccgcc gcccagtcct gctcgcttcg 2640
   ctacttggag ccactatcga ctacgcgatc atggcgacca cacccgtcct gtggatcctc 2700
   tacgccggac gcatcgtggc cggcatcacc ggcgccacag gtgcggttgc tggcgcctat 2760
   atcgccgaca tcaccgatgg ggaagatcgg gctcgccact tcgggctcat gagcgcttgt 2820
   ttcggcgtgg gtatggtggc aggccccgtg gccgggggac tgttgggcgc catctccttg 2880
   catgcaccat tccttgcggc ggcggtgctc aacggcctca acctactact gggctgcttc 2940
   ctaatgcagg agtcgcataa gggagagcgt cgaccgatgc ccttgagagc cttcaaccca 3000
   gtcagctcct tccggtgggc gcggggcatg actatcgtcg ccgcacttat gactgtcttc 3060
   tttatcatgc aactcgtagg acaggtgccg gcagcgctct gggtcatttt cggcgaggac 3120
   cgctttcgct ggagcgcgac gatgatcggc ctgtcgcttg cggtattcgg aatcttgcac 3180
   gccctcgctc aagccttcgt cactggtccc gccaccaaac gtttcggcga gaagcaggcc 3240
   attatcgccg gcatggcggc cgacgcgctg ggctacgtct tgctggcgtt cgcgacgcga 3300
   ggctggatgg ccttccccat tatgattctt ctcgcttccg gcggcatcgg gatgcccgcg 3360
   ttgcaggcca tgctgtccag gcaggtagat gacgaccatc agggacagct tcaaggatcg 3420
   ctcgcggctc ttaccagcct aacttcgatc attggaccgc tgatcgtcac ggcgatttat 3480
   gccgcctcgg cgagcacatg gaacgggttg gcatggattg taggcgccgc cctatacctt 3540
   gtctgcctcc ccgcgttgcg tcgcggtgca tggagccggg ccacctcgac ctgagtggca 3600
   gggcggggcg taaggcgcgc catttaaatg aagttcctat tccgaagttc ctattctcta 3660
   gaaagtatag gaacttcgaa gcagctccag cctacacaat cgctcaagac gtgtaatgct 3720
   gcaatctgca tgcaagcttg gcactgggat ggcgcctcat ccctgaagcc aatagggata 3780
   acagggtaat aagcttggcg taatcatgtc atgatcggat cccgggcccg tcgactgcag 3840
   aggcctgcat gcaagcttgg cgtaatcatg gtcatagctg tttcctgtgt gaaattgtta 3900
   tccgctcaca attccacaca acatacgagc cggaagcata aagtgtaaag cctggggtgc 3960
   ctaatgagtg agctaactca cattaattgc gttgcgctca ctgcccgctt tccagtcggg 4020
   aaacctgtcg tgccagctgc attaatgaat cggccaacgc gcggggagag gcggtttgcg 4080
   tattgggcgc tcttccgctt cctcgccaac aaaaattagg aattaatcat ctggccaatt 4140
   tcaggtggca cttttcgggc agaccgggga cttatcagcc aacctgt 4187
<210> 12
   <211> 2836
   <212> DNA
   <213> Artificial Sequence
<220>
   <223> Pt5-galE-FRT-cat-FRT
<400> 12
   agtttaaacg cggccgcgat atcgttgtaa aacgacggcc agtgcaagaa tcataaaaaa      60
   tttatttgct ttcaggaaaa tttttctgta taatagattc ataaatttga gagaggagtt     120
   tttgtgagcg gataacaatt ccccatctta gtatattagt taagtataaa tacaccgcgg     180
   aggcgtcgaa ggagatacaa ccatgagagt tctggttacc ggtggtagcg gttacattgg     240
   aagtcatacc tgtgtgcaat tactgcaaaa cggtcatgat gtcatcattc ttgataacct     300
   ctgtaacagt aagcgcagcg tactgcctgt tatcgagcgt ttaggcggca aacatccaac     360
   gtttgttgaa ggcgatattc gtaacgaagc gttgatgacc gagatcctgc acgatcacgc     420
   tatcgacacc gtgatccact tcgccgggct gaaagccgtg ggcgaatcgg tacaaaaacc     480
   gctggaatat tacgacaaca atgtcaacgg cactctgcgc ctgattagcg ccatgcgcgc     540
   cgctaacgtc aaaaacttta tttttagctc ctccgccacc gtttatggcg atcagcccaa     600
   aattccatac gttgaaagct tcccgaccgg cacaccgcaa agcccttacg gcaaaagcaa     660
   gctgatggtg gaacagatcc tcaccgatct gcaaaaagcc cagccggact ggagcattgc     720
   cctgctgcgc tacttcaacc cggttggcgc gcatccgtcg ggcgatatgg gcgaagatcc     780
   gcaaggcatt ccgaataacc tgatgccata catcgcccag gttgctgtag gccgtcgcga     840
   ctcgctggcg atttttggta acgattatcc gaccgaagat ggtactggcg tacgcgatta     900
   catccacgta atggatctgg cggacggtca cgtcgtggcg atggaaaaac tggcgaacaa 960
   gccaggcgta cacatctaca acctcggcgc tggcgtaggc aacagcgtgc tggacgtggt 1020
   taatgccttc agcaaagcct gcggcaaacc ggttaattat cattttgcac cgcgtcgcga 1080
   gggcgacctt ccggcctact gggcggacgc cagcaaagcc gaccgtgaac tgaactggcg 1140
   cgtaacgcgc acactcgatg aaatggcgca ggacacctgg cactggcagt cacgccatcc 1200
   acagggatat cccgattaac gccatttaaa tcaacctcag cggtcatagc tgtttcctgt 1260
   gactgagcaa taactagcat aaccccttgg ggcctctaaa cgggtcttga ggggtttttt 1320
   gctgaaacca atttgcctgg cggcagtagc gcggtggtcc cacctgaccc catgccgaac 1380
   tcagaagtga aacgccgtag cgccgatggt agtgtggggt ctccccatgc gagagtaggg 1440
   aactgccagg catcaaataa aacgaaaggc tcagtcgaaa gactgggcct ttcgggatcc 1500
   aggccggcct gttaacgaat taatcttccg cggcggtatc gataagcttg atatcgaggc 1560
   tgacatggga attagccatg gtccatatga atatcctcct tagttcctat tccgaagttc 1620
   ctattctcta gaaagtatag gaacttcggc gcgcctacct gtgacggaag atcacttcgc 1680
   agaataaata aatcctggtg tccctgttga taccgggaag ccctgggcca acttttggcg 1740
   aaaatgagac gttgatcggc acgtaagagg ttccaacttt caccataatg aaataagatc 1800
   actaccgggc gtattttttg agttgtcgag attttcagga gctaaggaag ctaaaatgga 1860
   gaaaaaaatc actggatata ccaccgttga tatatcccaa tggcatcgta aagaacattt 1920
   tgaggcattt cagtcagttg ctcaatgtac ctataaccag accgttcagc tggatattac 1980
   ggccttttta aagaccgtaa agaaaaataa gcacaagttt tatccggcct ttattcacat 2040
   tcttgcccgc ctgatgaatg ctcatccgga attacgtatg gcaatgaaag acggtgagct 2100
   ggtgatatgg gatagtgttc acccttgtta caccgttttc catgagcaaa ctgaaacgtt 2160
   ttcatcgctc tggagtgaat accacgacga tttccggcag tttctacaca tatattcgca 2220
   agatgtggcg tgttacggtg aaaacctggc ctatttccct aaagggttta ttgagaatat 2280
   gtttttcgtc tcagccaatc cctgggtgag tttcaccagt tttgatttaa acgtggccaa 2340
   tatggacaac ttcttcgccc ccgttttcac catgggcaaa tattatacgc aaggcgacaa 2400
   ggtgctgatg ccgctggcga ttcaggttca tcatgccgtt tgtgatggct tccatgtcgg 2460
   cagatgctta atgaatacaa cagtactgcg atgagtggca gggcggggcg taaggcgcgc 2520
   catttaaatg aagttcctat tccgaagttc ctattctcta gaaagtatag gaacttcgaa 2580
   gcagctccag cctacacaat cgctcaagac gtgtaatgct gcaatctgca tgcaagcttg 2640
   gcactggcga tggcgcctca tccctgaagc caatagggat aacagggtaa tgatcggatc 2700
   ccgggcccgt cgactgcaga ggcctgcatg caagcttggc gtaatcatgg tcatagctgt 2760
   ttcctgtgtg aaattgttat ccgctcacaa ttccacacaa catacgagcc ggaagcataa 2820
   agtgtaaagc ctgggg 2836
<210> 13
   <211> 6770
   <212> DNA
   <213> Artificial Sequence
<220>
   <223> pINT-malE-lgtB
<400> 13
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgaagatcct ttgatctttt 420
   ctacggggtc tgacgctcag tggaacgaaa actcacgtta agggattttg gtcatgagat 480
   tatcaaaaag gatcttcacc tagatccttt taaactagtg aagttaccat cacggaaaaa 540
   ggttatgctg cttttaagac ccactttcac atttaagttg tttttctaat ccgcatatga 600
   tcaattcaag gccgaataag aaggctggct ctgcaccttg gtgatcaaat aattcgatag 660
   cttgtcgtaa taatggcggc atactatcag tagtaggtgt ttccctttct tctttagcga 720
   cttgatgctc ttgatcttcc aatacgcaac ctaaagtaaa atgccccact gcgctgagtg 780
   catataatgc attctctagt gaaaaacctt gttggcataa aaaggctaat tgattttcga 840
   gagtttcata ctgtttttct gtaggccgtg tacctaaatg tacttttgct ccatcgcgat 900
   gacttagtaa agcacatcta aaacttttag cgttattacg taaaaaatct tgccagcttt 960
   ccccttctaa agggcaaaag tgagtatggt gcctatctaa catctcaatg gctaaggcgt 1020
   cgagcaaagc ccgcttattt tttacatgcc aatacaatgt aggctgctct acacctagct 1080
   tctgggcgag tttacgggtt gttaaacctt cgattccgac ctcattaagc agctctaatg 1140
   cgctgttaat cactttactt ttatctaaac gagacatact cttccttttt caatattatt 1200
   gaagcattta tcagggttat tgtctcatga gcggatacat atttgaatgt atttagaaaa 1260
   ataaacaaat aggggttccg cgcacatttc cccgaaaagt gccacctgaa attggccaga 1320
   tgattaattc ctaatttttg ttgacactct atcattgata gagttatttt accactccct 1380
   atcagtgata gagaaaagtg aaatgaatag ttcgacaaaa atctagaaat aattttgttt 1440
   aactttaaga aggagatata caaatgaaaa tcgaagaagg taaactggta atctggatta 1500
   acggcgataa aggctataac ggtctcgctg aagtcggtaa gaaattcgag aaagataccg 1560
   gaattaaagt caccgttgag catccggata aactggaaga gaaattccca caggttgcgg 1620
   caactggcga tggccctgac attatcttct gggcacacga ccgctttggt ggctacgctc 1680
   aatctggcct gttggctgaa atcaccccgg acaaagcgtt ccaggacaag ctgtatccgt 1740
   ttacctggga tgccgtacgt tacaacggca agctgattgc ttacccgatc gctgttgaag 1800
   cgttatcgct gatttataac aaagatctgc tgccgaaccc gccaaaaacc tgggaagaga 1860
   tcccggcgct ggataaagaa ctgaaagcga aaggtaagag cgcgctgatg ttcaacctgc 1920
   aagaaccgta cttcacctgg ccgctgattg ctgctgacgg gggttatgcg ttcaagtatg 1980
   aaaacggcaa gtacgacatt aaagacgtgg gcgtggataa cgctggcgcg aaagcgggtc 2040
   tgaccttcct ggttgacctg attaaaaaca aacacatgaa tgcagacacc gattactcca 2100
   tcgcagaagc tgcctttaat aaaggcgaaa cagcgatgac catcaacggc ccgtgggcat 2160
   ggtccaacat cgacaccagc aaagtgaatt atggtgtaac ggtactgccg accttcaagg 2220
   gtcaaccatc caaaccgttc gttggcgtgc tgagcgcagg tattaacgcc gccagtccga 2280
   acaaagagct ggcaaaagag ttcctcgaaa actatctgct gactgatgaa ggtctggaag 2340
   cggttaataa agacaaaccg ctgggtgccg tagcgctgaa gtcttacgag gaagagttgg 2400
   cgaaagatcc acgtattgcc gccactatgg aaaacgccca gaaaggtgaa atcatgccga 2460
   acatcccgca gatgtccgct ttctggtatg ccgtgcgtac tgcggtgatc aacgccgcca 2520
   gcggtcgtca gactgtcgat gaagccctga aagacgcgca gactatggcc caaaaccacg 2580
   ttatcagctt agcttccgcc gcagaacgca gggcgcacat tgccgatacc ttcggcaggc 2640
   acggcatccc gtttcagttt ttcgacgcac tgatgccgtc tgaaaggctg gaacaggcaa 2700
   tggcggaact cgtccccggc ttgtcggcgc acccctattt gagcggagtg gaaaaagcct 2760
   gctttatgag ccacgccgta ttgtggaagc aggcattgga cgaaggtctg ccgtatatca 2820
   ccgtatttga ggacgacgtt ttactcggcg aaggtgcgga aaaattcctt gccgaagacg 2880
   cttggctgca agaacgcttt gacccggata ccgcctttat cgtccgcttg gaaacgatgt 2940
   ttatgcacgt cctgacctcg ccctccggcg tggcggatta ctgcgggcgc gcctttccgc 3000
   tgttggaaag cgaacactgg gggacggcgg gctatatcat ttcccgaaaa gcgatgcggt 3060
   ttttcctgga caggtttgcc gccctgccgc ccgaagggct gcaccccgtc gatctgatga 3120
   tgttcagcga ttttttcgac agggaaggaa tgccggtttg ccagctcaat cccgccttgt 3180
   gcgcccaaga gctgcattat gccaagtttc acgaccaaaa cagcgcattg ggcagcctga 3240
   tcgaacacga ccgcctcctg aaccgcaaac agcaaaggcg cgattccccc gccaacacat 3300
   tcaaacaccg cctgatccgc gccttgaccg atatcagcag ggaaagggaa aaacgccggc 3360
   aaaggcgcga acagttcatt gtgcctttcc aataagcggc cgcgtcgaca cgcaaaaagg 3420
   ccatccgtca ggatggcctt ctgcttaatt tgatgcctgg cagtttatgg cgggcgtcct    3480
   gcccgccacc ctccgggccg ttgcttcgca acgttcaaat ccgctcccgg cggatttgtc    3540
   ctactcagga gagcgttcac cgacaaacaa cagataaaac gaaaggccca gtctttcgac    3600
   tgagcctttc gttttatttg atgcctggca gttccctact ctcgcatggg gagaccccac    3660
   actaccatca tgtatgaata tcctccttag ttcctattcc gaagggtaat ggcatcaggg    3720
   aatggcgaac gcgctcccca cactaccatc atgtatgaat atcctcctta gttcctattc    3780
   cgaagttcct attctctaga aagtatagga acttcggtgg aacgacgcgt aactcacgtt    3840
   aagggatttt ggtcatgatc agcacgtgtt gacaattaat catcggcata gtatatcggc    3900
   atagtataat acgacaaggt gaggaactaa accatggcca agttgaccag tgccgttccg    3960
   gtgctcaccg cgcgcgacgt cgccggagcg gtcgagttct ggaccgaccg gctcgggttc    4020
   tcccgggact tcgtggagga cgacttcgcc ggtgtggtcc gggacgacgt gaccctgttc    4080
   atcagcgcgg tccaggacca ggtggtgccg gacaacaccc tggcctgggt gtgggtgcgc    4140
   ggcctggacg agctgtacgc cgagtggtcg gaggtcgtgt ccacgaactt ccgggacgcc    4200
   tccgggccgg ccatgaccga gatcggcgag cagccgtggg ggcgggagtt cgccctgcgc    4260
   gacccggccg gcaactgcgt gcacttcgtg gccgaggagc aggactgagt ggcagggcgg    4320
   ggcgtaaggc gcgccattta aatgaagttc ctattccgaa gttcctattc tctagaaagt    4380
   ataggaactt cgaagcagct ccagcctaca caatcgctca agacggaacc cgcgcttggc    4440
   aggaaagtaa tagggatagc agctccagcc tacacaatcg ctcaagacgt gtaatgctgc    4500
   acaataaccc tgctgcagag gcctgcatgc aagcttggcg taatcatggt catagctgtt    4560
   tcctgtgtga aattgttatc cgctcacaat tccacacaac atacgagccg gaagcataaa    4620
   gtgtaaagcc tggggtgcct aatgagtgag ctaactcaca ttaattgcgt tgcgctcact    4680
   gcccgctttc cagtcgggaa acctgtcgtg ccagctgcat taatgaatcg gccaacgcgc    4740
   ggggagaggc ggtttgcgta ttgggcgctc ttccgcttcc tcgctcactg actcgctgcg    4800
   ctcggtcgtt cggctgcggc gagcggtatc agctcactca aaggcggtaa tacggttatc    4860
   cacagaatca ggggataacg caggaaagaa catgtgagca aaaggccagc aaaaggccag    4920
   gaaccgtaaa aaggccgcgt tgctggcgtt tttccatagg ctccgccccc ctgacgagca    4980
   tcacaaaaat cgacgctcaa gtcagaggtg gcgaaacccg acaggactat aaagatacca    5040
   ggcgtttccc cctggaagct ccctcgtgcg ctctcctgtt ccgaccctgc cgcttaccgg    5100
   atacctgtcc gcctttctcc cttcgggaag cgtggcgctt tctcatagct cacgctgtag    5160
   gtatctcagt tcggtgtagg tcgttcgctc caagctgggc tgtgtgcacg aaccccccgt    5220
   tcagcccgac cgctgcgcct tatccggtaa ctatcgtctt gagtccaacc cggtaagaca    5280
   cgacttatcg ccactggcag cagccactgg taacaggatt agcagagcga ggtatgtagg     5340
   cggtgctaca gagttcttga agtggtggcc taactacggc tacactagaa gaacagtatt     5400
   tggtatctgc gctctgctga agccagttac cttcggaaaa agagttggta gctcttgatc     5460
   cggcaaacaa accaccgctg gtagcggtgg tttttttgtt tgcaagcagc agattacgcg     5520
   cagaaaaaaa ggatctcaag aagatccttt gatcttttct acggggtctg acgctcagtg     5580
   gaacgaaaac tcacgttaag ggattttggt catgagatta tcaaaaagga tcttcaccta     5640
   gatcctttta aattaaaaat gaagttttaa atcaatctaa agtatatatg agtaaacttg     5700
   gtctgacagt taccaatgct taatcagtga ggcacctatc tcagcgatct gtctatttcg     5760
   ttcatccata gttgcctgac tccccgtcgt gtagataact acgatacggg agggcttacc     5820
   atctggcccc agtgctgcaa tgataccgcg agacccacgc tcaccggctc cagatttatc     5880
   agcaataaac cagccagccg gaagggccga gcgcagaagt ggtcctgcaa ctttatccgc     5940
   ctccatccag tctattaatt gttgccggga agctagagta agtagttcgc cagttaatag     6000
   tttgcgcaac gttgttgcca ttgctacagg catcgtggtg tcacgctcgt cgtttggtat     6060
   ggcttcattc agctccggtt cccaacgatc aaggcgagtt acatgatccc ccatgttgtg     6120
   caaaaaagcg gttagctcct tcggtcctcc gatcgttgtc agaagtaagt tggccgcagt     6180
   gttatcactc atggttatgg cagcactgca taattctctt actgtcatgc catccgtaag     6240
   atgcttttct gtgactggtg agtactcaac caagtcattc tgagaatagt gtatgcggcg     6300
   accgagttgc tcttgcccgg cgtcaatacg ggataatacc gcgccacata gcagaacttt     6360
   aaaagtgctc atcattggaa aacgttcttc ggggcgaaaa ctctcaagga tcttaccgct     6420
   gttgagatcc agttcgatgt aacccactcg tgcacccaac tgatcttcag catcttttac     6480
   tttcaccagc gtttctgggt gagcaaaaac aggaaggcaa aatgccgcaa aaaagggaat     6540
   aagggcgaca cggaaatgtt gaatactcat actcttcctt tttcaatatt attgaagcat     6600
   ttatcagggt tattgtctca tgagcggata catatttgaa tgtatttaga aaaataaaca     6660
   aataggggtt ccgcgcacat ttccccgaaa agtgccacct gacgtctaag aaaccattat     6720
   tatcatgaca ttaacctata aaaataggcg tatcacgagg ccctttcgtc                6770
<210> 14
   <211> 6788
   <212> DNA
   <213> Artificial Sequence
<220>
   <223> pINT-malE-lexl
<400> 14
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgaagatcct ttgatctttt 420
   ctacggggtc tgacgctcag tggaacgaaa actcacgtta agggattttg gtcatgagat 480
   tatcaaaaag gatcttcacc tagatccttt taaactagtg aagttaccat cacggaaaaa 540
   ggttatgctg cttttaagac ccactttcac atttaagttg tttttctaat ccgcatatga 600
   tcaattcaag gccgaataag aaggctggct ctgcaccttg gtgatcaaat aattcgatag 660
   cttgtcgtaa taatggcggc atactatcag tagtaggtgt ttccctttct tctttagcga 720
   cttgatgctc ttgatcttcc aatacgcaac ctaaagtaaa atgccccact gcgctgagtg 780
   catataatgc attctctagt gaaaaacctt gttggcataa aaaggctaat tgattttcga 840
   gagtttcata ctgtttttct gtaggccgtg tacctaaatg tacttttgct ccatcgcgat 900
   gacttagtaa agcacatcta aaacttttag cgttattacg taaaaaatct tgccagcttt 960
   ccccttctaa agggcaaaag tgagtatggt gcctatctaa catctcaatg gctaaggcgt 1020
   cgagcaaagc ccgcttattt tttacatgcc aatacaatgt aggctgctct acacctagct 1080
   tctgggcgag tttacgggtt gttaaacctt cgattccgac ctcattaagc agctctaatg 1140
   cgctgttaat cactttactt ttatctaaac gagacatact cttccttttt caatattatt 1200
   gaagcattta tcagggttat tgtctcatga gcggatacat atttgaatgt atttagaaaa 1260
   ataaacaaat aggggttccg cgcacatttc cccgaaaagt gccacctgaa attggccaga 1320
   tgattaattc ctaatttttg ttgacactct atcattgata gagttatttt accactccct 1380
   atcagtgata gagaaaagtg aaatgaatag ttcgacaaaa atctagaaat aattttgttt 1440
   aactttaaga aggagatata caaatgaaaa tcgaagaagg taaactggta atctggatta 1500
   acggcgataa aggctataac ggtctcgctg aagtcggtaa gaaattcgag aaagataccg 1560
   gaattaaagt caccgttgag catccggata aactggaaga gaaattccca caggttgcgg 1620
   caactggcga tggccctgac attatcttct gggcacacga ccgctttggt ggctacgctc 1680
   aatctggcct gttggctgaa atcaccccgg acaaagcgtt ccaggacaag ctgtatccgt 1740
   ttacctggga tgccgtacgt tacaacggca agctgattgc ttacccgatc gctgttgaag 1800
   cgttatcgct gatttataac aaagatctgc tgccgaaccc gccaaaaacc tgggaagaga 1860
   tcccggcgct ggataaagaa ctgaaagcga aaggtaagag cgcgctgatg ttcaacctgc 1920
   aagaaccgta cttcacctgg ccgctgattg ctgctgacgg gggttatgcg ttcaagtatg 1980
   aaaacggcaa gtacgacatt aaagacgtgg gcgtggataa cgctggcgcg aaagcgggtc 2040
   tgaccttcct ggttgacctg attaaaaaca aacacatgaa tgcagacacc gattactcca 2100
   tcgcagaagc tgcctttaat aaaggcgaaa cagcgatgac catcaacggc ccgtgggcat 2160
   ggtccaacat cgacaccagc aaagtgaatt atggtgtaac ggtactgccg accttcaagg 2220
   gtcaaccatc caaaccgttc gttggcgtgc tgagcgcagg tattaacgcc gccagtccga 2280
   acaaagagct ggcaaaagag ttcctcgaaa actatctgct gactgatgaa ggtctggaag 2340
   cggttaataa agacaaaccg ctgggtgccg tagcgctgaa gtcttacgag gaagagttgg 2400
   cgaaagatcc acgtattgcc gccactatgg aaaacgccca gaaaggtgaa atcatgccga 2460
   acatcccgca gatgtccgct ttctggtatg ccgtgcgtac tgcggtgatc aacgccgcca 2520
   gcggtcgtca gactgtcgat gaagccctga aagacgcgca gactatgcac ttcattgaaa 2580
   acaaaaactt cgtcatctcc attccgaccg cagacaaacg ccgtaatcac atcatccaac 2640
   agttcggcca aaagaaaatt ccgtttgaat ttttcgatgc cttcaccccg agcgaacgtc 2700
   tgaacgacca tctgcagcgc tatctgccga atgtcgcagc aaccccgcgt ctgacgatgg 2760
   gtgaaaaagg ttgcctgatg tctcacttta tgctgtggaa aaaatgtgtt gatgacggcc 2820
   tggattacat taccctgttt gaagatgaca tcctgctggg tgaaaacgcg gaacaattcc 2880
   tggccgaaga cgaatggctg aaagtgcgtt ttaatttcca ggaaatcttt gttctgcgcc 2940
   tggaaacctt cctgatgccg gtgaaaattg aaaaacagca aggcatcctg ccgtttcagc 3000
   aacgcgaaat cgatatcctg aaatcaaaac atttcggcac ggcaggttat gttatttcgc 3060
   acggtgcagc taaatacctg atcgaagtct tcgaaaaatt cagctctgaa gaagtgaaac 3120
   cgattgatga aatcatgttt aaccagctga ttgacatctc cggctatcag gtttaccaac 3180
   tgaatccggc tatttgcgtc caggaactgc aactgaacca ggaaaatagt gtgctggaat 3240
   ccggtctgca gaaagagcgt aagaaaaaca ccgttagcca taccaagaaa accctgaaat 3300
   atcgtctgac gcgcatgaaa gaaaacatcc tgcgcgcact gaataaaaag aaatgggaag 3360
   aacgccaata catcaaaggt ctgcaaggca aaaatatcat cctgtttatc taggcggccg 3420
   cgtcgacacg caaaaaggcc atccgtcagg atggccttct gcttaatttg atgcctggca 3480
   gtttatggcg ggcgtcctgc ccgccaccct ccgggccgtt gcttcgcaac gttcaaatcc 3540
   gctcccggcg gatttgtcct actcaggaga gcgttcaccg acaaacaaca gataaaacga 3600
   aaggcccagt ctttcgactg agcctttcgt tttatttgat gcctggcagt tccctactct 3660
   cgcatgggga gaccccacac taccatcatg tatgaatatc ctccttagtt cctattccga 3720
   agggtaatgg catcagggaa tggcgaacgc gctccccaca ctaccatcat gtatgaatat 3780
   cctccttagt tcctattccg aagttcctat tctctagaaa gtataggaac ttcggtggaa 3840
   cgacgcgtaa ctcacgttaa gggattttgg tcatgatcag cacgtgttga caattaatca 3900
   tcggcatagt atatcggcat agtataatac gacaaggtga ggaactaaac catggccaag 3960
   ttgaccagtg ccgttccggt gctcaccgcg cgcgacgtcg ccggagcggt cgagttctgg 4020
   accgaccggc tcgggttctc ccgggacttc gtggaggacg acttcgccgg tgtggtccgg 4080
   gacgacgtga ccctgttcat cagcgcggtc caggaccagg tggtgccgga caacaccctg 4140
   gcctgggtgt gggtgcgcgg cctggacgag ctgtacgccg agtggtcgga ggtcgtgtcc 4200
   acgaacttcc gggacgcctc cgggccggcc atgaccgaga tcggcgagca gccgtggggg 4260
   cgggagttcg ccctgcgcga cccggccggc aactgcgtgc acttcgtggc cgaggagcag 4320
   gactgagtgg cagggcgggg cgtaaggcgc gccatttaaa tgaagttcct attccgaagt 4380
   tcctattctc tagaaagtat aggaacttcg aagcagctcc agcctacaca atcgctcaag 4440
   acggaacccg cgcttggcag gaaagtaata gggatagcag ctccagccta cacaatcgct 4500
   caagacgtgt aatgctgcac aataaccctg ctgcagaggc ctgcatgcaa gcttggcgta 4560
   atcatggtca tagctgtttc ctgtgtgaaa ttgttatccg ctcacaattc cacacaacat 4620
   acgagccgga agcataaagt gtaaagcctg gggtgcctaa tgagtgagct aactcacatt 4680
   aattgcgttg cgctcactgc ccgctttcca gtcgggaaac ctgtcgtgcc agctgcatta 4740
   atgaatcggc caacgcgcgg ggagaggcgg tttgcgtatt gggcgctctt ccgcttcctc 4800
   gctcactgac tcgctgcgct cggtcgttcg gctgcggcga gcggtatcag ctcactcaaa 4860
   ggcggtaata cggttatcca cagaatcagg ggataacgca ggaaagaaca tgtgagcaaa 4920
   aggccagcaa aaggccagga accgtaaaaa ggccgcgttg ctggcgtttt tccataggct 4980
   ccgcccccct gacgagcatc acaaaaatcg acgctcaagt cagaggtggc gaaacccgac 5040
   aggactataa agataccagg cgtttccccc tggaagctcc ctcgtgcgct ctcctgttcc 5100
   gaccctgccg cttaccggat acctgtccgc ctttctccct tcgggaagcg tggcgctttc 5160
   tcatagctca cgctgtaggt atctcagttc ggtgtaggtc gttcgctcca agctgggctg 5220
   tgtgcacgaa ccccccgttc agcccgaccg ctgcgcctta tccggtaact atcgtcttga 5280
   gtccaacccg gtaagacacg acttatcgcc actggcagca gccactggta acaggattag 5340
   cagagcgagg tatgtaggcg gtgctacaga gttcttgaag tggtggccta actacggcta 5400
   cactagaaga acagtatttg gtatctgcgc tctgctgaag ccagttacct tcggaaaaag 5460
   agttggtagc tcttgatccg gcaaacaaac caccgctggt agcggtggtt tttttgtttg 5520
   caagcagcag attacgcgca gaaaaaaagg atctcaagaa gatcctttga tcttttctac 5580
   ggggtctgac gctcagtgga acgaaaactc acgttaaggg attttggtca tgagattatc 5640
   aaaaaggatc ttcacctaga tccttttaaa ttaaaaatga agttttaaat caatctaaag 5700
   tatatatgag taaacttggt ctgacagtta ccaatgctta atcagtgagg cacctatctc 5760
   agcgatctgt ctatttcgtt catccatagt tgcctgactc cccgtcgtgt agataactac 5820
   gatacgggag ggcttaccat ctggccccag tgctgcaatg ataccgcgag acccacgctc 5880
   accggctcca gatttatcag caataaacca gccagccgga agggccgagc gcagaagtgg 5940
   tcctgcaact ttatccgcct ccatccagtc tattaattgt tgccgggaag ctagagtaag 6000
   tagttcgcca gttaatagtt tgcgcaacgt tgttgccatt gctacaggca tcgtggtgtc 6060
   acgctcgtcg tttggtatgg cttcattcag ctccggttcc caacgatcaa ggcgagttac 6120
   atgatccccc atgttgtgca aaaaagcggt tagctccttc ggtcctccga tcgttgtcag 6180
   aagtaagttg gccgcagtgt tatcactcat ggttatggca gcactgcata attctcttac 6240
   tgtcatgcca tccgtaagat gcttttctgt gactggtgag tactcaacca agtcattctg 6300
   agaatagtgt atgcggcgac cgagttgctc ttgcccggcg tcaatacggg ataataccgc 6360
   gccacatagc agaactttaa aagtgctcat cattggaaaa cgttcttcgg ggcgaaaact 6420
   ctcaaggatc ttaccgctgt tgagatccag ttcgatgtaa cccactcgtg cacccaactg 6480
   atcttcagca tcttttactt tcaccagcgt ttctgggtga gcaaaaacag gaaggcaaaa 6540
   tgccgcaaaa aagggaataa gggcgacacg gaaatgttga atactcatac tcttcctttt 6600
   tcaatattat tgaagcattt atcagggtta ttgtctcatg agcggataca tatttgaatg 6660
   tatttagaaa aataaacaaa taggggttcc gcgcacattt ccccgaaaag tgccacctga 6720
   cgtctaagaa accattatta tcatgacatt aacctataaa aataggcgta tcacgaggcc 6780
   ctttcgtc 6788
<210> 15
   <211> 35
   <212> DNA
   <213> Artificial Sequence
<220>
   <223> Primer gal fwd
<400> 15
   ttactcagca ataaactgat attccgtcag gctgg 35
<210> 16
   <211> 86
   <212> DNA
   <213> Artificial Sequence
<220>
   <223> Primer gal rev
<400> 16
   ttgataatct cgcgctcttc agcagtcaga ctttccatat agagcgtaat ttccgttaac 60
   gtcggtagtg ctgaccttgc cggagg 86
<210> 17
   <211> 58
   <212> DNA
   <213> Artificial Sequence
<220>
   <223> Primer ME for
<400> 17
   ctgtctctta tacacatctc ctgaaattgg ccagatgatt aattcctaat ttttgttg 58
<210> 18
   <211> 50
   <212> DNA
   <213> Artificial Sequence
<220>
   <223> Primer ME rev
<400> 18
   ctgtctctta tacacatctc agcattacac gtcttgagcg attgtgtagg 50
<210> 19
   <211> 30
   <212> DNA
   <213> Artificial Sequence
<220>
   <223> Primer galE_NdeI-for
<400> 19
   gatcacatat gagagttctg gttaccggtg 30
<210> 20
   <211> 40
   <212> DNA
   <213> Artificial Sequence
<220>
   <223> Primer galE_XhoI-rev
<400> 20
   gatcactcga gtcattaatc gggatatccc tgtggatggc 40
<210> 21
   <211> 35
   <212> DNA
   <213> Artificial Sequence
<220>
   <223> Primer gal fwd
<400> 21
   ttactcagca ataaactgat attccgtcag gctgg 35
<210> 22
   <211> 83
   <212> DNA
   <213> Artificial Sequence
<220>
   <223> Primer gal rev
<400> 22
   ttgtaatctc gcgctcttca catcagactt tccatataga gcgtaatttc cgttaacgtc 60
   ggtagtgctg accttgccgg agg 83
<210> 23
   <211> 54
   <212> DNA
   <213> Artificial Sequence
<220>
   <223> Primer ME for
<400> 23
   ctgtctctta tcacatctcc tgaaatggcc agatgtaatt cctaattttt gttg 54
<210> 24
   <211> 44
   <212> DNA
   <213> Artificial Sequence
<220>
   <223> Primer ME rev
<400> 24
   ctgtctctta tcacatctca cattacatct gagcgattgt tagg 44
<210> 25
   <211> 30
   <212> DNA
   <213> Artificial Sequence
<220>
   <223> Primer galE_NdeI-for
<400> 25
   gatcacatat gagagttctg gttaccggtg 30
<210> 26
   <211> 40
   <212> DNA
   <213> Artificial Sequence
<220>
   <223> Primer galE_XhoI-rev
<400> 26
   gatcactcga gtcattaatc gggatatccc tgtggatggc 40
<210> 27
   <211> 60
   <212> DNA
   <213> Artificial Sequence
<220>
   <223> Primer lexl pINT-f
<400> 27
   gtcgatgaag ccctgaaaga cgcgcagact atgcacttca ttgaaaacaa aaacttcgtc 60
<210> 28
   <211> 60
   <212> DNA
   <213> Artificial Sequence
<220>
   <223> Primer lexl pINT-r
<400> 28
   gatggccttt ttgcgtgtcg acgcggccgc ctagataaac aggatgatat ttttgccttg 60
<210> 29
   <211> 60
   <212> DNA
   <213> Artificial Sequence
<220>
   <223> Primer pINT lexl-f
<400> 29
   caaggcaaaa atatcatcct gtttatctag gcggccgcgt cgacacgcaa aaaggccatc 60
<210> 30
   <211> 60
   <212> DNA
   <213> Artificial Sequence
<220>
   <223> Primer pINT lexl-r
<400> 30
   gacgaagttt ttgttttcaa tgaagtgcat agtctgcgcg tctttcaggg cttcatcgac 60
<210> 31
   <211> 60
   <212> DNA
   <213> Artificial Sequence
<220>
   <223> Primer lgtB pINT-f
<400> 31
   gtcgatgaag ccctgaaaga cgcgcagact atggcccaaa accacgttat cagcttagct 60
<210> 32
   <211> 60
   <212> DNA
   <213> Artificial Sequence
<220>
   <223> Primer lgtB pINT-r
<400> 32
   gatggccttt ttgcgtgtcg acgcggccgc ttattggaaa ggcacaatga actgttcgcg 60
<210> 33
   <211> 60
   <212> DNA
   <213> Artificial Sequence
<220>
   <223> Primer pINT lgtB-f
<400> 33
   cgcgaacagt tcattgtgcc tttccaataa gcggccgcgt cgacacgcaa aaaggccatc 60
<210> 34
   <211> 60
   <212> DNA
   <213> Artificial Sequence
<220>
   <223> Primer pINT lgtB-r
<400> 34
   agctaagctg ataacgtggt tttgggccat agtctgcgcg tctttcaggg cttcatcgac 60

## Description

The present invention relates to a method for producing oligosaccharides comprising a terminal galactose-(1->4)-glucose disaccharide.

Human milk represents a complex mixture of carbohydrates, fats, proteins, vitamins, minerals and trace elements. The by far most predominant fraction is represented by carbohydrates, which can be further divided into lactose and more complex oligosaccharides (Human milk oligosaccharides, HMO). Whereas lactose is used as an energy source, the complex oligosaccharides are not metabolized by the infant. The fraction of complex oligosaccharides accounts for up to 1/10 of the total carbohydrate fraction and consists of probably more than 150 different oligosaccharides. The occurrence and concentration of these complex oligosaccharides are specific to humans and thus cannot be found in large quantities in the milk of other mammals, like for example domesticated dairy animals.

The most prominent oligosaccharides are 2'-fucosyllactose and 3'-fucosyllactose which together can contribute up to 1/3 of the total HMO fraction. Further prominent HMOs present in human milk are lacto-*N*-tetraose, lacto-*N*-neotetraose and the lacto-*N*-fucopentaoses I. Besides these neutral oligosaccharides, also acidic HMOs can be found in human milk, like for e.g. 3'-sialyllactose, 6'-sialyllactose and 3-fucosyl-3'-sialyllactose, disialyl-lacto-*N*-tetraose etc. These structures are closely related to epitopes of epithelial cell surface glycoconjugates, the Lewis histoblood group antigens such as Lewis x (LeX), and the structural homology of HMO to epithelial epitopes accounts for protective properties against bacterial pathogens.

The existence of these complex oligosaccharides in human milk is known already for a long time and the physiological functions of these oligosaccharides were subject to medicinal research for many decades. For some of the more abundant human milk oligosaccharides, specific functions have already been identified.

Besides the mentioned local effects in the intestinal tract, HMOs have also been shown to elicit systemic effects in infants by entering the systemic circulation. Also, the impact of HMOs on protein-carbohydrate interactions, e.g., selectin-leukocyte binding, can modulate immune responses and reduce inflammatory responses.

Due to the well-studied beneficial properties of prebiotic oligosaccharides, in particular of HMOs, connected with their limited availability, an effective commercial, i.e. large scale production is highly desirable.

Due to the limited supply and difficulties of obtaining pure fractions of individual human milk oligosaccharides, chemical routes to some of these complex molecules were developed. However, production of human milk oligosaccharides by chemical synthesis, enzymatic synthesis or fermentation proved to be challenging. At least large-scale quantities as well as qualities sufficient for food applications cannot be provided until today through chemical synthesis. In this regard, particularly chemical synthetic routes to human milk oligosaccharides involve several noxious chemicals, which impose the risk to contaminate the final product.

Due to the challenges involved in the chemical synthesis of human milk oligosaccharides, several enzymatic methods and fermentative approaches were developed. Today, for several HMOs such as 2'-fucosyllactose, 3-fucosyllactose, lacto-*N-*tetraose, lacto-*N*-*neo*tetraose, 3'-sialyllactose and 6'-sialyllactose fermentative approaches have been developed, using mainly genetically engineered bacterial strains such as recombinant *Escherichia coli.*

Today, biocatalytic as well as fermentative approaches to HMOs were all based exclusively on lactose as a starting substrate, to which additional monosaccharides are added, as disclosed in, e.g., US 7,521,212 B1 or Albermann et al., (2001) Carbohydr. Res. 334(2) p 97-103. The addition of monosaccharides to lactose can be either catalyzed by glycosyltransferases or glycosidases using suitable activated monosaccharide substrates. In addition, additional monosaccharides can be added to lactose by transglucosidase reactions.

In particular the fermentative synthesis of HMOs proved particular efficient, because the required and difficult to synthesized nucleotide activated monosaccharides are provided by the metabolism of the used microbe. However, the use of whole cells for the synthesis of HMOs also bears - in comparison to the biocatalytic approach - several major disadvantages, which are related to transport processes across the cell membrane, metabolic side reactions and the fact that the synthesized oligosaccharides have to be purified from a complex mixture containing, *inter alia,* various polyols (e.g. carbohydrates), nucleic acids, polypeptides, inorganic material etc.

US 2009/0082307 A1 discloses the production of oligopolysaccharides by a microbiological process, wherein an exogenous precursor, such as lactose, is added to the medium a host cell is cultivated in, and wherein the precursor is internalized by said cell.

A technical problem related to the use of lactose in fermentative processes is presented by the rearrangement of lactose (beta-D-galactopyranosyl-(1->4)-D-glucose) into lactulose (beta-D-galactopyranosyl-(1->4)-D-fructofuranose) by thermal treatment of lactose. This rearrangement can occur extensively by autoclaving lactose, leading to the rearrangement of several percent of the lactose present in a fermentation medium or lactose fermentation feed to lactulose. However, lactulose represents a non-digestible sugar for humans and is widely used in the treatment of chronic constipation.

Also, the conversion of lactose to lactulose not only leads to the generation of unwanted side products, but furthermore it also generates a potential substrate for glycosylation and the generation of more complex oligosaccharides (e.g. 2'-fucosyl-lactulose). Thus, the generation of lactulose from lactose is leading to the contamination of the desired product with closely related oligosaccharides, which are difficult or even impossible to separate away from the desired product.

Above all, the addition of lactose in particular leads to a well-documented effect known as lactose induced cell killing. This effect is most likely caused by the excessive uptake of lactose and the associated collapse of the proton gradient across the bacterial membrane. In particular the overexpression of the lactose permease gene (e.g. *lacY* of *E. coli*) in combination with the exposure of the recombinant cell can cause considerable growth delay of the recombinant strain and the increased synthesis of cellular polysaccharides (Grube et al., "Hydrogen-producing Escherichia coli strains overexpressing lactose permease: FT-IR analysis of the lactose-induced stress" (2013) Biotechnol. Appl. Biochem. 5, 31.).

Furthermore, today, lactose on commercial scale is entirely derived from whey, a waste product of the dairy industry. Whey is produced in enormous quantities in cheese and casein manufacturing. Thus being derived from the dairy industry there is still concerns related to possible contamination of lactose with prion proteins the causative agent of bovine spongiform encephalopathy (BSE), also widely known as mad cow disease. BSE a fatal neurodegenerative disease in cattle, causing spongy degeneration of the brain and spinal cord, can be transmitted to humans and is there known as variant of Creutzfeldt-Jakob disease. Furthermore lactose, if supplied to a beta-galactosidase positive *E*. *coli* strain, can be converted to allolactose (beta-D-galactopyranosyl-(1->6)-D-glucopyranose), another unwanted contaminant (Huber et al., "Efflux of beta-galactosidase products from Escherichia coli" (1980) J. Bacteriol. 141, 528-533).

In addition lactose is still one of the most expensive components of the fermentation medium and its substitution by glucose, glycerol, sucrose etc. would lead to a more cost effective production of HMOs.

In view of the above, it would be desirable to develop improved means and methods for the production of HMO, which allow overcoming the above mentioned drawbacks of the state of the art.

According to the invention, this and other objects are solved by a method for producing at least one oligosaccharide of interest, the oligosaccharide of interest comprising a terminal galactose-(1->4)-glucose disaccharide, as claimed in the attached claims, wherein the method, in an alternative, comprises the step of
a) obtaining a bacterial host cell, cell being capable to produce oligosaccharides comprising a terminal galactose-(1->4)-glucose disaccharide, and comprising
   (i) at least one recombinant nucleic acid sequence encoding for a protein having a beta-1,4-galactosyltransferase activity and being able to galactosylate a free glucose monosaccharide to intracellularly generate lactose,
   (ii) at least one recombinant nucleic acid sequence encoding a glycosyltransferase that is selected from at least one of the following: a fucosyltransferase, a sialyltransferase, a glucosamyltransferase, a galactosyltransferase,
   and which bacterial host cell is capable of generating the oligosaccharide without the exogenous addition of lactose, and
b) culturing said bacterial host cell, wherein said culturing is performed (i) from a carbon source that is selected from at least one of the following: glucose, sucrose, glycerol, molasses, corn syrup, galactose, syngas, carbon monoxide, methanol, succinate, pyruvate, raffinose, wherein the host cell is capable of generating free glucose within the cell from said carbon source, and (ii) without externally supplying lactose during execution of the method, thus producing said oligosaccharide of interest.

The object underlying the invention is completely solved in this way.

With the method according to the invention and the bacterial cell used in the method, it is possible to totally ferment a desired oligosaccharide comprising a terminal galactose-(1->4)-glucose disaccharide, in particular one that carries a galactose-(1->4)-glucose disaccharide at its reducing end, such as e.g. 2'-fucosyllactose and other HMOs from a simple carbon source such as glucose, sucrose, glycerol, and other c-sources as claimed in the attached claims, without the need to externally supply lactose as substrate to the fermentation method of such oligosaccharides.

The simple carbon sources can be used to attain a pool of free glucose within the producing cell, such, that the cell can synthesize lactose. Then, the obtained lactose can either be exported from the synthesizing cell and used by other cells in the fermentation for the synthesis of more complex oligosaccharides, or more advantageously by the synthesizing cell itself for the synthesis of more complex oligosaccharides. The synthesized oligosaccharide products are then exported from the producing cell into the fermentation medium by exporters or permeases.

Presently, the term "nucleic acid" refers to a deoxyribonucleotide or ribonucleotide polymer in either single- or double-stranded form, and unless otherwise limited, encompasses known analogues of natural nucleotides that hybridize to nucleic acids in a manner similar to naturally occurring nucleotides. Unless otherwise indicated, a particular nucleic acid sequence includes the complementary sequence thereof.

Presently, the term "operably linked" as used herein, shall mean a functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence. Accordingly, the term "Promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

The term "recombinant", as used herein with reference to a bacterial host cell indicates that the bacterial cell replicates a heterologous nucleic acid, or expresses a peptide or protein encoded by a heterologous nucleic acid (i.e., a sequence "foreign to said cell"). Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques. Accordingly, a "recombinant polypeptide" is one which has been produced by a recombinant cell. A "heterologous sequence" or a "heterologous nucleic acid", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form. The heterologous sequence may be stably introduced, e.g. by transfection, transformation, conjugation or transduction, into the genome of the host microorganism cell, wherein techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989).

Accordingly, a "bacterial host cell" is presently understood as a bacterial cell which has been transformed or transfected, or is capable of transformation or transfection by an exogenous polynucleotide sequence.

Thus, the nucleic acid sequences as used in the present invention, may, e.g., be comprised in a vector which is to be stably transformed/transfected or otherwise introduced into host microorganism cells.

A great variety of expression systems can be used to produce the polypeptides of the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and to synthesize a polypeptide in a host may be used for expression in this regard. The appropriate DNA sequence may be inserted into the expression system by any of a variety of well-known and routine techniques, such as, for example, those set forth in Sambrook *et al., supra.*

The art is rich in patent and literature publications relating to "recombinant DNA" methodologies for the isolation, synthesis, purification and amplification of genetic materials for use in the transformation of selected host organisms. Thus, it is common knowledge to transform host organisms with "hybrid" viral or circular plasmid DNA which includes selected exogenous (i.e. foreign or "heterologous") DNA sequences. The procedures known in the art first involve generation of a transformation vector by enzymatically cleaving circular viral or plasmid DNA to form linear DNA strands. Selected foreign DNA strands usually including sequences coding for desired protein product are prepared in linear form through use of the same/similar enzymes. The linear viral or plasmid DNA is incubated with the foreign DNA in the presence of ligating enzymes capable of effecting a restoration process and "hybrid" vectors are formed which include the selected exogenous DNA segment "spliced" into the viral or circular DNA plasmid.

The term "nucleic acid sequence encoding..." generally refers to any polyribonucleotide or polydeoxyribonucleotide, which may be unmodified RNA or DNA or modified RNA or DNA, and generally represents a gene which encodes a certain polypeptide or protein. The term includes, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. The term also encompasses polynucleotides that include a single continuous region or discontinuous regions encoding the polypeptide (for example, interrupted by integrated phage or an insertion sequence or editing) together with additional regions that also may contain coding and/or non-coding sequences.

As used herein, the term "cultivating" means growing a bacterial cell in a medium and under conditions permissive and suitable for the production of the desired oligosaccharide(s). A couple of suitable bacterial host cells as well as mediums and conditions for their cultivation will be readily available for one skilled in the art upon reading the disclosure of this invention in connection with the skilled person's technical and expert background.

It is to be understood that with the invention as disclosed therein, the production of one or more, i.e. two, three, four, etc., oligosaccharides as defined herein is possible, as long as the respective nucleic acids encoding the relevant proteins/enzymes as disclosed herein are comprised in the cell(s).

According to a preferred embodiment, the method according to the invention comprises the step c) recovering the oligosaccharide from the medium and/or the bacterial cells. It is to be understood that step c) follows step b), i.e. the cultivating step, and recovering may be accomplished whenever the desired oligosaccharide as defined herein, or a desired amount thereof, has been reached by the cultivating step.

As used herein, the term "recovering" means isolating, harvesting, purifying, collecting or otherwise separating from the host microorganism culture the oligosaccharide produced by the host microorganism according to the invention.

Further, as used herein term "enzymatic activities" is meant to comprise any molecule displaying enzymatic activity, in particular a protein, and acting as a catalyst to bring about a specific biochemical reaction while remaining unchanged by the reaction. In particular, proteins with enzymatic activities are meant to be comprised by this term, which are able to convert a substrate into a product.

In enzymatic reactions, the molecules at the beginning of the process, called substrates, are converted into different molecules, called products. Almost all chemical reactions in a biological cell need enzymes in order to occur at rates sufficient for life. Since enzymes are selective for their substrates and speed up only a few reactions from among many possibilities, the set of enzymes made in a cell determines which metabolic pathways occur in that cell.

With the bacterial cell, its use and the methods as described herein, it was surprisingly found that total fermentation of a desired oligosaccharide as defined herein can be achieved. The present invention utilizes a bacterial microorganism basically having two specific enzymatic activities, i.e. a beta-1,4-galactosyltransferase activity and at least one glycosyltransferase activity, in a method, where the bacterial host microorganism - growing on a simple carbon source - effectively produces the desired oligosaccharide without adding lactose.

Within the context of the present invention, the expression "without the exogenous addition of lactose" is meant to designate that lactose is not added to the cultivation medium the bacterial host cell according to the invention is cultivated in. "Exogenous" shall be contrary to "endogenously produced" by said cell, and shall mean that the bacterial host cell is not fed with lactose during any time of the method.

Although unmodified microorganisms having the above described enzymatically features can be employed with the present method, according to one aspect of the invention, the bacterial microorganism is a recombinant bacterial microorganism or cell, wherein the recombinant bacterial cell has been transformed to comprise at least the above mentioned enzymatic activities.

The bacterial host cell used in the method according to the invention uses the carbon source, e.g. glucose, as follows: firstly, the carbon source, i.e. glucose, is internalized by the bacterial cell, i.e. transported over the cytoplasmic membrane into the cytosol. In the cytosol, glucose is galactosylated - i.e. a galactose-residue is transferred from an nucleotide activated galactose present (or generated in the cell) to the glucose - by the beta-1,4-galactosyltransferase activity displaying enzyme to produce lactose. Subsequently, the lactose is used as acceptor for the glycosyltransferase activity transferring, e.g., a fucosyl-residue from a nucleotide-activated fucose to the lactose, thus generating fucosyllactose. This process can either be accomplished in the same bacterial cell comprising the beta-1,4-galactosyltransferase activity and at least one glycosyltransferase activity, or the lactose is exported from the cell carrying a beta-1,4-galactosyltransferase activity and used by another (bacterial) cell, which cell is present or added to the medium, in which case the lactose is taken up/internalized by said other cell, which comprises the glycosyltransferase activity using the lactose as acceptor.

The fucosyllactose - or any other produced oligosaccharide as defined herein - is then transported from the cell into the medium the bacterial cell is cultivated in and can be recovered, e.g. isolated, therefrom, and, thus, provided for further use.

Generally, and throughout the present invention, the term "glycosyltransferase activity" or "glycosyltransferase" designates and encompasses enzymes that are responsible for the biosynthesis of disaccharides, oligosaccharides and polysaccharides, and they catalyse the transfer of monosaccharide moieties from an activated nucleotide monosaccharide/sugar (the "glycosyl donor") to a glycosyl acceptor molecule.

According to the invention, the glycosyltransferase is selected from a galactosyltransferase, a sialyltransferase, a *N*-acetylglucosaminyltransferase and a fucosyltransferase.

According to a preferred embodiment, in the method of the invention and in the bacterial host cell used therein, the glycosyltransferase is selected from at least one of the following: alpha-1,2-fucosyltransferase, alpha-1,3-fucosyltransferase, beta-1,3-*N-*acetylglucosamyltransferase, beta-1,3-galactosyltransferase, alpha-2,3-sialyltransferase, alpha-2,6-sialyltansferase, beta-1,4-galactosyltransferase or beta-1,6-galactosyltransferase.

According to preferred embodiments, for the synthesis of 2'-fucosyllactose a suitable 2-fucosyltransferase is expressed in addition to the beta-1,4-galactosyltransferase activity. For the synthesis of 3-fucosyllactose a suitable 3-fucosyltransferase is expressed in addition to the beta-1,4-galactosyltransferase. For the synthesis of 2',3-difucosyllactose, both, a suitable alpha-1,2-fucosyltransferase and a alpha-1,3-fucosyltransferase is expressed in addition to the beta-1,4-galactosyltransferase. For the synthesis of lacto-*N*-tetraose a suitable beta-1,3-*N-*acetylglucosamyltransferase and a beta-1,3-galactosyltransferase is expressed in the producing bacterial host cell in addition to the beta-1,4-galactosyltransferase. For the synthesis of even more complex pentasaccharides such as lacto-*N*-fucopenatose I, the recombinant cell is prepared by introducing nucleic acids to be capable of functional expression of a suitable beta-1,3- -*N*-acetyl-glucosamyltransferase, beta-1,3-galactosyltransferase and alpha-1,2-fucosyltransferase in addition to the beta-1,4-galactosyltransferase.

Non-limiting examples for glycosyltransferases which can be used according to the invention are, e.g., bacterial fucosyl-transferases, and preferably an alpha-1,2-fucosyltransferase, and more preferably the alpha-1,2-fucosyltransferase encoded by the *wbgL* gene of *E.* co*li*:O126 (genebank acc. No. ADN43847), or an alpha-1,3-fucosyltransferase, and more preferably an alpha-1,3-fucosyltransferase from *Akkermansia muciniphila*, *Bacteroides fragilis, Helicobacter pylori, Escherichia coli, Salmonella enterica, mammals, Caenorhabditis elegans and Schistosoma mansoni.* Preferably, a glycosyltransferase is used or variants thereof which is disclosed in EP 2 479 263 A1or from EP 2 439 264 or in WO 2010/142305.

"Variant(s)" as the term is used herein, is a polynucleotide or polypeptide that differs from a reference polynucleotide or polypeptide respectively, but retains the essential (enzymatic) properties of the reference polynucleotide or polypepeitde. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques, by direct synthesis, and by other recombinant methods known to the persons skilled in the art.

Also nucleic acid/polynucleotide and polypeptide polymorphic variants, alleles, mutants, and interspecies homologs are comprised by those terms, that have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200,500, 1000, or more amino acids, to a polypeptide encoded by a wildtype glycosyltransferase/beta-1,4-galactosyltransferase activity displaying protein.

Accordingly, a "functional fragment" of any of the genes/proteins disclosed therein, is meant to designate sequence variants of the genes/proteins still retaining the same or somewhat lesser activity of the gene or protein the respective fragment is derived from.

Accordingly, the terms "alpha-1,2-fucosyltransferase" or "fucosyltransferase" or a nucleic acid/polynucleotide encoding an "alpha-1,2-fucosyltranferase" or "fucosyltransferase" refer to a glycosyltransferase that catalyzes the transfer of fucose from a donor substrate, for example, GDP-fucose, to an acceptor molecule in an alpha-1,2-linkage. The terms "alpha-1,3-fucosyltranferase or fucosyltransferase" or a nucleic acid/polynucleotide encoding an "alpha-1,3-fucosyltranferase or fucosyltransferase" refer to a glycosyltransferase that catalyzes the transfer of fucose from a donor substrate, for example, GDP-fucose, to an acceptor molecule in an alpha-1,3-linkage. The acceptor molecule can be, e.g., lactose, lacto-*N*-triose II, lacto-*N*-tetraose, lacto-*N*-*neo*tetraose or any derivative thereof.

According to a preferred embodiment, in the method according to the invention and in the bacterial host cell used therein, it is further comprised a nucleic acid sequence encoding at least one of the following additional proteins: a oligosaccharide exporter protein or a permease exporting the synthesized oligosaccharide from the host cell, an invertase, a fructokinase, proteins involved in the biosynthesis of GDP-fucose, GDP-glucose, GDP-mannose, UDP-*N*-acetylglucosamine, UDP-galactose, UDP-*N-*acetylgalactosamine, acid, and/or CMP-sialic acid.

Preferably, the oligosaccharide exporter protein or the permease is a protein capable of exporting a soluble oligosaccharide product out of the producing cell. An exemplary embodiment, which, according to one aspect of the invention, can be used as nucleic acid expressing an exporter protein, is the *yberc0001_9420* gene of *Yersinia bercovieri* ATCC 43970 or variants or homologs or functional fragments thereof. Accordingly, an "exporter protein" or "oligosaccharide exporter" protein" is any protein being able to export an oligosaccharide from a bacterial cell. In this regard it is also referred to the disclosure and particularly to the examples of WO 2010/142305, which describes and discloses oligosaccharide exporters and their use.

Exemplary embodiments for the above mentioned exporter or permease proteins are, e.g., the proteins encoded by the csc-gene cluster, which comprises four genes for sucrose permease, fructokinase, sucrose hydrolase and a transcriptional repressor (*cscB, cscK, cscA,* and *cscR,* respectively), the glucose permease *of Bacillus subtiilis,* glucose permease of *Streptomyces coelicolor or Streptomyces clavuligerus.* Accordingly, in a preferred embodiment the bacterial host cell further comprises the csc-gene cluster.

Exemplary embodiments for proteins involved in the biosynthesis of nucleoside diphosphate sugars are, e.g., a nucleoside diphosphate sugar pyrophosphorylase, a sugar kinase, and preferably, fucosekinase, GDP-fucose-pyrophosphorylase, GDP-mannose 4,6-dehydratase (GMD), GDP-keto-6-deoxymannose 3,5-epimerase, 4-reductase, glucosekinase, glucose-6-phosphate 1-epimerase, phosphoglucomutase, glucose-1-phosphate guanylyltransferase, CMP-sialate synthase.

The nucleic acid may be an endogenous or a recombinant nucleic acid.

As defined above, the term "endogenous" herein and generally within the field means that the nucleic acid encoding for an enzyme of interest is originating from the bacterial host cell and has not been introduced into said host cell, whereas a "recombinant" nucleic acid has been introduced into said host cell and does not originates from said host cell.

The nucleic acid may be homologous or heterologous. Presently, and as generally understood in the relevant field, the expression "homologous" refers to a nucleic acid sequence that encodes for a specific product or products and is derived from the same species, in which said nucleic acid sequence is inserted. Accordingly, the term "heterologous" refers to a nucleic acid sequence encoding for a specific product or products and being derived from a species other than those in which said nucleic acid sequence is inserted.

According to another embodiment, the bacterial host cell used in the method of the invention further comprises control sequences enabling the controlled overexpression of endogenous or recombinant nucleic acid sequences. As defined above, the term "control sequence" which herein is synonymously used with the expression "nucleic acid expression control sequence", comprises promoter sequences, signal sequence, or array of transcription factor binding sites, which sequences affect transcription and/or translation of a nucleic acid sequence operably linked to the control sequences.

As outlined already further above, the nucleic acid sequence which is used according to the invention, may, e.g., be comprised in a vector which is to be stably transformed/transfected into bacterial host cells. The definitions and detailed description for recombinant production as outlined above shall apply for this paragraph.

In some embodiments, the nucleic acid sequence is placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the proteins used in the present invention. For *E*. *coli* -and other bacterial host cells, inducible promoters are known to those of skill in the art.

According to an aspect of the invention, the beta- 1,4-galactosyltransferase is a beta-1,4-galactosyltransferase derived from *Neisseria meningitidis,* and is preferably encoded by a gene selected from the group consisting of LgtB from *Neisseria meningitidis,* or *lex-1* from *Aggregatibacter aphrophilus,* or a functional fragments thereof. It is to be understood, that a person skilled in the art will, upon reading this invention, be able to identify any other enzyme having beta- 1,4-galactosyltransferase and accepting the monosaccharide glucose as substrate and being eventually derived from any other organism, which beta-1,4-galactosyltransferase will operate in the presently described and claimed way.

With the beta-1,4-galactosyltransferase that has been introduced in the bacterial host cell according to the invention, intracellular free glucose, which serves as an acceptor substrate for the β-1,4-galactosyltransferase reaction, is converted to a lactose disaccharide structure for HMO synthesis.

The pool of free glucose for lactose synthesis can be provided by supplying glucose via the cultivation medium.

Alternatively, free glucose can be provided intracellular by using a glucose containing disaccharide or oligosaccharide as carbon source for the recombinant bacterial host cell. The so supplied disaccharide or oligosaccharide is cleaved into monosaccharides thus providing free glucose and other monosaccharides for intracellular HMO synthesis. Besides providing free glucose or glucose containing saccharides, glucose for the oligosaccharide synthesis can be also provided via the metabolism (gluconeogenesis) of the recombinant cell.

Besides a pool of free glucose acting as an acceptor substrate within the bacterial host cell, also an effective supply of, e.g., UDP-galactose is needed. This supply for UDP-galactose can, e.g., be obtained from the cells own metabolism, and the metabolism can be improved by further genetic modification, such as the overexpression of the UDP-galactose-4'-epimerase, or the UDP-galactose-4'-epimerase in combination with the glucose-1-phosphate-1-uridinyltransferase.

Alternatively, UDP-galactose can be obtained by feeding galactose to the HMO producing bacterial host cell via the fermentation medium. The galactose is then taken up by the cell, phosphorylated to galactose-1-phosphate and then converted to UDP-galactose. Genes encoding these enzymatic activities are well known in the literature (Grossiord et al., "Characterization, Expression, and Mutation of the Lactococcus lactis galPMKTE Genes, Involved in Galactose Utilization via the Leloir Pathway" (2003) J. Bacteriol 185(3) 870-878.

For the synthesis of, e.g. lacto-*N*-tetraose, in addition to UDP-galactose also UDP-*N*-acetylglucosamine is required. UDP-*N*-acetlyglucosamine can be also obtained from the bacterial host cell's own UDP-*N*-acetylglucosamine metabolism. The provision of UDP-*N*-acetylglucosamine for the synthesis of *N*-aectylglucosamine containing oligosaccharides can be improved by the inactivation of the *N*-acetylglucosamine catabolism within the producing cell.

For the synthesis of fucose containing HMO, such as 2'-fucosyllactose, 3-fucosyllactose and 2',3-fucosyllactose but not limited, GDP-L-fucose is needed. GDP-fucose for the synthesis of 2'-fucosyllactose can be provided, e.g., by the action of a bifunctional L-fucokinase/GDP-pyrophosphorylase enzyme, encoded by the *Bacteroidis fragilis fkp* gene - and by supplying L-fucose to the HMO synthesizing cell. Similarly sialic acid can be supplied to the cell that is then taken up by the cell and activated by a CMP-sialate synthase to CMP-sialic acid. CMP-sialic acid (also known as CMP-neuraminic acid) can then be used for the synthesis of sialic acid containing oligosaccharides (such as 3'-sialyllactose, 6'-sialyllactose, 3-fucosyl-3'-sialyllactose).

Alternatively for the provision of GDP-L-fucose genes of the GDP-L-fucose pathway can be overexpressed in order to increase the supply of GDP-L-fucose for the synthesis of fucosylated HMOs such as 2'-fucosyllactose, 3-fucosyllactose, 2',3-difucosyllactos, lacto-*N*-fucopentaose I and other fucosylated HMOs.

According to a preferred embodiment of the method according to the invention and the bacterial host cell used therein, the bacterial host cell is a *Escherichia coli* strain, a *Lactobacillus* species, a *Corynebacterium* strain, a *Bacillus* strain, a *Lactobacillus* strain, a *Streptococcus* strain, an *Enterococcus* strain, a *Lactococcus* strain, or a *Clostridium* strain. Preferably, the host cell is a bacterial host cell selected from a *Corynebacterium glutamicum, Clotridium cellulolyticum, Clostridium Ijungdahlii, Clostridium autoethanogenum, Clostridium acetobutylicum, Bacillus subtilis, Bacillus megaterium, Lactobacillus casei, Lactobacillus acidophilus, Lactobacillus helveticus, Lactobacillus delbrueckii, Lactococcus lactis* cell. A person skilled in the art will be aware of further bacterial strains when reading the present disclosure.

According to a preferred embodiment, the bacterial host used in the method according to the invention is further modified (i) not to express proteins intracellularly degrading precursors of said oligosaccharide, or (ii) to have blocked the expression of proteins intracellulary degrading precursors of said oligosaccharide (iii) or regulated in such a way that they do not interfere with the synthase of the desired oligosaccharides. In a preferred embodiment, said protein is a beta-galactosidase, and preferably lacZ.

Further, with the expression "precursor" compounds are encompassed which are involved in the biosynthetic pathway of the oligosaccharide according to the invention, in particular endogenous precursors, i.e. precursors which are produced and naturally present in the host cell.

The invention, in an alternative, also relates to a method for producing at least one oligosaccharide of interest by total fermentation through genetically modified host cells, the oligosaccharide of interest comprising a terminal galactose-(1->4)-glucose disaccharide, and the method comprising the steps of:
a) obtaining a first bacterial host cell comprising a recombinant nucleic acid sequence encoding for a protein having a beta 1,4-galactosyltransferase activity and being able to galactosylate a free glucose monosaccharide to intracellularly generate lactose, and obtaining a second bacterial host cell comprising at least one recombinant nucleic acid sequence encoding a glycosyltransferase that is selected from at least one of the following: a fucosyltransferase, a sialyltransferase, a glucosamyltransferase, a galactosyltransferase;
b) culturing said bacterial host cells from step a), wherein said culturing is performed (I) from a carbon source that is selected from at least one of the following: glucose, sucrose , glycerol, molasses, corn syrup, galactose, syngas, carbon monoxide, methanol, succinate, pyruvate, raffinose, wherein the host cell is capable of generating free glucose within the cell from said carbon source, and (II) without externally supplying lactose during execution of the method, thus producing said oligosaccharide of interest.

As already for the bacterial host cell explained above, with the method according to the invention, it is possibly to fully or totally ferment an oligosaccharide of interest, without the need of adding precursors of said oligosaccharide, since with the method presented herein, the oligosaccharide can be microbially produced by host cells that grow on simple carbon sources. According to the method of the invention, a bacterial host cell is provided as outlined above, and is cultured on a simple carbon source, e.g. on glucose, which is internalized by the bacterial cell, i.e. transported over the cytoplasmic membrane into the cytosol. In the cytosol, the carbon source, e.g. the glucose, is galactosylated - i.e. a galactose-residue is transferred from an nucleotide activated galactose present (or generated in the cell) to the glucose - which step is catalyzed by the beta-1,4-galactosyltransferase activity to produce lactose. Subsequently, the lactose is used as acceptor for a glycosyltransferase activity transferring, e.g., a fucosyl-residue from a nucleotide-activated fucose to the lactose, thus generating, e.g., fucosyllactose.

According to the method of the invention, this process can either be accomplished in the same bacterial host cell comprising the beta-1,4-galactosyltransferase activity and at least one glycosyltransferase activity, or only the first step, i.e. step (i), the galactosylation is performed in a first bacterial host cell, the thus generated lactose is exported from the first bacterial cell carrying a beta-1,4-galactosyltransferase activity into the medium the first cell is cultured in, and subsequently used by another, a second bacterial host cell, which second bacterial host cell is present or added to the medium, and the lactose is taken up/internalized by said second bacterial host cell, which comprises the glycosyltransferase activity using the lactose as acceptor.

According to a preferred embodiment of the method according to the invention, the oligosaccharide of interest is a human milk oligosaccharide, preferably a human milk oligosaccharide selected from at least one of the one listed in the attached table 1, preferably selected from 2'-fucosyllactose, 3-fucosyllactose, 2'3-difucosyllactose, 3'-sialyllactose, 6'-sialyllactose, 3-fucosyl-3'-sialyllactose, lacto-*N*-tetraose, lacto-*N-*neotetraose, lacto-*N*-fucopentaose I, lacto-*N*-fucopentaose II, lacto-*N*-fucopentaose III, lacto-*N*-fucopentaose V, lacto-*N*-difucosylhexose I, lacto-*N*-difucosylhexaose II, lacto-N-sialylpentaose LSTa, LSTb, LSTc.

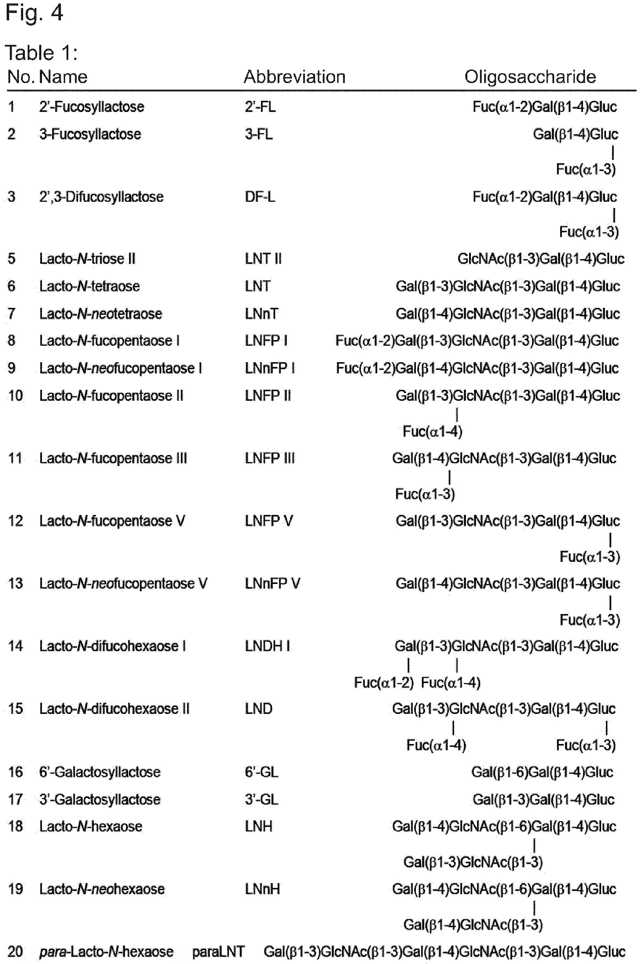

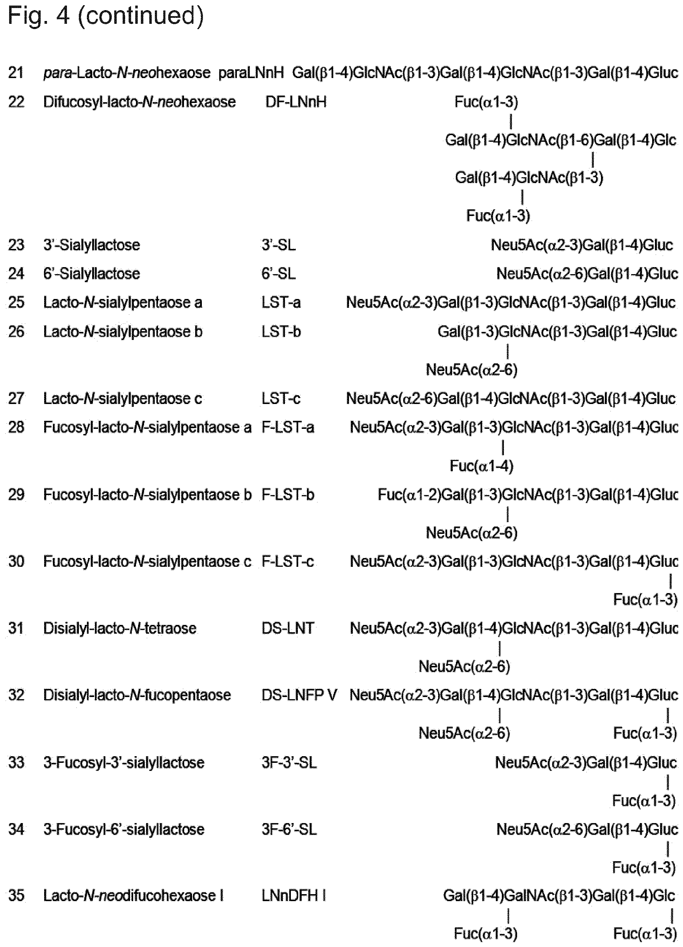

**Table 1: list of oligosaccharides that can be produced according to the invention (Fig. 4)**

| No. | Name | Abbreviation | Oligosaccharide |
|---|---|---|---|
| 1 | 2'-Fucosyllactose | 2'-FL | Fuc(α1-2)Gal(β1-4)Gluc |
| 2 | 3-Fucosyllactose | 3-FL | |
| 3 | 2',3-Difucosyllactose | DF-L | |
| 5 | Lacto-*N*-triose II | LNTII | GlcNAc(β1-3)Gal(β1-4)Gluc |
| 6 | Lacto-*N*-tetraose | LNT | Gal(β1-3)GlcNAc(β1-3)Gal(β1-4)Gluc |
| 7 | Lacto-*N*-neotetraose | LNnT | Gal(β1-4)GlcNAc(β1-3)Gal(β1-4)Gluc |
| 8 | Lacto-*N*-fucopentaose I | LNFPI | Fuc(α1-2)Gal(β1-3)GlcNAc(β1-3)Gal(β1-4)Gluc |
| 9 | Lacto-*N*-neofucopentaose I | LNnFP I | Fuc(α1-2)Gal(β1-4)GlcNAc(β1-3)Gal(β1-4)Gluc |
| 10 | Lacto-*N*-fucopentaose II | LNFP II | |
| 11 | Lacto-*N*-fucopentaose III | LNFP III | |
| 12 | Lacto-*N*-fucopentaose V | LNFP V | |
| 13 | Lacto-*N*-neofucopentaose V | LNnFP V | |
| | | | |
| 14 | Lacto-*N*-difucohexaose I | LNDH I | |
| 15 | Lacto-*N*-difucohexaose II | LND | |
| 16 | 6'-Galactosyllactose | 6'-GL | Gal(β1-6)Gal(β1-4)Gluc |
| 17 | 3'-Galactosyllactose | 3'-GL | Gal(β1-3)Gal(β1-4)Gluc |
| 18 | Lacto-*N*-hexaose | LNH | |
| 19 | Lacto-*N*-*neo*hexaose | LNnH | |
| 20 | *para*-Lacto-*N*-hexaose | paraLNT | Gal(β1-3)GlcNAc(β1-3)Gal(β1-4)GlcNAc(β1-3)Gal(β1-4)Gluc |
| 21 | *para*-Lacto-*N-*neohexaose | paraLNnH | Gal(β1-4)GlcNAc(β1-3)Gal(β1-4)GlcNAc(β1-3)Gal(β1-4)Gluc |
| 22 | Difucosyl-lacto-*N-neo*hexaose | DF-LNnH | |
| 23 | 3'-Sialyllactose | 3'-SL | Neu5Ac(α2-3)Gal(β1-4)Gluc |
| 24 | 6'-Sialyllactose | 6'-SL | Neu5Ac(α2-6)Gal(β1-4)Gluc |
| 25 | Lacto-*N*-sialylpentaose a | LST-a | Neu5Ac(α2-3)Gal(β1-3)GlcNAc(β1-3)Gal(β1-4)Gluc |
| 26 | Lacto-*N*-sialylpentaose b | LST-b | |
| 27 | Lacto-*N*-sialylpentaose c | LST-c | Neu5Ac(α2-6)Gal(β1-4)GlcNAc(β1-3)Gal(β1-4)Gluc |
| 28 | Fucosyl-lacto-*N*-sialylpentaose a | F-LST-a | |
| 29 | Fucosyl-lacto-*N*-sialylpentaose b | F-LST-b | |
| 30 | Fucosyl-lacto-*N*-sialylpentaose c | F-LST-c | |
| 31 | Disialyl-lacto-*N*-tetraose | DS-LNT | |
| 32 | Disialyl-lacto-*N-*fucopentaose | DS-LNFP V | |
| 33 | 3-Fucosyl-3'-sialyllactose | 3F-3'-SL | |
| | | | |
| 34 | 3-Fucosyl-6'-sialyllactose | 3F-6'-SL | |
| 35 | Lacto-*N*-*neo*difucohexaose I | LNnDFH I | |

According to a preferred embodiment of the method of the invention in step b), an intracellulary produced nucleotide activated sugar is used by the cell during execution of the method, the nucleotide activated sugar being preferably at least one of the following: GDP-fucose, UDP-*N*-acetylglucosamine, UDP-galactose, UDP-*N-*acetylgalactosamine, CMP-sialic acid

According to another preferred embodiment of the method of the invention, during execution of the method a (non-nucleotide activated) sugar is externally added, wherein the sugar is selected from fucose, glucose, mannose, *N-*acetylglucosamine, galactose, *N*-acetylgalactosamine, sialic acid, and wherein the sugar is nucleotide-activated in said bacterial host cell.

According to one aspect of the invention, the method is a batch or a continuous total fermentation method.

Thus, according to one aspect of the invention, i.e. in a continuous method, a carbon source is constantly added to the medium during the cultivating step of the host cell. By constantly adding the carbon source during the cultivation step, a constant and effective production of the oligosaccharide is accomplished.

According to another aspect, the method according to the invention is or includes a fed-batch fermentation method, with either a constant volume fed-batch culture, where the substrate is fed without diluting the culture, or variable volume fed-batch culture, where the fermentation volume changes with the fermentation time due to the substrate feed.

It is further preferred in an embodiment of the present invention, if the bacterial host cell is further transformed to either lack a gene encoding beta-galactosidase - or comprise a deregulated beta-galactosidase encoding gene-, and L-fucose isomerase, L-fuculose kinase, and UDP-glucose:undecaprenyl phosphate glucose-1-phosphate transferase.

This embodiment has the advantage that intracellular degradation of monosaccharides and production of colonic acid is prevented and in the case of the beta-galctosidase the acceptor molecule is not degraded.

According to a another preferred embodiment, a glycosidase is applied in the method for producing an oligosaccharide, wherein the glycosidase is used for degrading hindering and/or undesired side-products, unused starting substrates and intermediate products generated during the production of the desired oligosaccharide. By means of the glycosidase it can be achieved that, e.g. other (oligo-) saccharides - besides the desired oligosaccharide- , which other (oligo-)saccharides are produced in the microorganism during the synthesis of the desired oligosaccharide, and which other oligosaccharides interfere with the purification step of the desired oligosaccharide, can be metabolised.

One or more glycosidases can be either externally added to the cultivation at the end of the production process according to the invention, which is particularly preferred when endogenous genes of the host microorganism encoding glycosidases have been inactivated or deleted. Alternatively, the glycosidase is endogenously produced by said host microorganism, wherein the nucleic acid sequences encoding the endogenously produced glycosidase is not naturally occurring in said host cell, and wherein said host cell has been stably transformed to express the not naturally occurring glycosidase, and wherein the expression of the glycosidase in the host microorganism is inducable.

According to this embodiment, the oligosaccharide producing microorganism endogenously, i.e. contained in its genome, expresses the glycosidase, e.g. beta-galactosidase, upon external induction, e.g., via temperature or substrate-induced expression, which is otherwise deregulated. This means that during synthesis of the desired oligosaccharide, the glycosidase is deregulated and may be induced, e.g. by temperature or adding a inductor, e.g. tetracyclin, at the end of the fermentation process. The expression of the glycosidase will be induced after a sufficient and/or essentially maximum amount of oligosaccharide has been produced during cultivation of the host microorganism. Subsequently, the expressed glycosidase will degrade undesired saccharide intermediates, substrates, etc., rendering the medium essentially free of the saccharide intermediates or substrates that would otherwise hinder or complicate the purification of the desired oligosaccharide. A couple of suitable inducable expression tools are known in the prior art (see, e.g. Sambrook *et. al,* 1989, *supra*), and one skilled will be able to apply a respectively suitable one for the desired oligosaccharide.

"Regulated" within the present context with reference to a gene is generally understood as a gene, whose expression can be regulated in a controlled fashion, e.g. down- or up-regulated, i.e. the quantity of the synthesised protein encoded by the regulated gene is different, e.g. de-/downregulated or upregulated, from the otherwise unregulated gene.

According to another aspect of the invention, the glycosidase is produced by a second microorganism additionally added to the medium and expressing the glycosidase.

In this embodiment, the glycosidase expressed by the microorganism is either a naturally occurring glycosidase or a glycosidase that is encoded by a nucleic acid sequence that has been stably integrated into the genome of the second microorganism. This embodiment is particularly suitable in a continuous fermentation process for the production of the oligosaccharide, where, e.g., two separate fermentation vessels or containers are provided, whereas one vessel/container is used for the oligosaccharide synthesis reaction and the second vessel/container is essentially employed for the degradation of undesired saccharides.

In the present invention bacterial host cells as disclosed herein are used for producing oligosaccharides comprising a terminal galactose-(1->4)-glucose disaccharide by total fermentation through said cells.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described above and below are those well known and commonly employed in the art.

Further advantages follow from the description of the embodiments and the attached drawings.

It goes without saying that the abovementioned features and the features which are still to be explained below can be used not only in the respectively specified combinations, but also in other combinations or on their own, without departing from the scope of the present invention.

Several embodiments of the invention are illustrated in the figures and explained in more detail in the following description. In the figures:

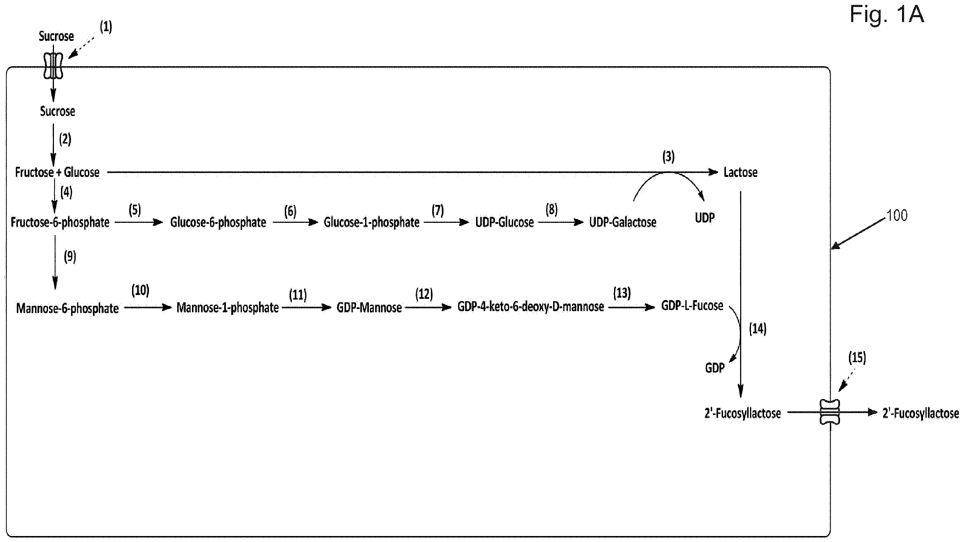

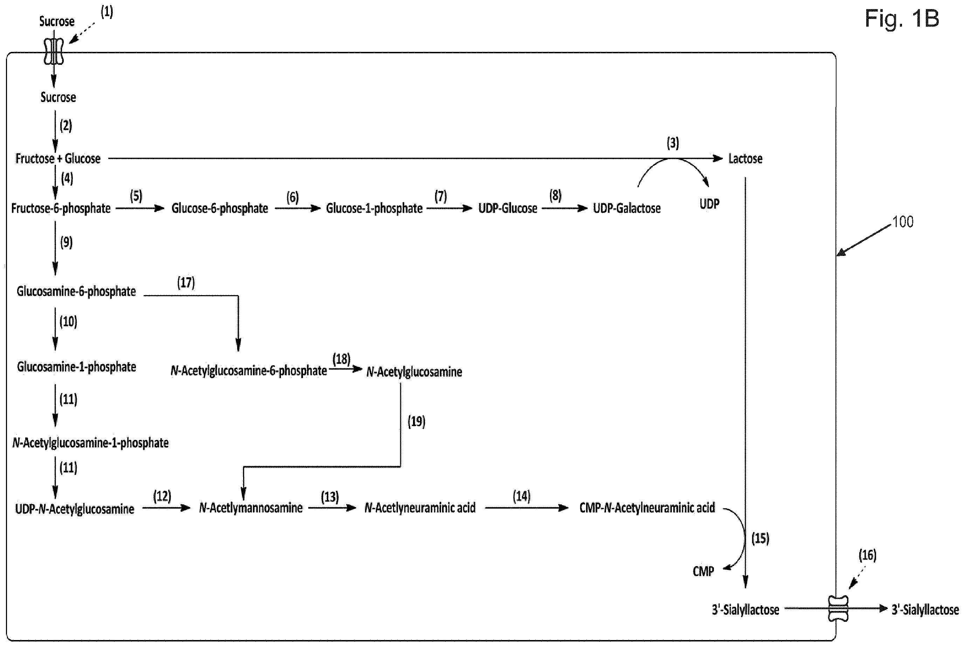

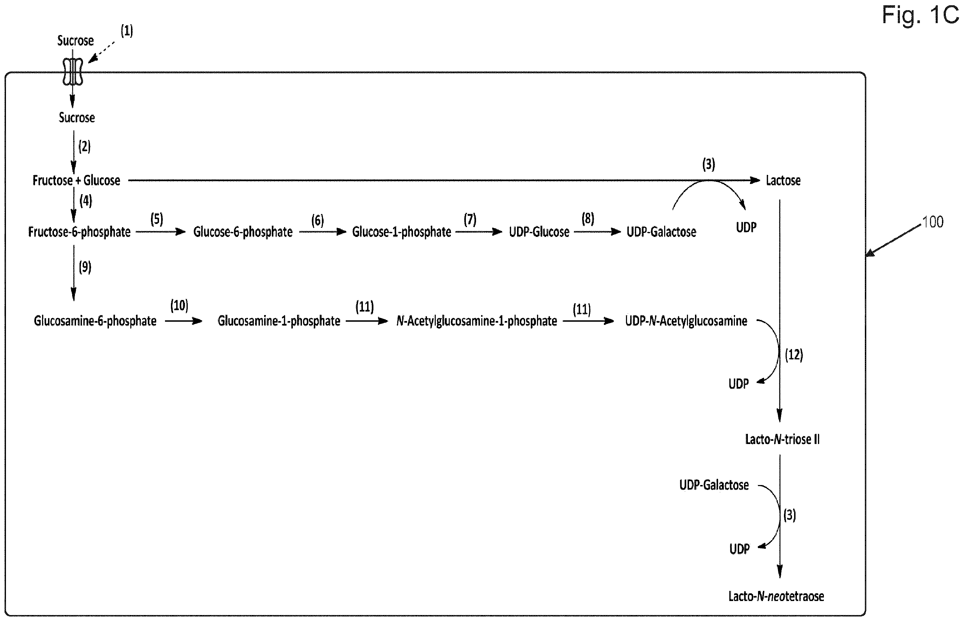

- Fig. 1: shows a schematic drawing for an embodiment of the method according to the invention: production of 2'-fucosyllactose (**A**); 3'-sialyllactose (**B**); and lacto-*N*-*neo*tetraose (**C**);

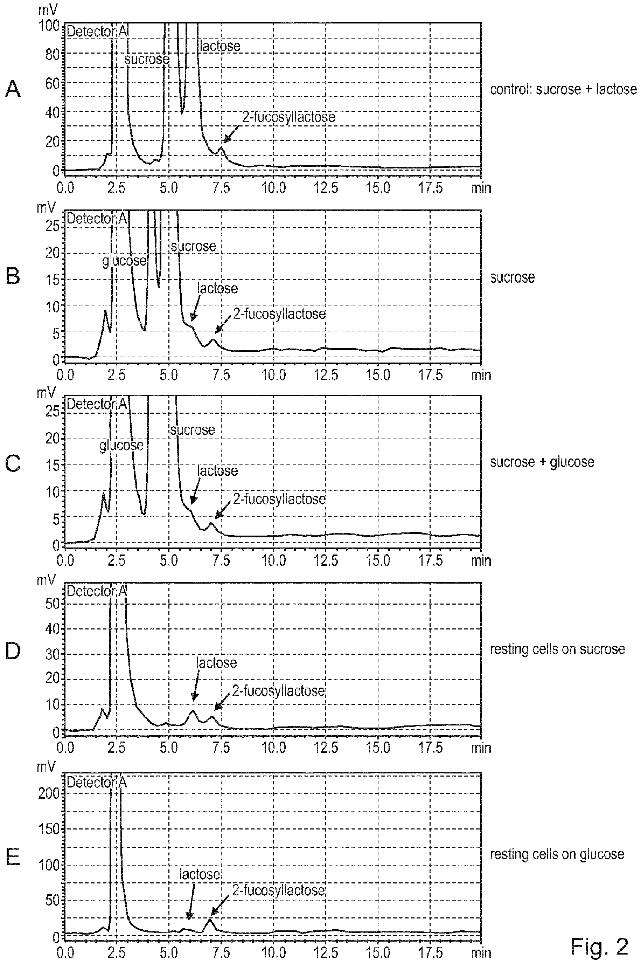

- Fig. 2: shows HPLC analyses of supernatants of cultures of 2'-fucosyllactose producing *E. coli* strains: cells grown in the presents of sucrose and lactose without addition of IPTG (**A**); cells grown on sucrose (**B**), cells grown on sucrose plus glucose (**C**); resting cells: grown in mineral salts media containing sucrose (**D**), and glucose (**E**);

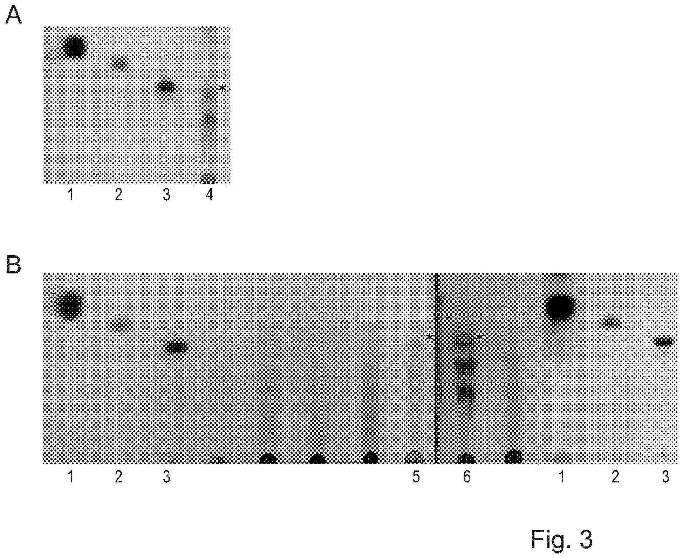

- Fig. 3: shows the results of TLC analyses of culture extracts of lac-to-*N*-neotetraose producing *E. coli* BL21(DE3) strain: (**A**) Culture extract of *E. coli* BL21 (DE3) 1046 (4) grown in the presence of glucose; (**B**) Culture extracts of *E. coli* BL21 (DE3) 724 pCDF-*galE* pINT-*malE-lex1* and *E. coli* BL21 (DE3) 724 pCDF-ga/E pINT*-malE-IgtB,* grown on glucose. LNnT;
- Fig. 4: shows a list (table 1) of oligosaccharides that can be produced according to the invention; and

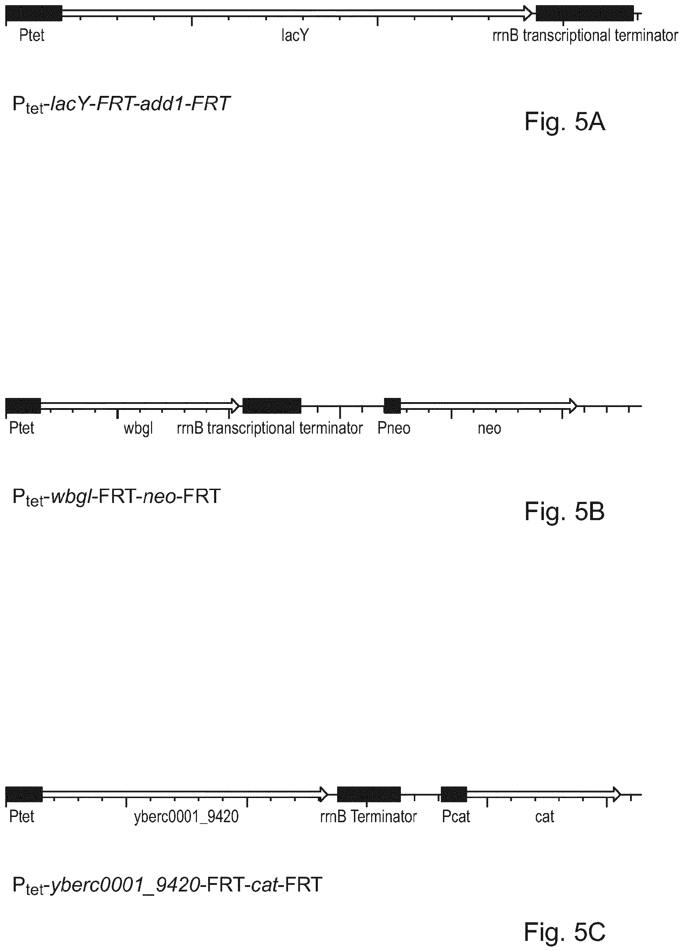

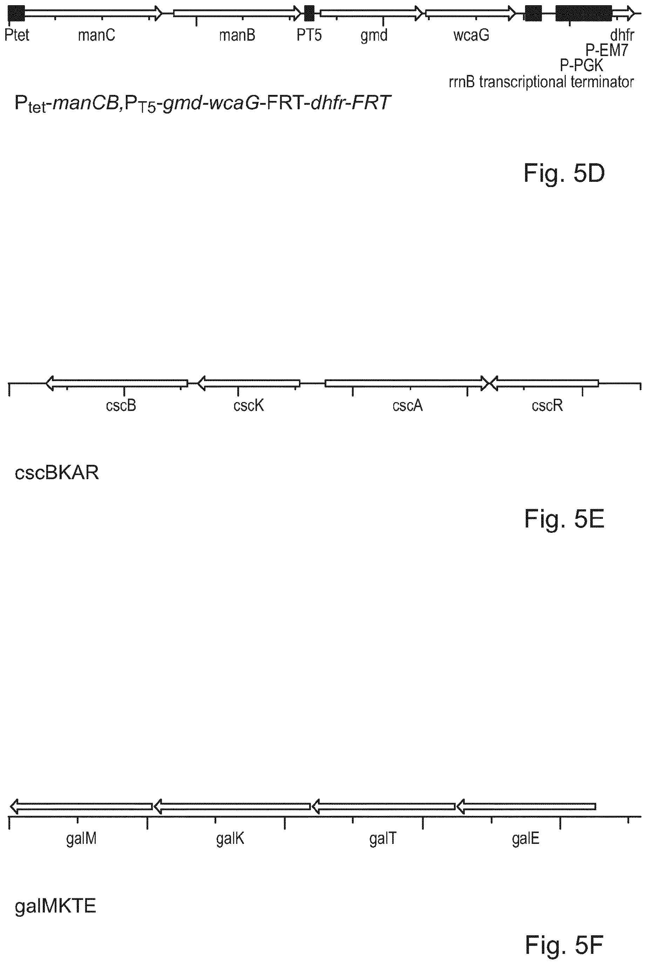

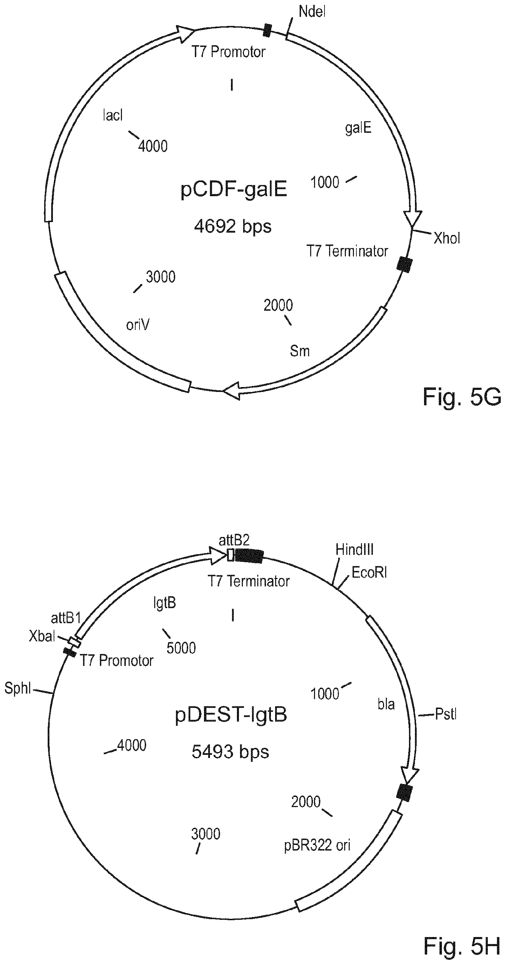

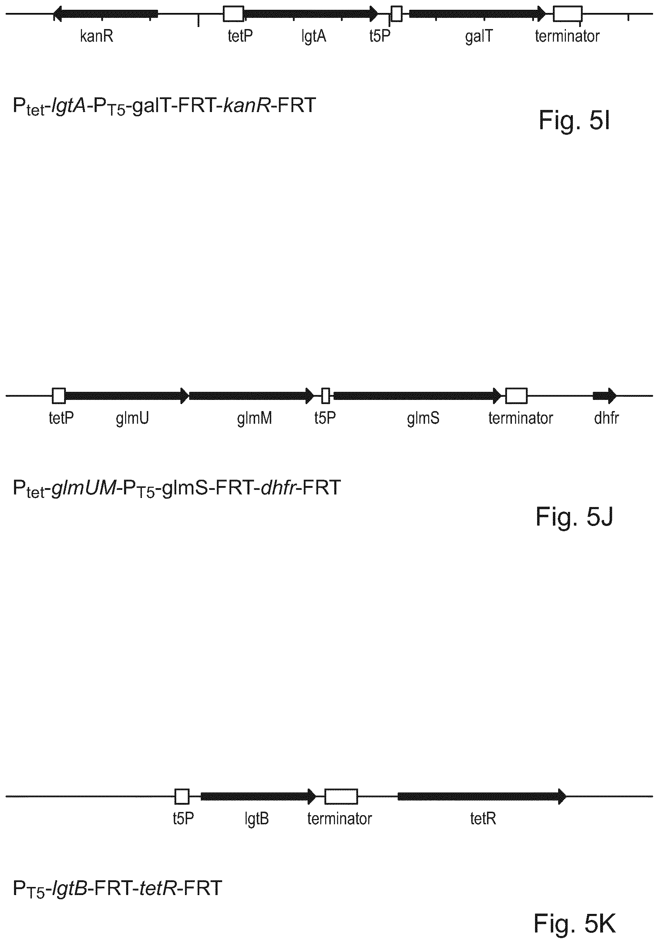

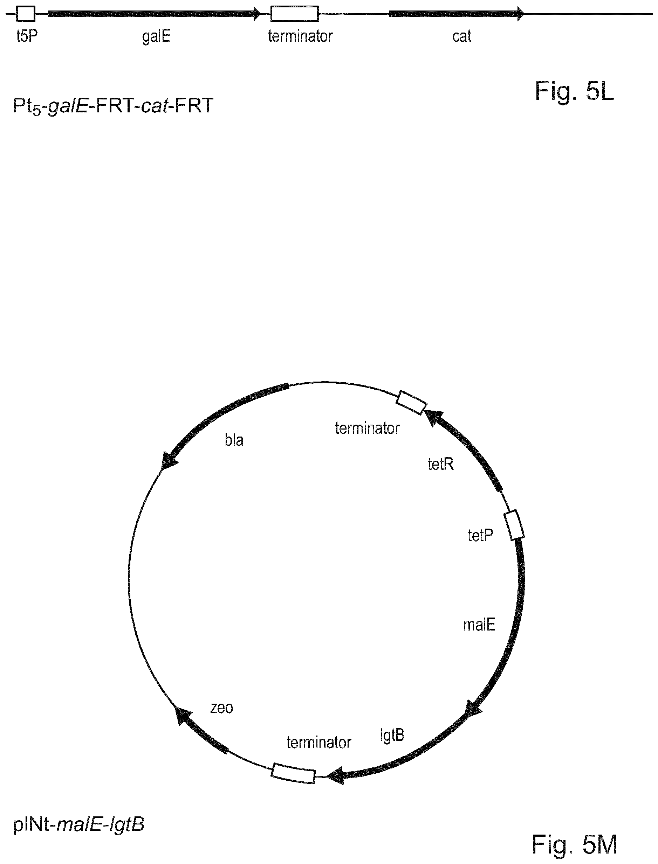

- Fig. 5: shows schematic maps of sequences/plasmids used as exemplary embodiments in the bacterial host cell according to the invention: Pₜₑₜ*-lacY-FRT-add1-FRT* (SEQ ID No. 1 of the attached sequence listing) (**A**); Pₜₑₜ-*wbgL*-FRT-*neo*-FRT SEQ ID No. 2 of the attached sequence listing) (**B**); Pₜₑₜ-*yberc0001_9420-*FRT*-cat-*FRT (SEQ ID No. 3 of the attached sequence listing) (**C**); Pₜₑₜ-*manCB*,P_{T5}-*gmd-wcaG-*FRT-*dhfr-FRT* (SEQ ID No. 4 of the attached sequence listing) (**D**); cscBKAR (SEQ ID No. 5 of the attached sequence listing) (**E**); galMKTE (SEQ ID No. 6 of the attached sequence listing) (**F**); pCDF-*galE* (SEQ ID No. 7 of the attached sequence listing) (**G**); pDEST-*IgtB* (SEQ ID No. 8 of the attached sequence listing) (**H**); Pₜₑₜ-*IgtA*-P_{T5}-galT-FRT-*kanR-*FRT (SEQ ID No. 9 of the attached sequence listing) (**I**); Pₜₑₜ-*glmUM-*P_{T5}-*glmS*-FRT*-dhfr*-FRT (SEQ ID No. 10 of the attached sequence listing) (**J**); P_{T5}-*IgtB*-FRT-*tetR*-FRT (SEQ ID No. 11 of the attached sequence listing) (**K**); Pₜ₅-*galE*-FRT-*cat*-FRT FRT (SEQ ID No. 12 of the attached sequence listing) (**L**); pINT-*malE-IgtB* (SEQ ID No. 13 of the attached sequence listing) (**M**).

### Detailed Description of preferred embodiments

Fig. 1 shows exemplary pathways for the total fermentation of exemplary oligosaccharides using exemplary embodiments of the bacterial host cells and using the methods of the invention. In figures 1A, 1B and 1C, reference number 100 designates an exemplary bacterial host cell that has been genetically modified according to the invention, with respect to different oligosaccharides of interest.

As can be seen from fig. 1A, which shows the schematic exemplary production, i.e. total fermentation, of 2'-fucosyllactose, sucrose is exemplary used as carbon source. Via a sucrose permease (1), the sucrose is taken up or internalized into the host cell. Within the bacterial host cell, sucrose is degraded by invertase (2) to fructose and glucose. The fructose is phosphorylated by fructokinase (4) to fructose-6-phosphate, which can either be converted by a phosphohexose isomerase (5) to glucose-6-phosphate, fod by a conversion by phosphoglucomutase (6) to glucose-1-phosphate, which in turn is converted by glucose-1-phosphate uridyltransferase (7) to UDP-glucose. UDP-glucose is reacted to UDP-galactose, catalyzed by enzyme UDP-galactose-4-epimerase (8). beta-1,4-galactosyltransferase (3) then catalyzses the reaction UDP-galactose + glucose => UDP + lactose.

Thus, the intracellularly produced lactose can be subsequently converted and used as a basis for oligosacchride production (see examples above). In the embodiment shown in Fig. 1, lactose is converted by enzyme alpha-1,2-fucosyltransferase (14) - using GDP-fucose as donor substrate - to the end product, i.e. 2'-fucosyllactose.

The UDP-fucose may be produced intracellularly (or added): enzyme mannose phosphate isomerase (9) catalyzes the conversion of fructose-6-phosphate to mannose-6-phosphate, which is converted by enzyme phosphomannomutase (10) to mannose-1-phosphate. Enzyme mannose-1-phosphate guanylyltransferase (11) catalyzes the conversion of mannose-1-phosphate to GDP-mannose, which is converted to GDP-4-keto-6-deoxy-D-mannose by GDP-D-mannose 4,6 dehydratase (12). Then, GDP-L-fucose synthase (13) catalyzes the reaction to GDP-fucose, which is used as donor substrate by enzyme alpha-1,2-fucosyltransferas (14) as described above. The thus intracellularly produced 2'-fucosyllactose is subsequently exported by an exporter or permease (15).

Fig. 1B shows the schematic exemplary production, i.e. total fermentation of 3'-sialyllactose using an exemplary embodiment of the bacterial host cell and using the method according to the invention, using the following enzymes: (1) sucrose permease, (2) invertase, (3) β-1,4-galactosyltransferase, (4) fructokinase, (5) glucose-6-phosphate isomerase, (6) phosphoglucomutase, (7) glucose-1-phosphate uridylyltransferase, (8) UDP-galactose-4-epimerase, (9) L-glutamine:D-fructose-6-phosphate aminotransferase, (10) phosphoglucosamine mutase, (11) *N*-acetylglucosamine-1-phosphate uridyltransferase/ glucosamine-1-phosphate acetyl-transferase, (12) UDP-*N*-acetylglucosamin-2-epimerase, (13) sialic acid synthase, (14) CMP-sialic acid synthetase, (15) alpha-2,3-sialyltransferase, (16) sugar efflux transporter, (17) glucosamine-6-phosphate-acetyltransferase, (18) phosphatase, (19) *N*-acetylglucosamine-2-epimerase.

Fig. 1C shows the schematic exemplary production, i.e. total fermentation of lacto-*N-neo*tetraose, using an exemplary embodiment of the bacterial host cell and using the method according to the invention, using the following enzymes: (1) sucrose permease, (2) sucrose-6-phosphate hydrolase, (3) beta-1,4-galactosyltransferase, (4) fructokinase, (5) glucosephosphate isomerase, (6) phosphoglucomutase, (7) glucose-1-phosphate uridylyltransferase, (8) UDP-galactose-4-epimerase, (9) L-glutamine:D-fructose-6-phosphate aminotransferase, (10) phosphoglucosamine mutase, (11) *N-*acetylglucosamine-1-phosphate uridyltransferase/ glucosamine-1-phosphate acetyltransferase, (12) beta-1,3-*N*-acetylglucosamine transferase.

As outlined further above, the bacterial host cell according to the invention may be transformed to comprise - besides the beta-1,4,galactosyltransferade and a glycosyltransferase as defined above, at least one, i.e. one or more, of the enzymes as shown in fig. 1A to 1C, and outlined in the description.

### Example 1

### Development of the E. coli 2'-fucosyllactose production strain

*Escherichia coli* BL21 (DE3) was used to construct a 2'-fucosyllactose producing strain. Metabolic engineering included mutagenesis and deletions of specific genes, respectively, and genomic integrations of heterologous genes.

The genes *lacZ* and *araA* were inactivated by mutagenesis using mismatch-oligonucleotides as described by Ellis et al., "High efficiency mutagenesis, repair, and engineering of chromosomal DNA using single-stranded oligonucleotides", Proc. Natl. Acad. Sci. USA 98: 6742-6746 (2001).

Genomic deletions were performed according to the method of Datsenko and Warner ("One-step inactivation of chromosomal genes in Escherichia coli K-12 using PCR products", Proc. Natl. Acad. Sci. USA 97:6640-6645 (2000)). To prevent intracellular degradation of L-fucose, genes encoding L-fucose isomerase (*fucI*) and L-fuculose kinase (*fucK*) had been deleted from the genome of the *E. coli* strain BL21 (DE3) strain. Also genes *wzxC-wcaJ* were deleted. *WcaJ* probably encodes a UDP-glucose:undecaprenyl phosphate glucose-1-phosphate transferase catalysing the first step in colanic acid synthesis (Stevenson et al., "Organization of the Escherichia coli K-12 gene cluster responsible for production of the extracellular polysaccharide colonic acid", J. Bacteriol. 178:4885-4893; (1996)); production of colanic acid would compete for GDP-fucose with the fucosyltransferase reaction. In addition the genes *nagA* and *nagB,* coding for *N*-acetylglucosamine-6-phosphate deacetylase and glucosamine-6-phosphate deaminase, respectively, were removed.

Genomic integration of heterologous genes was performed by transposition. Either the EZ-Tn5™ transposase (Epicentre, USA) was used to integrate linear DNA-fragments or the hyperactive C9-mutant of the mariner transposase *Himar1* (Lampe et al., "Hyperactive transposase mutants of the Himar1 mariner transposon", Proc. Natl. Acad. Sci. USA 96:11428-11433 (1999)) was used for transposition. To produce EZ-Tn5 transposomes the gene of interest together with a FRT-site flanked antibiotic resistance cassette was amplified with primers 1119 and 1120 (all primers used are listed in table 3); the resulting PCR-product carried on both sites the 19-bp Mosaic End recognition sites for the EZ-Tn5 transposase. For integration using *Himar1* transposase operons of interest together with FRT-site flanked antibiotic resistance cassettes were cloned into vector pEcomar. The transposase is encoded on pEcomar under control of P_{araB}. EZ-Tn5 transposons <Pₜₑₜ-*lacY*-FRT-*aadA*-FRT> (SEQ ID No. 1 of the sequence listing), <Pₜₑₜ-*wbgLco-*FRT-*neo*-FRT> (SEQ ID No. 2 of the sequence listing), and <Pₜₑₜ-*yberc0001_9420*-FRT-*cat*-FRT> (SEQ ID No. 3 of the sequence listing) were integrated mediated by EZ-Tn5 transposase.

After successful integration of the gene for the lactose importer LacY from *E. coli* K12 TG1 (acc. no. ABN72583) the resistance gene was eliminated from streptomycin resistant clones by the FLP recombinase encoded on plasmid pCP20 (Datsenko and Warner, "One-step inactivation of chromosomal genes in Escherichia coli K-12 using PCR products", Proc. Natl. Acad. Sci. USA 97:6640-6645 (2000)).

The alpha-1,2-fucosyltransferase gene *wbgL* from *E. coli*:O126 (acc. no. ADN43847) was codon-optimized for expression in *E. coli* and prepared synthetically by GenScript Cooperation (USA). The gene *yberc0001_9420* encoding a sugar efflux transporter of the major facilitator superfamily from *Yersinia bercovieri* ATCC 43970 (acc.no. EEQ08298) was synthesized by GenScript Cooperation (USA) and inserted as EZ-Tn5 <Pₜₑₜ-*yberc0001_9420*-FRT*-cat*-FRT> transposon.

To enhance *de novo* synthesis of GDP-fucose, genes encoding phosphomannomutase (*manB*), mannose-1-phosphate guanosyltransferase (*manC*), GDP-mannose-4,6-dehydratase (*gmd*), and GDP-L-fucose synthase (wcaG) from *E. coli* K12 DH5α were overexpressed in the *E*. *coli* BL21(DE3) strain; the operon *manCB* is under control of Pₜₑₜ, the operon *gmd, wcaG* is transcribed from the P_{T5} promoter. The transposon cassette <Pₜₑₜ-*manCB*-P_{T5}-*gmd*, *wcaG*-FRT-*dhfr*-FRT>>SEQ ID No. 4, flanked by the inverted terminal repeats specifically recognized by the mariner-like element *Himar1* transposase was inserted from pEcomar *C9-manCB-gmd, wcaG-dhfr.*

The csc-gene cluster (SEQ ID No. 5 of the sequence listing) of *E*. *coli W* (acc. no. CP002185.1) comprises four genes for sucrose permease, fructokinase, sucrose hydrolase, and a transcriptional repressor (genes *cscB, cscK, cscA,* and *cscR,* respectively), that enable the strain to grow on sucrose as sole carbon source. This *csc*-cluster was integrated into the genome of the *E*. *coli* BL21 (DE3) strain by transposition using plasmid pEcomar-cscABKR.

The *gal*-operon (*galETKM*) (SEQ ID No. 6 of the sequence listing) was amplified from *E. coli* K12 TG1 using primers 605 and 606 and inserted into the *galM ybhJ* locus of *E. coli* BL21 (DE3) strain by homologous recombination facilitated by using the red recombinase helper plasmid pKD46 (Datsenko and Warner, "One-step inactivation of chromosomal genes in Escherichia coli K-12 using PCR products", Proc. Natl. Acad. Sci. USA 97:6640-6645 (2000)).

### Plasmid construction

pCDF-ga/E (SEQ ID No. 7 of the sequence listing): Using genomic DNA of *E. coli* K12 as template, *galE* was amplified using primers 1163 and 1162. The PCR product was purified, restricted with restriction endonucleases *Nde*I and *Xho*I and ligated into the second multiple cloning site of vector pCDF (Novagen), which was cut with the same enzymes.

pDest-*lgtB* (SEQ ID No. 8 of the sequence listing): The gene for the b-1,4-galactosyltransferase *lgtB* from *Neisseria meningitis* was cloned via a gateway reaction (Invitrogen) into vector pDEST14.

**Table 2: Strains and plasmids**

| Strain | Genotype | Ref. |
|---|---|---|
| *E. coli* BL21 (DE3) | F-*omp*T *hsd*SB(rB-, mB-) *gal dcm* (DE3) | Novagen |
| *E. coli* BL21 (DE3) 878 | *E. coli* BL21 (DE3) *ΔlacZ Δara ΔwcaJ ΔfuclK ΔnagAB* harboring genomic integrations of: *galETKM, lacY, cscBKAR, manCB-gmd, wcaG-dhfr, wbglco-neo, yberc0001_9420-cat* | This study |
| pCDF-galE | *galE* integrated into *Nde*I *Xho*I sites of pCDF | This study |
| pDEST-IgtB | *IgtB* integrated into pDEST expression vector | This study |

**Table 3: oligonucleotides used for PCR**

| Primer | Sequence 5'-3' |
|---|---|
| 605 Kl gal fwd | TTACTCAGCAATAAACTGATATTCCGTCAGGCTGG (SEQ ID No. 15) |
| 606 Kl gal rev | |
| 1119 ME-for | |
| 1120 ME rev | |
| 1163 galE_NdeI-for | GATCACATATGAGAGTTCTGGTTACCGGTG (SEQ ID No. 19) |
| 1164 galE_XhoI-rev | GATCACTCGAGTCATTAATCGGGATATCCCTGTGGATGGC (SEQ ID No. 20) |

### 2'-fucosyllactose production by resting cells

*E. coli* BL21 (DE3) 878 harboring plamids pCDF-*galE* and pDEST-*IgtB* was grown in 2YT broth containing ampicillin 100 µg ml⁻¹ and streptomycin 50 µg ml⁻¹ at 30°C. 300 ml fresh 2YT broth containing the same antibiotics were inoculated with 0.3 % (vol/vol) of the overnight grown culture. The cells were grown at 30°C to an OD660nm of 0.3, and gene expression of *galE* and *IgtB* was induced by addition of 0.35 mM IPTG. The cells were sterile harvested by centrifugation after 5 h growth at 30°C, and resuspended in 50 ml sterile mineral salts medium (Samain et al., "Production of O-acetylated and sulphated chitooligosaccharides by recombinant Escherichia coli strains harbouring different combinations of nod genes", J. Biotechnol. 72:33-47 (1999)) containing the above mentioned antibiotics and 2 % (wt/vol) sucrose, and glucose, respectively, as carbon source. The cells were cultivated at 25°C for four days.

2'-fucosyllactose secreted into the supernatant was detected by HPLC.

### Fermentation of 2'-fucosyllactose by overexpression of galE and IgtB

*E. coli* BL21 (DE3) 878 harboring plamids pCDF-*galE* and pDEST-*IgtB* was grown at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) containing ampicillin 100 µg ml⁻¹ and streptomycin 50 µg ml⁻¹ and as carbon source 2% (wt/vol) sucrose, and 1.6 % (wt/vol) sucrose and 0.4 % (wt/vol) glucose, respectively. When the cultures reached an OD660nm of 0.3 gene expression of *galE* and *IgtB* was induced by addition of 0.35 mM IPTG. After induction cells were grown for four days at 25°C.

As a control *E*. *coli* BL21 (DE3) 878 was grown in mineral salts medium containing 2 % (wt/vol) sucrose as carbon source. When an OD660nm of 0.5 was reached 10 mM lactose was added. Cells were grown for two days at 30°C.

2'-fucosyllactose secreted into the supernatant was detected by HPLC.

### Analysis of culture supernatant and detection of 2'-fucosyllactose by HPLC

Analysis by high performance liquid chromatography (HPLC) was performed using a refractive index detector (RID-10A) (Shimadzu, Germany) and a ReproSil Carbohydrate, 5 µm (250mm × 4,6mm) (Dr. Maisch GmbH, Germany) connected to an HPLC system (Shimadzu, Germany). Elution was performed isocratically with acetonitril:H₂O (68/32 (v/v)) as eluent at 35°C and a flow rate of 1.4 ml/min. 40 µl of the sample were applied to the column. Samples were filtered (0,22 µm pore size) and cleared by solid phase extraction on an ion exchange matrix (Strata ABW, Phenomenex).

The results of the HPLC analyses are shown in Fig. 2, which shows the HPLC analyses of culture supernatants of 2'-fucosyllactose producing *E. coli* BL21 (DE3) 878 pCDF-*galE* pDEST-*IgtB*. Fig. 2A shows *E*. *coli* BL21 (DE3) 878 pCDF-*galE* pDEST-*IgtB* grown in the presents of sucrose and lactose without addition of IPTG; Fig. 2B shows cells grown on sucrose, Fig. 2C shows cells grown on sucrose plus glucose; expression of *galE* and *IgtB* was induced with 0.35 mM IPTG at an OD660nm of 0.3.

Fig. 2D and 2E show the HPLC results of resting cells: *E*. *coli* BL21 (DE3) 878 pCDF-*galE* pDEST-*IgtB* cells were pre-grown in 2YT broth and induced with 0.35 mM IPTG; after harvesting, the cells were grown in mineral salts media containing sucrose (Fig. 2D), and glucose (Fig. 2E), respectively.

As can be seen from the HPLC analyses of Fig. 2, the end product of the exemplary embodiments outlined above, i.e. 2'-fucosyllactose, was efficiently produced upon fermentation of the bacterial host cells according to the invention on sucrose, sucrose plus glucose, resting cells on sucrose, and resting cells on glucose.

### Example 2

### LNnT production by a recombinant E. coli strain by overexpression of galE and a beta-1,4-galactosyltransferase without addition of extracellular lactose

### Development of the E. coli lacto-N-neotetraose production strain

*Escherichia coli* BL21 (DE3) was used to construct an lacto-*N-*neotetraose (LNnT) producing strain. Metabolic engineering included mutagenesis and deletions of specific genes, respectively, and genomic integrations of heterologous genes.

The genes *lacZ* and *araA* were inactivated by mutagenesis using mismatch-oligonucleotides as described by Ellis et al., "High efficiency mutagenesis, repair, and engineering of chromosomal DNA using single-stranded oligonucleotides", Proc. Natl. Acad. Sci. USA 98: 6742-6746 (2001).

Genomic deletions were performed according to the method of Datsenko and Warner ("One-step inactivation of chromosomal genes in Escherichia coli K-12 using PCR products", Proc. Natl. Acad. Sci. USA 97:6640-6645 (2000)). To prevent intracellular degradation of *N*-acetylglucosamine, genes encoding *N*-acetylglucosamine-6-phosphate deacetylase (*nagA*) and glucosamine-6-phosphate deaminase (*nagB*) were deleted from the genome of the *E. coli* strain BL21 (DE3) strain. Also genes *wzxC-wcaJ* were deleted. *WcaJ* encodes an UDP-glucose:undecaprenyl phosphate glucose-1-phosphate transferase catalysing the first step in colanic acid synthesis (Stevenson et al., "Organization of the Escherichia coli K-12 gene cluster responsible for production of the extracellular polysaccharide colonic acid", J. Bacteriol. 178:4885-4893; (1996)). In addition the genes *fucl and fucJ,* coding for L-fucose isomerase and L-fuculose kinase, respectively, were removed.

Genomic integration of heterologous genes was performed by transposition. Either the EZ-Tn5™ transposase (Epicentre, USA) was used to integrate linear DNA-fragments or the hyperactive C9-mutant of the mariner transposase *Himar1* (Lampe et al., "Hyperactive transposase mutants of the Himar1 mariner transposon", Proc. Natl. Acad. Sci. USA 96:11428-11433 (1999)) was employed for transposition. To produce EZ-Tn5 transposomes the gene of interest together with a FRT-site flanked antibiotic resistance marker was amplified with primers 1119 and 1120 (all primers used are listed in table 5); the resulting PCR-product carried on both sites the 19-bp Mosaic End recognition sites for the EZ-Tn5 transposase. For integration using *Himar1* transposase expression constructs (operons) of interest were similarly cloned together with a FRT-site flanked antibiotic resistance marker into the pEcomar vector. The pEcomar vector encodes the hyperactive C9-mutant of the mariner transposase *Himar1* under the control of the arabinose inducible promoter P_{araB}.

The expression fragments <Pₜₑₜ-*lacY*-FRT-*aadA*-FRT> and < Pₜ₅-*galE-*FRT-cat-FRT> (SEQ ID No.12 of the sequence listing) were integrated by using the EZ-Tn5 transposase. After successful integration of the gene for the lactose importer *LacY* from *E*. *coli* K12 TG1 (acc. no. ABN72583) the resistance gene was eliminated from streptomycin resistant clones by the FLP recombinase encoded on plasmid pCP20 (Datsenko and Warner, "One-step inactivation of chromosomal genes in Escherichia coli K-12 using PCR products", Proc. Natl. Acad. Sci. USA 97:6640-6645 (2000)). The *N-*acetylglucosamine glycosyltransferase gene *IgtA* from *Neisseria meningitidis* MC58 (acc. no. NP_274923) was codon-optimized for expression in *E*. *coli and* prepared synthetically by gene synthesis. Together with the gene *galT,* encoding a galactose-1-phosphate uridylyltransferase from *E*. *coli* K-12 substr. MG1655 (acc. no. NP_415279) that was similarly obtained by gene synthesis, *IgtA* was inserted by transposition (SEQ ID No. 9 of the sequence listing) using plasmid pEcomar-*IgtA-galT*. To enhance *de novo* synthesis of UDP-*N*-acetylglucosamine, genes encoding L-glutamine:*D*-fuctose-6-phosphate aminotransferase (*glmS*), phosphoglucosamine mutase from *E*. *coli* K-12 substr. MG1655 (*glmM*) and *N*-acetylglucosamine-1-phosphate uridyltransferase/ glucosamine-1-phosphate acetyltransferase (*glmU*) from *E*. *coli* K-12 substr. MG1655 (acc. no. NP_418185, NP_417643, NP_418186, respectively) were codon-optimized and obtained by gene synthesis. The operon *glmUM* was cloned under the control of constitutive tetracyclin promoter Pₜₑₜ while *glmS* was cloned under the constitutive P_{T5} promoter. The transposon cassette <Pₜₑₜ₋*glmUM-*P_{T5}-*glmS*-FRT-*dhfr*-FRT> (SEQ ID No. 10 of the sequence listing), flanked by the inverted terminal repeats specifically recognized by the mariner-like element *Himar1* transposase was inserted from pEcomar-glmUM-glmS. The beta-1,4-galactosyltransferase gene *IgtB* from *N. meningitidis* MC58 (acc. no. NP_274922) was codon-optimized for expression in *E*. *coli and* prepared synthetically. Incorporation into the genome of *E*. *coli* BL21(DE3) occurred by transposition of the cassette <Pₜ₅-*IgtB*-FRT-*tetR*-FRT> (SEQ ID No. 11 of the sequence listing) from the plasmid pEcomar-*IgtB*. Furthermore, to improve UDP-galactose synthesis, the UDP-galactose-4-epimerase gene *galE* from *E*. *coli* K12 DH5α was overexpressed in the *E*. *coli* BL21(DE3) strain.

The *gal*-operon (*galETKM*) was amplified from *E*. *coli* K12 TG1 using primers 605 and 606 and inserted into the *galM ybhJ* locus of *E*. *coli* BL21(DE3) strain by homologous recombination facilitated by using the red recombinase helper plasmid pKD46 (Datsenko and Warner, "One-step inactivation of chromosomal genes in Escherichia coli K-12 using PCR products", Proc. Natl. Acad. Sci. USA 97:6640-6645 (2000)). Sequences of the heterologous genes and gene clusters are deposit in appendix 1.

### Plasmid construction

Using genomic DNA of *E*. *coli* K12 DH5α as template, *galE* was amplified using primers 1163 and 1162. The PCR product was purified, restricted with restriction endonucleases *Nde*I and *Xho*I and ligated into the second multiple cloning site of vector pCDF (Novagen), which was cut with the same enzymes. *GalE* is expressed from the IPTG inducible T7 promoter. The genes for the β-1,4-galactosyltransferases *IgtB* from *Neisseria meningitis* (acc. no. NP_274922) and lex1 from *Aggregatibacter aphrophilus NJ8700* (acc. no. YP_003008647) were codon-optimized and synthetically synthesized by GenScript Cooperation (USA). Cloning of both genes occurred by sequence and ligation-independent cloning (Li and Elledge, "Harnessing homologous recombination in vitro to generate recombinant DNA via SLIC.", Nat. Methods. 2007 Mar;4(3):251-6. Epub 2007 Feb 11.) Therefore, the plasmid pINT, harbouring the *malE* gene under control of an anhydro-tetracyline-inducible promoter, was used, enabling the generation of a N-terminal fusion of *malE* with the gene the beta-1,4-galactosyltransferase encoded by the *IgtB* gene or *lex1* gene.

**Table 4: Strains and plasmids**

| Strain | Genotype | Ref. |
|---|---|---|
| *E. coli* BL21 (DE3) | F-*omp*T *hsd*SB(rB-, mB-) *gal dcm* (DE3) | Novagen |
| *E. coli* BL21 (DE3) 1046 | *E. coli* BL21 (DE3) *ΔlacZ Δara ΔwcaJ ΔfuclK ΔnagAB* harbouring genomic integrations of: *galETKM, lacY, IgtA-galT-kanR, glmUM-glmS-dhfr, IgtB-tetR, galE-cat* | This study |
| *E. coli* BL21(DE3) 724 | *E. coli* BL21 (DE3) *ΔlacZ Δara ΔwcaJ ΔfuclK ΔnagAB* harbouring genomic integrations of: *galETKM, lacY, IgtA-galT-kanR, glmUM-glmS-dhfr* | This study |
| pCDF-*galE* | *galE* of *E*. *coli* K12 integrated into *Nde*I *Xho*I sites of pCDF | This study |
| pINT-*malE-IgtB* (SEQ ID No. 13) | Gene fusion of *malE with IgtB* of *Neisseria meningitis* integrated into vector pINT | This study |
| pINT-*malE-lex1* (SEQ ID No. 14) | Gene fusion of *malE* with *lex-1* of *Aggregatibacter aphrophilus NJ8700* integrated into vector pINT | This study |

**Table 5: Oligonucleotides used for PCR**

| Primer | Sequence 5'-3' |
|---|---|
| 605 Kl gal fwd | TTACTCAGCAATAAACTGATATTCCGTCAGGCTGG (SEQ ID No. 21) |
| 606 Kl gal rev | |
| 1119 ME-for | |
| 1120 ME rev | CTGTCTCTTATCACATCTCACATTACATCTGAGCGATTGTTAGG (SEQ ID No. 24) |
| 1163 galE_NdeI-for | GATCACATATGAGAGTTCTGGTTACCGGTG (SEQ ID No. 25) |
| 1164 galE-XhoI-rev | GATCACTCGAGTCATTAATCGGGATATCCCTGTGGATGGC (SEQ ID No. 26) |
| 5176 lex1 pINT-f | |
| 5177 lex1 pINT-r | |
| | |
| 5178 pINT lex1-f | |
| 5179 pINT lex1-r | |
| 5180 IgtB pINT-f | |
| 5181 IgtB pINT-r | |
| 5182 pINT IgtB-f | |
| 5183 pINT IgtB-r | |

### Batch fermentation of E. coli BL21 (DE3) 1046

*E. coli* BL21 (DE3) 1046 was grown for 48 hours at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) containing 2 % (wt/vol) glucose as sole carbon and energy source. Cells were harvested and mechanically disrupted. The production of LN*n*T was detected by thin layer chromatography.

### Batch fermentation of E. coli BL21 (DE3) 724 pCDF-galE pINT-malE-IgtB/pINT-malE-lex1

*E. coli* BL21 (DE3) 724 harbouring plamids pCDF-ga/E and either pINT-malE-*IgtB* or pINT-malE-*lex1* was grown at 30°C in mineral salts medium (Samain et al., "Production of O-acetylated and sulphated chitooligosaccharides by recombinant Escherichia coli strains harbouring different combinations of nod genes", J. Biotechnol. 72:33-47 (1999)) supplemented with 2 % (wt/vol) glucose, ampicillin 100 µg ml⁻¹ and zeocin 40 µg ml⁻¹. When the cultures reached an OD660nm of 0.1, gene expression of the *galE* gene and the β1,4-galactosyltransferase was induced by addition of 0.3 mM IPTG and 200 ng/ml anhydrotetracycline. After incubation for 48 hours at 30°C in shaking flasks cells were harvested and mechanically disrupted. LNnT was detected by thin layer chromatography.

### Detection of lacto-N-neotetraose (LnNT) by thin layer chromatography (TLC)

Cells war mechanically disrupted using glass beads. Subsequently, samples were applied on TLC Silica Gel 60 F₂₅₄ (Merck, Germany). The mobile phase was composed of acetone:butanol:acetic acid:water (35:35:7:23).

### TLC analyses of culture extracts of lacto-N-neotetraose producing E. coli BL21 (DE3) strain

Fig. 3 shows TLC (thin layer chromatography) analyses of culture extracts of lacto-*N*-*neo*tetraose producing *E. coli* BL21 (DE3) cells. Fig. 2A shows the results for a culture extract of *E*. *coli* BL21 (DE3) 1046 (4) grown in the presence of glucose, with the following standards: lactose (lane 1), lacto-*N*-triose II (lane 2) and lacto-*N*-neotetraose (lane 3). Fig. 3B shows the results for culture extracts of *E*. *coli* BL21 (DE3) 724 pCDF-*galE* pINT-*malE-lex1* (lane 5) and *E*. *coli* BL21 (DE3) 724 pCDF-*galE* pINT-*malE-IgtB* (lane 6) grown on glucose. LN*n*T product formation in the samples is marked with asterisks.

Thus, the results presented in Fig. 3 show the efficient production of lacto-N-neotetraose that was effected with the methods according to the invention.

## Claims

1. A method for producing at least one oligosaccharide of interest by total fermentation through genetically modified host cells, the oligosaccharide of interest comprising a terminal galactose-(1->4)-glucose disaccharide, and the method comprising the steps of:
a) obtaining
(i) a bacterial host cell being capable to produce oligosaccharides comprising a terminal galactose-(1->4)-glucose disaccharide, and comprising
- at least one recombinant nucleic acid sequence encoding for a protein having a beta-1,4-galactosyltransferase activity and being able to galactosylate a free glucose monosaccharide to intracellularly generate lactose,
- at least one recombinant nucleic acid sequence encoding a glycosyltransferase that is selected from at least one of the following: a fucosyltransferase, a sialyltransferase, a glucosamyltransferase, a galactosyltransferase,
and which bacterial host cell is capable of generating the oligosaccharide without the exogenous addition of lactose, or
(ii) a first bacterial host cell comprising a recombinant nucleic acid sequence encoding for a protein having a beta-1,4-galactosyltransferase activity and being able to galactosylate a free glucose monosaccharide to intracellularly generate lactose, and a second bacterial host cell comprising at least one recombinant nucleic acid sequence encoding a glycosyltransferase that is selected from at least one of the following: a fucosyltransferase, a sialyltransferase, a glucosamyltransferase, a galactosyltransferase;
b) culturing said bacterial host cells (i) or (ii) from step a), wherein said culturing is performed (I) from a carbon source that is selected from at least one of the following: glucose, sucrose, glycerol, molasses, corn syrup, galactose, syngas, carbon monoxide, methanol, succinate, pyruvate, raffinose, wherein the host cell is capable of generating free glucose within the cell from said carbon source, and (II) without externally supplying lactose during execution of the method, thus producing said oligosaccharide of interest.

2. The method of claim 1, wherein the oligosaccharide of interest is a human milk oligosaccharide, preferably a human milk oligosaccharide selected from at least one of the ones listed in the attached table 1, preferably selected from 2'-fucosyllactose, 3-fucosyllactose, 2',3-difucosyllactose, 3'-sialyllactose, 6'-sialyllactose, 3-fucosyl-3'-sialyllactose, lacto-*N*-tetraose, lacto-*N*-*neo*tetraose, lacto-*N*-fucopentaose I, lacto-*N-*fucopentaose II, lacto-*N*-fucopentaose III, lacto-*N*-fucopentaose V, lacto-*N-*difucosylhexaose I, lacto-*N*-difucosylhexaose II, lacto-*N*-sialylpentaose LSTa, LSTb, LSTc.

3. The method of claim 1 or 2, wherein in step b), an intracellulary produced nucleotide activated sugar is used by the cell during execution of the method, the nucleotide activated sugar being preferably at least one of the following: GDP-fucose, GDP-mannose, UDP-glucose, UDP-*N*-acetylglucosamine, UDP-galactose, UDP-*N-*acetylgalactosamine, CMP-sialic acid.

4. The method of any of the preceding method claims, wherein during execution of the method a non-nucleotide activated sugar is externally added, wherein the non-nucleotide activated sugar is selected from fucose, glucose, mannose, *N-*acetylglucosamine, galactose, *N*-acetylgalactosamine, sialic acid, and wherein the non-nucleotide activated sugar is nucleotide-activated in said bacterial host cell.

5. The method of any of the preceding method claims, further including step c) following step b), wherein step c) is performed after a desired amount of said oligosaccharide has been produced, and wherein step c) comprises (i) inducing the expression of at least one glycosidase contained in said bacterial host cell, or (ii) adding of a second bacterial host expressing at least one glycosidase to the medium the bacterial host cell is cultivated in, thus degrading unwanted oligosaccharides.

6. The method of any of the preceding claims, wherein the glycosyltransferase is selected from at least one of the following: alpha-1,2-fucosyltransferase, alpha-1,3-fucosyltransferase, beta-1,3-*N*-acetyl-glucosamyltransferase, beta-1,3-galactosyltransferase, beta-1,4-galactosyltransferase, alpha-2,6-sialyltransferase, alpha-2,3-sialyltransferase, beta-1,6-galactosyltransferase, or a functional fragment thereof.

7. The method of any of the preceding claims, wherein the bacterial host cell further comprises a nucleic acid sequence encoding at least one of the following additional proteins: an exporter protein or a permease exporting the synthesized oligosaccharide from the host cell.

8. The method of claim 7, wherein the exporter protein is the sugar efflux transporter encoded by the yberc0001_9420 gene of *Yersinia bercovieri* ATCC 43970 or a functional fragment thereof.

9. The method of any of the preceding claims, wherein the bacterial host cell further comprises a nucleic acid sequence encoding at least one of the following additional proteins: an invertase, a fructokinase, proteins involved in the biosynthesis of GDP-fucose, GDP-mannose, UDP-glucose, UDP-*N*-acetylglucosamine, UDP-galactose, UDP-*N*-acetylgalactosamine, UDP-glucuronic acid, CMP-sialic acid.

10. The method of claim 8 or 9, wherein the nucleic acid is an endogenous or a recombinant nucleic acid.

11. The method of any of claims 8 to 10, wherein the nucleic acid is homologous or heterologous.

12. The method of any of the preceding claims, wherein the bacterial host cell further comprises control sequences enabling the controlled overexpression of endogenous or recombinant nucleic acid sequences.

13. The method of any of the preceding claims, wherein the beta-1,4-galactosyltransferase is encoded by a gene selected from the group consisting of IgtB from *Neisseria meningitidis,* and lex-1 from *Aggregatibacter aphrophilus* NJ8700, or functional fragments thereof.

14. The method of any of the preceding claims, wherein the bacterial host cell is a *Escherichia coli* strain, a *Lactobacillus* species, a *Corynebacterium glutamicum* strain, or a *Clostridium* strain.

15. The method of any of the preceding claims, wherein the bacterial host cell further comprises at least one of the genes of the *E. coli* W csc gene cluster.

16. The method of any of the preceding claims, wherein the bacterial host cell is modified not to express proteins intracellularly degrading precursors of said oligosaccharide.

17. The method of any of the preceding claims, wherein the bacterial host cell is further genetically modified to contain a nucleic acid sequence encoding a glycosidase for the specific degradation of interfering oligosaccharides, such as intermediates, side products or endogenous oligosaccharides generated by bacterial host cell, wherein the expression of said glycosidase is under control of a regulatory sequences.

## Patentansprüche

1. Ein Verfahren zur Herstellung mindestens eines Oligosaccharids von Interesse durch Totalfermentation mittels genetisch veränderter Wirtszellen, das Oligosaccharid von Interesse weist ein endständiges Galactose-(1->4)-Glucose Disaccharid auf, und das Verfahren umfasst die Schritte:
a) Erhalten
(i) einer bakteriellen Wirtszelle, die in der Lage ist Oligosaccharide herzustellen, welche ein endständiges Galactose-(1->4)-Glucose Disaccharid aufweisen, und aufweist
- mindestens eine rekombinante Nukleinsäuresequenz, die für ein Protein kodiert, das eine beta-1,4-Galactosyltransferase-Aktivität hat und in der Lage ist, ein freies Glucose Monosaccharid zu galactosylieren, um intrazellulär Lactose zu generieren,
- mindestens eine rekombinante Nukleinsäuresequenz, die eine Glycosyltransferase kodiert, welche aus mindestens einer der Folgenden ausgewählt ist: einer Fucosyltransferase, einer Sialyltransferase, einer Glucosamyltransferase, einer Galactosyltransferase,
und welche bakterielle Wirtszelle in der Lage ist, das Oligosaccharid ohne Zugabe von Laktose von außen zu generieren, oder
(ii) einer ersten bakteriellen Wirtszelle, die eine rekombinante Nukleinsäuresequenz aufweist, die ein Protein kodiert, das eine beta-1,4-Galactosyltransferase-Aktivität hat und in der Lage ist, ein freies Glucose Monosaccharid zu galactosylieren, um intrazellulär Lactose zu generieren, und einer zweiten bakteriellen Wirtszelle, die mindestens eine rekombinante Nukleinsäuresequenz aufweist, die eine Glycosyltransferase kodiert, welche aus mindestens einer der folgenden ausgewählt ist: einer Fucosyltransferase, einer Sialyltransferase, einer Glucosamyltransferase, einer Galactosyltransferase;
b) Kultivieren besagter bakterieller Wirtszellen (i) oder (ii) aus Schritt a), wobei das Kultivieren erfolgt (I) mit einer Kohlenstoffquelle, die aus mindestens einer der Folgenden ausgewählt ist: Glucose, Saccharose, Glycerin, Molasse, Maissirup, Galactose, Synthesegas, Kohlenmonoxid, Methanol, Succinat, Pyruvat, Raffinose, wobei die Wirtszelle in der Lage ist, freie Glucose innerhalb der Zelle aus der Kohlenstoffquelle zu generieren, und (II) ohne Zugabe von Lactose von außen während der Durchführung des Verfahrens, wodurch das Oligosaccharid von Interesse herstellt wird.

2. Das Verfahren nach Anspruch 1, wobei das Oligosaccharid von Interesse ein humanes Milcholigosaccharid ist, vorzugsweise ein humanes Milcholigosaccharid, das aus mindestens einem der in der beigefügten Tabelle 1 ausgewählt ist, vorzugsweise ausgewählt ist aus 2'-Fucosyllactose, 3-Fucosyllactose, 2',3-Difucosyllactose, 3'-Sialyllactose, 6'-Sialyllactose, 3-Fucosyl-3'-sialyllactose, Lacto-*N*-tetraose, Lacto-*N-*neotetraose, Lacto-*N*-fucopentaose I, Lacto-*N*-fucopentaose II, Lacto-*N*-fucopentaose III, Lacto-*N*-fucopentaose V, Lacto-*N*-difucosylhexaose I, Lacto-*N*-difucosylhexaose II, Lacto-*N*-sialylpentaose LSTa, LSTb, LSTc.

3. Das Verfahren nach Anspruch 1 oder 2, wobei in Schritt b), ein intrazellulär produzierter Nukleotid-aktivierter Zucker von der Zelle während der Durchführung des Verfahrens verwendet wird, der Nukleotid-aktivierte Zucker ist vorzugsweise mindestens einer der Folgenden: GDP-Fucose, GDP-Mannose, UDP-Glucose, UDP-*N-*Acetylglucosamin, UDP-Galactose, UDP-*N*-Acetylgalactosamin, CMP-Sialinsäure.

4. Das Verfahren nach einem der vorhergehenden Verfahrensansprüche, wobei während der Durchführung des Verfahrens ein nicht-Nukleotid-aktivierter Zucker von außen zugegeben wird, wobei der nicht-nukleotidaktivierte Zucker ausgewählt ist aus Fucose, Glucose, Mannose, *N*-Acetylglucosamin, Galactose, *N*-Acetylgalactosamin, Sialinsäure, und wobei der nicht-nukleotidaktivierte Zucker in der bakteriellen Wirtszelle nukleodidaktiviert wird.

5. Das Verfahren nach jedem der vorhergehenden Ansprüche, weiterhin umfassend Schritt c), der Schritt b) folgt, wobei Schritt c) durchgeführt wird nachdem eine gewünschte Menge des Oligosaccharids hergestellt wurde, und wobei Schritt c) (i) Induzieren der Expression mindestens einer Glycosidase, die in der bakteriellen Wirtszelle enthalten ist, oder (ii) Zugeben einer zweiten bakteriellen Wirtszelle, die mindestens eine Glycosidase exprimiert, zu dem Medium, in dem die bakterielle Wirtszelle kultiviert wird, umfasst, wodurch unerwünschte Oligosaccharide abgebaut werden.

6. Das Verfahren nach jedem der vorhergehenden Ansprüche, wobei die Glycosyltransfersase aus mindestes einer der Folgenden ausgewählt ist: alpha-1,2-Fucosyltransferase, alpha-1,3-Fucosyltransferase, beta-1,3-*N*-Acetylglucosamyltransferase, beta-1,3-Galactosyltransferase, beta-1,4-Galactosyltransferase, alpha-2,6-Sialyltransferase, alpha-2,3-Sialyltransferase, beta-1,6-Galactosyltransferase, oder einem funktionellen Fragment davon.

7. Das Verfahren nach jedem der vorhergehenden Ansprüche, wobei die bakterielle Wirtszelle zudem eine Nukleinsäuresequenz aufweist, die mindestens eines der folgenden zusätzlichen Proteine kodiert: ein Exporterprotein oder eine Permease, die das synthetisierte Oligosaccharid aus der Wirtszelle exportiert.

8. Das Verfahren nach Anspruch 7, wobei das Exporterprotein der Zucker-Efflux-Transporter ist, der durch das yberc0001_9420 Gen von *Yersinia bercovieri* ATCC 43970 oder einem funktionellen Fragment davon kodiert wird.

9. Das Verfahren nach jedem der vorhergehenden Ansprüche, wobei die bakterielle Wirtszelle zudem eine Nukleinsäuresequenz aufweist, die mindestens eines der folgenden zusätzlichen Proteine kodiert: eine Invertase, eine Fructokinase, Proteine die in der Biosynthese von GDP-Fucose, GDP-Mannose, UDP-Glucose, UDP-*N-*Acetylglucosamin, UDP-Galactose, UDP-*N*-Acetylgalactosamin, UDP-Glucuronsäure, CMP-Sialinsäure beteiligt sind.

10. Das Verfahren nach Anspruch 8 oder 9, wobei die Nukleinsäure eine endogene oder eine rekombinante Nukleinsäure ist.

11. Das Verfahren nach jedem der Ansprüche 8 bis 10, wobei die Nukleinsäure homolog oder heterolog ist.

12. Das Verfahren nach jedem der vorhergehenden Ansprüche, wobei die bakterielle Wirtszelle zudem Kontrollsequenzen aufweist, die die kontrollierte Überexpression von endogenen oder rekombinanten Nukleinsäuresequenzen ermöglicht.

13. Das Verfahren nach jedem der vorhergehenden Ansprüche, wobei die beta-1,4-Galactosyltransferase von einem Gen kodiert wird, das aus der Gruppe ausgewählt ist, die aus IgtB von *Nesseria meningitidis,* und lex-1 aus *Aggregatibacter aphrophilus* NJ8700, oder funktionellen Fragmenten davon besteht.

14. Das Verfahren nach jedem der vorhergehenden Ansprüche, wobei die bakterielle Wirtszelle ein *Escherichia coli* Stamm, eine *Lactobacillus* Art, ein *Corynebacterium glutamicum* Stamm oder ein *Clostridium* Stamm ist.

15. Das Verfahren nach jedem der vorhergehenden Ansprüche, wobei die bakterielle Wirtszelle zudem mindestens eines der Gene des *E. coli* W csc Genclusters aufweist.

16. Das Verfahren nach jedem der vorhergehenden Ansprüche, wobei die bakterielle Wirtszelle verändert ist, um Proteine, die Vorläufer des Oligosaccharids intrazellulär abbauen, nicht zu exprimieren.

17. Das Verfahren nach jedem der vorhergehenden Ansprüche, wobei die bakterielle Wirtszelle zudem genetisch verändert ist, um eine Nukleinsäuresequenz zu enthalten, die eine Glycosidase für den spezifischen Abbau interferierender Oligosaccharide, wie Intermediate, Nebenprodukte oder endogene Oligosaccharide die von der bakteriellen Wirtszelle generiert werden, kodiert, wobei die Expression der Glycosidase unter Kontrolle einer regulatorischen Sequenz steht.

## Revendications

1. Méthode de production d'au moins un oligosaccharide d'intérêt par fermentation totale via des cellules hôtes génétiquement modifiées, l'oligosaccharide d'intérêt comprenant un disaccharide de galactose-(1→4)-glucose terminal, et la méthode comprenant les étapes consistant à :
a) obtenir
(i) une cellule hôte bactérienne capable de produire des oligosaccharides comprenant un disaccharide de galactose-(1→4)-glucose terminal, et comprenant
- au moins une séquence d'acide nucléique recombinée codant pour une protéine ayant une activité de bêta-1,4-galactosyltransférase et étant capable de galactosyler un monosaccharide de glucose libre afin de générer du lactose par voie intracellulaire,
- au moins une séquence d'acide nucléique recombinée codant pour une glycosyltransférase qui est choisie parmi au moins l'une parmi ce qui suit : une fucosyltransférase, une sialyltransférase, une glucosamyltransférase, une galactosyltransférase, et laquelle cellule hôte bactérienne est capable de générer l'oligosaccharide sans l'addition exogène de lactose, ou
(ii) une première cellule hôte bactérienne comprenant une séquence d'acide nucléique recombinée codant pour une protéine ayant une activité de bêta-1,4-galactosyltransférase et étant capable de galactosyler un monosaccharide de glucose libre afin de générer du lactose par voie intracellulaire, et une deuxième cellule hôte bactérienne comprenant au moins une séquence d'acide nucléique recombinée codant pour une glycosyltransférase qui est choisie parmi au moins l'une parmi ce qui suit : une fucosyltransférase, une sialyltransférase, une glucosamyltransférase, une galactosyltransférase,
b) la culture desdites cellules hôtes bactériennes (i) et (ii) issues de l'étape a), où ladite culture est effectuée (I) à partir d'une source de carbone qui est choisie parmi au moins l'un parmi ce qui suit : le glucose, le saccharose, le glycérol, les mélasses, le sirop de glucose, le galactose, le gaz de synthèse, le monoxyde de carbone, le méthanol, le succinate, le pyruvate, le raffinose, où la cellule hôte est capable de générer du glucose libre au sein de la cellule à partir de ladite source de carbone, et (II) sans apport externe de lactose lors de la mise en oeuvre de la méthode, produisant ainsi ledit oligosaccharide d'intérêt.

2. Méthode selon la revendication 1, dans laquelle l'oligosaccharide d'intérêt est un oligosaccharide de lait humain, préférablement un oligosaccharide de lait humain choisi parmi au moins l'un de ceux énumérés dans le tableau 1 annexé, préférablement choisi parmi le 2'-fucosyllactose, le 3-fucosyllactose, le 2',3-difucosyllactose, le 3'-sialyllactose, le 6'-sialyllactose, le 3-fucosyl-3'-sialyllactose, le lacto-N-tétraose, le lacto-N-néotétraose, le lacto-N-fucopentaose I, le lacto-N-fucopentaose II, le lacto-N-fucopentaose III, le lacto-N-fucopentaose V, le lacto-N-difucosylhexaose I, le lacto-N-difucosylhexaose II, le lacto-N-sialylpentaose LSTa, LSTb, LSTc.

3. Méthode selon la revendication 1 ou 2, dans laquelle, dans l'étape b), un sucre à activation nucléotidique et produit par voie intracellulaire est utilisé par la cellule lors de la mise en oeuvre de la méthode, le sucre à activation nucléotidique étant préférablement au moins l'un parmi ce qui suit : le GDP-fucose, le GDP-mannose, l'UDP-glucose, l'UDP-N-acétylglucosamine, l'UDP-galactose, l'UDP-N-acétylgalactosamine, le CMP-acide sialique.

4. Méthode selon l'une quelconque des revendications précédentes, dans laquelle, lors de la mise en oeuvre de la méthode, un sucre à activation non nucléotidique est ajouté par voie externe, où le sucre à activation non nucléotidique est choisi parmi le fucose, le glucose, le mannose, la N-acétylglucosamine, le galactose, la N-acétylgalactosamine, l'acide sialique et où le sucre à activation non nucléotidique est activé par un nucléotide dans ladite cellule hôte bactérienne.

5. Méthode selon l'une quelconque des revendications précédentes de méthode, comportant en outre une étape c) suivant l'étape b), où l'étape c) est mise en oeuvre après qu'une quantité désirée dudit oligosaccharide a été produite, et où l'étape c) comprend (i) l'induction de l'expression d'au moins une glycosidase contenue dans ladite cellule hôte bactérienne, ou (ii) l'addition d'un deuxième hôte bactérien exprimant au moins une glycosidase au milieu dans lequel la cellule hôte bactérienne est cultivée, dégradant ainsi les oligosaccharides non désirés.

6. Méthode selon l'une quelconque des revendications précédentes, dans laquelle la glycosyltransférase est choisie parmi au moins l'une parmi ce qui suit : l'alpha-1,2-fucosyltransférase, l'alpha-1,3-fucosyltransférase, la bêta-1,3-N-acétyl-glucosamyltransférase, la bêta-1,3-galactosyltransférase, la bêta-1,4-galactosyltransférase, l'alpha-2,6-sialyltransférase, l'alpha-2,3-sialyltransférase, la bêta-1,6-galactosyltransférase, ou un fragment fonctionnel de celles-ci.

7. Méthode selon l'une quelconque des revendications précédentes, dans laquelle la cellule hôte bactérienne comprend en outre une séquence d'acide nucléique codant pour au moins l'une parmi les protéines supplémentaires suivantes : une protéine exportatrice ou une perméase exportant l'oligosaccharide synthétisé hors de la cellule hôte.

8. Méthode selon la revendication 7, dans laquelle la protéine exportatrice est le transporteur d'efflux de sucre codé par le gène yberc0001_9420 de *Yersinia bercovieri* ATCC 43970 ou un fragment fonctionnel de celui-ci.

9. Méthode selon l'une quelconque des revendications précédentes, dans laquelle la cellule hôte bactérienne comprend en outre une séquence d'acide nucléique codant pour au moins l'une parmi les protéines supplémentaires suivantes : une invertase, une fructokinase, les protéines impliquées dans la biosynthèse de GDP-fucose, de GDP-mannose, d'UDP-glucose, d'UDP-N-acétylglucosamine, d'UDP-galactose, d'UDP-N-acétylgalactosamine, d'UDP-acide glucuronique, de CMP-acide sialique.

10. Méthode selon la revendication 8 ou 9, dans laquelle l'acide nucléique est un acide nucléique endogène ou recombiné.

11. Méthode selon l'une quelconque des revendications 8 à 10, dans laquelle l'acide nucléique est homologue ou hétérologue.

12. Méthode selon l'une quelconque des revendications précédentes, dans laquelle la cellule hôte bactérienne comprend en outre des séquences de contrôle permettant la surexpression contrôlée de séquences d'acides nucléiques endogènes ou recombinées.

13. Méthode selon l'une quelconque des revendications précédentes, dans laquelle la bêta-1,4-galactosyltransférase est codée par un gène choisi dans le groupe constitué par lgtB issu de *Neisseria meningitidis,* et lex-1 issu de *Aggregatibacter aphrophilus* NJ8700, ou des fragments fonctionnels de ceux-ci.

14. Méthode selon l'une quelconque des revendications précédentes, dans laquelle la cellule hôte bactérienne est une souche d'*Escherichia coli,* une espèce de *Lactobacillus,* une souche de *Corynebacterium glutamicum* ou une souche de *Clostridium.*

15. Méthode selon l'une quelconque des revendications précédentes, dans laquelle la cellule hôte bactérienne comprend en outre au moins l'un des gènes du groupe de gènes W csc d'*E. coli.*

16. Méthode selon l'une quelconque des revendications précédentes, dans laquelle la cellule hôte bactérienne est modifiée afin de ne pas exprimer des protéines de dégradation intracellulaire de précurseurs dudit oligosaccharide.

17. Méthode selon l'une quelconque des revendications précédentes, dans laquelle la cellule hôte bactérienne est en outre génétiquement modifiée afin de contenir une séquence d'acide nucléique codant pour une glycosidase pour la dégradation spécifique d'oligosaccharides interférents, tels que des intermédiaires, des produits secondaires ou des oligosaccharides endogènes générés par la cellule hôte bactérienne, où l'expression de ladite glycosidase se trouve sous le contrôle d'une séquence régulatrice.
